(19) Europäisches Patentamt
European Patent Office
Office européen des brevets

(11) Numéro de publication : **0 461 040 A1**

(12) **DEMANDE DE BREVET EUROPEEN**

(21) Numéro de dépôt : **91401481.6**

(22) Date de dépôt : **06.06.91**

(51) Int. Cl.$^5$ : **C07D 235/08,** A61K 31/415, C07D 235/02, C07D 473/04, C07D 473/28, C07D 473/30, C07D 403/12, C07D 471/04, C07D 487/04, A61K 31/52, A61K 31/40

(30) Priorité : **08.06.90 FR 9007136**
**13.03.91 FR 9103043**

(43) Date de publication de la demande :
**11.12.91 Bulletin 91/50**

(84) Etats contractants désignés :
**AT BE CH DE DK ES FR GB GR IT LI LU NL SE**

(71) Demandeur : **ROUSSEL-UCLAF**
**35, Boulevard des Invalides**
**F-75007 Paris (FR)**

(72) Inventeur : **Fortin, Michel**
**12, Passage Cottin**
**F-75018 Paris (FR)**

Inventeur : **Frechet, Daniel**
**45, Rue Lecourbe**
**F-75015 Paris (FR)**
Inventeur : **Hamon, Gilles**
**7, Boulevard de l'Ouest**
**F-93340 Le Raincy (FR)**
Inventeur : **Jouquey, Simone**
**137, rue des Pyrénées**
**F-75020 Paris (FR)**
Inventeur : **Vevert, Jean-Paul**
**55, rue Rouget de l'Isle**
**F-93500 Pantin (FR)**

(74) Mandataire : **Bourgouin, André et al**
**Département des Brevets ROUSSEL UCLAF**
**111, route de Noisy B.P. no 9**
**F-93230 Romainville (FR)**

(54) **Nouveaux dérivés de l'imidazole, leur procédé de préparation, les nouveaux intermédiaires obtenus, leur application à titre de médicaments et les compositions pharmaceutiques les renfermant.**

(57) L'invention concerne les produits :

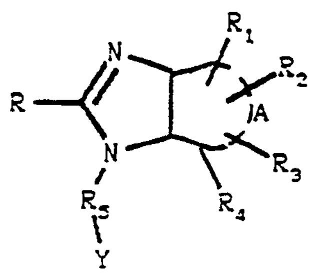

(I)

dans lesquels :
A représente reste carbocyclique ou hétérocyclique,
R représente alkyle, alkényle, alkynyle,
$R_1$, $R_2$, $R_3$ et $R_4$, représentent hydrogène ; halogène ; hydroxyle ; cyano ; nitro ; sulfo ; formyle ; benzoyle ; acyle ; acyloxy ; carboxy ; mercapto ; alkyle ; alkényle ; alkynyle ; alkoxy ; alkylthio ; aryle ; arylalkyle ; arylalkényle ; $-(CH_2)_m-SO_2-X-R_{14}$ ;

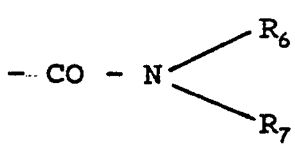

ou

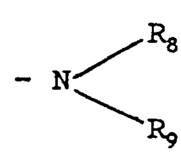

avec :

EP 0 461 040 A1

ou bien $R_6$ et $R_7$ ou $R_8$ et $R_9$ représentent hydrogène, alkyle, alkényle, aryle, arylalkyle, $-(CH_2)_m-SO_2-X-R_{14}$,
soit $X-R_{14}$ représente $NH_2$
soit X représente -NH-, -NH-CO-NH- ou -NH-CO-, simple liaison, et $R_{14}$ représente alkyle, alkényle, aryle,
ou bien $R_8$ et $R_7$ ou $R_8$ et $R_9$ forment avec azote un hétérocycle,
ou bien $R_8$ et $R_9$ représentent acyle, alkyle, arylsulfonyle.
$R_5$ représente alkylène,
Y représente $-Y_1-B-Y_2$ où :
$Y_1$ représente aryle carbocyclique ou hétérocyclique éventuellement substitués,
B représente :
soit simple liaison,
soit -CO-, -NH-CO-, -CO-NH- ou $-O-(CH_2)_n-$,
$Y_2$ représente $Y_1$, si B représente simple liaison, hydrogène, cyano, carboxy,
et leurs sels, leur préparation et leur application comme médicaments.

La présente invention concerne de nouveaux dérivés de l'imidazole, leur procédé de préparation, les nouveaux intermédiaires obtenus, leur application à titre de médicaments et les compositions pharmaceutiques les renfermant.

La présente invention a pour objet les produits de formule (I) :

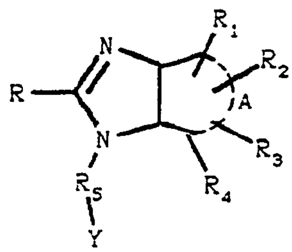

(I)

dans laquelle :

A représente le reste d'un radical monocyclique comprenant 3, 4 ou 5 chaînons ou d'un radical constitué de cycles condensés comprenant 6 à 12 chaînons, ces radicaux étant saturés ou insaturés et renfermant éventuellement un ou plusieurs hétéro-atomes choisis parmi les atomes d'oxygène, et les atomes d'azote et de soufre éventuellement oxydés,

R représente un radical alkyle, alkényle ou alkynyle linéaire ou ramifié renfermant au plus 6 atomes de carbone et éventuellement substitués,

$R_1$, $R_2$, $R_3$ et $R_4$, identiques ou différents, représentent :

a) un atome d'hydrogène, un atome d'halogène, un radical hydroxyle, cyano, nitro, sulfo, formyle, benzoyle, acyle ou acyloxy ayant au plus 12 atomes de carbone, carboxy libre, salifié ou estérifié, mercapto,

b) un radical alkyle, alkényle, alkynyle, alkoxy ou alkylthio, ces radicaux étant linéaires ou ramifiés, renfermant au plus 6 atomes de carbone et étant éventuellement substitués,

c) un radical aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués,

d) un radical

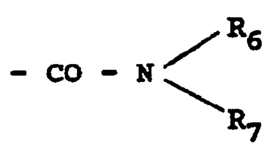

ou

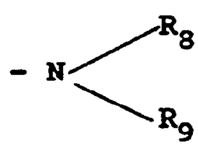

dans lesquels :

ou bien $R_6$ et $R_7$ ou $R_8$ et $R_9$, identiques ou différents, représentent :

– un atome d'hydrogène,

– un radical alkyle ou alkényle renfermant au plus 6 atomes de carbone et éventuellement substitué par un atome d'halogène, un radical hydroxyle ou un radical alkoxy renfermant au plus 6 atomes de carbone,

– un radical aryle ou arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié,

– un radical $-(CH_2)_m-SO_2-X-R_{14}$ dans lequel m représente un entier de 0 à 4 et

soit $X-R_{14}$ représente $NH_2$

soit X représente les radicaux -NH-, -NH-CO-NH- ou -NH-CO- ou une simple liaison

et $R_{14}$ représente un radical alkyle, alkényle et aryle, ces radicaux étant éventuellement substitués,

ou bien $R_6$ et $R_7$ ou $R_8$ et $R_9$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié,

ou bien $R_8$ et $R_9$, identiques ou différents, représentent un radical acyle dérivé d'acide carboxylique renfermant au plus 6 atomes de carbone, ou un radical alkyle ou arylsulfonyle dans lequel le radical alkyl renferme au plus 6 atomes de carbone et le radical aryle renferme au plus 8 atomes de carbone, ces radicaux étant éventuellement substitués par un ou plusieurs substituants choisis parmi les atomes d'halogène ou les radicaux alkyle renfermant au plus 6 atomes de carbone,

e) un radical $-(CH_2)_m-SO_2-X-R_{14}$ dans lequel m, X et $R_{14}$ ont la signification précédente,

$R_5$ représente un radical divalent alkylène, linéaire ou ramifié, renferment au plus 4 atomes de carbone,

Y représente le radical $-Y_1-B-Y_2$ dans lequel :

$Y_1$ représente un radical aryle monocyclique comprenant 5 ou 6 chaînons ou constitué de cycles condensés comprenant 8 à 10 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les radicaux que peuvent représenter $R_1$, $R_2$, $R_3$ et $R_4$,

B représente :

soit une simple liaison entre $Y_1$ et $Y_2$,

soit l'un des radicaux divalents suivants : -CO-, -O-, -NH-CO-, -CO-NH- ou $-O-(CH_2)_n-$ avec n représentant les valeurs 1 à 3,

$Y_2$ représente :

soit, quelle que soit la valeur de B autre qu'une simple liaison et $Y_2$ étant identique ou différent de $Y_1$, les valeurs définies pour $Y_1$,

soit, si B représente une simple liaison, un atome d'hydrogène, un radical cyano, carboxy libre, salifié ou estérifié, étant entendu que :

lorsque A représente un radical phényle éventuellement substitué par un ou deux radicaux choisis parmi les atomes d'halogène, les radicaux alkyle éventuellement substitué, alkoxy, acyle, carboxy libre ou phényle substitué par 5 atomes de fluor,

$R_5$ représente $-CH_2-$,

et Y représente le radical $-Y_1-B-Y_2$ dans lequel $Y_1$ représente un radical phényle non substitué et $Y_2$ représente un radical phényle substitué en ortho par un radical carboxy libre éventuellement salifié ou un radical tétrazolyle ou trifluorométhyl-sulfonamide et comportant éventuellement un second substituant choisi parmi les atomes d'halogène, les radicaux alkyle renfermant au plus 4 atomes de carbone, les radicaux nitro et méthoxy,

alors B représente -CO-NH- ou $-O-(CH_2)_n-$ avec n représentant les valeurs 2 ou 3,

lesdits produits de formule (I) étant sous toutes les formes isomères possibles racémiques, énantiomères et diastéréoisomères,

ainsi que les sels d'addition avec les acides minéraux et organiques ou avec les bases minérales et organiques desdits produits de formule (I).

Dans les produits de formule (I) et dans ce qui suit :

– les termes radical monocyclique et radical constitué de cycles condensés désignent des radicaux carbocycliques ou hétérocycliques saturés ou insaturés étant entendu que les radicaux hétérocycliques tels que définis ci-dessus peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents :

– le terme radical monocyclique désigne de préférence les radicaux qui renferment 5 ou 6 chaînons, le terme reste d'un radical monocyclique pour A désignant ainsi de préférence des restes de radicaux qui renferment 3 ou 4 chaînons :

parmi les radicaux monocycliques carbocycliques saturés, on peut citer, par exemple, les radicaux cyclohexyle et cyclopentyle ou encore cycloheptyle ;

parmi les radicaux monocycliques carbocycliques insaturés, on peut citer, par exemple, les radicaux cyclopentènyle, cyclohexènyle, cycloheptènyle, cyclopentadiényle, cyclohexadiényle et les radicaux aryles carbocycliques comme le radical phényle ;

parmi les radicaux monocycliques hétérocycliques saturés, on peut citer, par exemple, les radicaux pyrrolidinyle, imidazolidinyle, pyrazolidinyle, pipéridyle, pipérazinyle ou morpholinyle,

parmi les radicaux monocycliques hétérocycliques insaturés, on peut citer les radicaux aryliques,

par exemple, les radicaux thiényle, furyle, pyrannyle, pyrrolyle, imidazolyle, pyrazolyle, pyridyle, pyrazinyle, pyrimidinyle, pyridazinyle, thiazolyle, azépine, oxazolyle, furazannyle, pyrrolinyle tel que delta 2-pyrrolinyle, imidazolinyle tel que delta 2-imidazolinyle, pyrazolinyle tel que delta 3-pyrazolinyle ainsi que les isomères de position du ou des hétéroatomes que ces radicaux peuvent renfermer tels que, par exemple, les radicaux isothiazolyle ou isoxazolyle, tétrazolyle, thiadiazolyle, triazolyle (1,2,3 ou 1,3,4-triazolyl), cyanotriazolyle, carboxy triazolyle, méthoxycarbonyltriazolyle, trifluorométhyltriazolyle,

– le terme radical constitué de cycles condensés désigne de préférence les radicaux qui renferment 8 à 14 chaînons, le terme reste d'un radical constitué de cycles condensés désignant ainsi de préférence des restes de radicaux qui renferment 6 à 12 chaînons :

parmi les radicaux constitués de cycles condensés carbocycliques saturés, on peut citer, par exemple, le bicyclo(4,4,0) décyle, le bicyclo(4,4,1) undécyle,

parmi les radicaux constitués de cycles condensés carbocycliques insaturés, on peut citer les radicaux aryliques, par exemple, les radicaux naphtyle et phénanthryle,

parmi les radicaux constitués de cycles condensés hétérocycliques saturés, on peut citer, par exemple, le oxa-1-spiro(4,5)décane, le tétrahydropyranne-2-spirocyclohexane, le cyclohexanespiro-2'-(tétrahydrofurame) ou le diaza-1,10 anthryle-4,

parmi les radicaux constitués de cycles condensés hétérocycliques insaturés, on peut citer, par exemple, le benzothiényle, le naphto(2,3-b) thiényle, l'indane, l'indényle, le thianthrényle, l'isobenzofurannyle, le chroményle, le xanthényle, le phénoxathiinyle, l'indolizinyle, l'iso-indolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle, le phénazinyle, le phénothiazinyle, le phénoxazinyle, l'indolinyle, l'iso-indolinyle ou encore les systèmes polycycliques condensés constitués de monocycliques hétérocycliques tels que définis, par exemple, ci-dessus comme par exemple le furo(2,3-b) pyrrole ou le thiéno(2,3-b) furanne.

Lorsque A représente le reste d'un radical monocyclique comprenant 3 à 4 chaînons ou d'un radical constitué de cycles condensés comprenant 6 à 12 chaînons, ces radicaux dont A constitue un reste comportent respectivement 5 ou 6 chaînons ou 8 à 14 chaînons, peuvent être saturés ou insaturés, carbocycliques ou hétérocycliques, étant entendu que ces radicaux hétérocycliques peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents : ces radicaux monocycliques ou constitués de cycles condensés dont A peut constituer un reste peuvent donc être représentés par les radicaux indiqués ci-dessus.

Dans les produits de formule (I) et dans ce qui suit :

– l'atome de soufre peut être oxyde sous forme de sulfoxyde ou de sulfone,

– le terme radical alkyle linéaire ou ramifié désigne de préférence les radicaux méthyle, éthyle, n-propyle, isopropyle, n-butyle, isobutyle, sec-butyle et tert-butyle mais peut également représenter un radical pentyle ou hexyle et particulièrement isopentyle et isohexyle,

– le terme radical alkényle linéaire ou ramifié désigne de préférence un radical vinyle, allyle, 1-propényle, butényle et particulièrement butèn-1-yl, ou pentényle,

– le terme radical alkynyle linéaire ou ramifié désigne de préférence un radical éthynyle, propargyle, butynyle ou pentynyle,

– le terme atome d'halogène désigne de préférence l'atome de chlore, mais peut aussi représenter un atome de fluor, de brome ou d'iode,

– le terme radical acyle ayant de 2 à 6 atomes de carbone désigne de préférence un radical acétyle, propionyle, butyryle ou benzoyle, mais également un radical valéryle, hexanoyle, acryloyle, crotonoyle ou carbamoyle,

– le terme radical acyloxy désigne de préférence les groupements renfermant un des radicaux acyle tels que définis ci-dessus et liés à un atome d'oxygène tel que par exemple acétoxy ou benzoyloxy,

– le terme carboxy estérifié désigne de préférence un groupe alkoxy inférieur carbonyle tel que méthoxycarbonyle, éthoxycarbonyle, tert-butoxycarbonyle ou benzyloxycarbonyle,

– le terme radical alkoxy linéaire ou ramifié désigne de préférence les radicaux méthoxy ou éthoxy, mais peut aussi représenter un radical propoxy, isopropoxy, butoxy linéaire, secondaire ou tertiaire,

– le terme radical alkylthio linéaire ou ramifié désigne les radicaux dans lesquels le radical alkyle peut représenter, par exemple, les valeurs indiquées ci-dessus pour le radical alkyle ; le radical alkylthio représente de préférence les radicaux méthylthio ou éthylthio, mais peut aussi représenter un radical propylthio, isopropylthio, n-butylthio, sec-butylthio, tert-butylthio, isopentylthio ou isohexylthio,

– le terme radical aryle désigne les radicaux aryles tels que définis ci-dessus, soit les radicaux insaturés, monocycliques ou constitués de cycles condensés, carbocycliques ou hétérocycliques, étant entendu que

les radicaux hétérocycliques peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents :

comme exemples de tel radical aryle, on peut citer les radicaux phényle, naphtyle, thiényle tel que thién-2-yle et thién-3-yle, furyle tel que fur-2-yle, pyridyle tel que pyrid-3-yle, pyrimidyle, pyrrolyle, thiazolyle, isothiazolyle, diazolyle, triazolyle, tétrazolyle, thiadiazolyle, thiatriazolyle, oxazolyle, oxadiazolyle, 3- ou 4-isoxazolyle ; des groupes hétérocycliques condensés contenant au moins un hétéro-atome choisi parmi le soufre, l'azote et l'oxygène, par exemple benzothiényle tel que benzothién-3-yle, benzofuryle, benzopyrrolyle, benzimidazolyle, benzoxazolyle, thionaphtyle, indolyle ou purinyle.

Parmi les radicaux aryle on préfère le radical phényle et le radical tétrazolyle.

De tels radicaux aryles peuvent éventuellement être substitués comme par exemple le radical pyrrolyl N-substitué, par exemple N-méthylpyrrolyle, le radical 3- ou 4-isoxazolyle substitué, par exemple, 3-aryl-5-méthylisoxazol-4-yle, le groupe aryle étant par exemple, un groupe phényle ou halophényle.

Parmi les substituants des radicaux aryles et particulièrement du radical tétrazolyle on peut citer également les radicaux alkyle, alkényle, alkoxy ayant au plus 4 atomes de carbone et les radicaux alkoxyalkyle ou les radicaux arylalkyle, tel que benzyle lui-même éventuellement substitué par les radicaux nitro, méthoxy, hydroxyle, amino ou halogène.

– Les termes arylalkyle et arylalkanyle désignent des radicaux dans lesquels respectivement les radicaux alkyle, alkényle et aryle peuvent prendre les valeurs définies ci-dessus pour ces radicaux ; comme exemples de tels radicaux arylalkyle on peut citer les radicaux benzyle, diphénylméthyle, triphénylméthyle, naphtylméthyle, indénylméthyle, thiénylméthyle tel que thién-2-ylméthyle, furylméthyle tel que furfuryle, pyridylméthyle, pyrimidylméthyle ou pyrrolylméthyle, étant entendu que dans la liste non exhaustive d'exemples de radicaux telle que citée ci-dessus, le radical alkyle peut être représenté tout aussi également par les radicaux éthyle, propyle ou butyle tel que, par exemple, dans le radical phényléthyle ;

comme exemples de radicaux arylalkényle, on peut citer les exemples donnés ci-dessus de radicaux arylalkyle dans lesquels le radical alkyle est remplacé par un radical alkényle tel que par exemple dans les radicaux phénylvinyle ou phénylallyle, étant entendu que dans ces radicaux le radical phényle peut être remplacé tout aussi également par un radical naphtyle, pyridyle ou encore par exemple l'un des radicaux aryles tels que définis ci-dessus dans la liste non exhaustive des radicaux aralkyles.

Les radicaux alkyle, alkényle et alkynyle tels que définis ci-dessus ainsi que les radicaux alkyle ou alkényle des radicaux alkylthio, arylalkyle et arylalkényle tels que définis ci-dessus, peuvent ne pas être substitués ou porter un ou plusieurs substituants choisis, par exemple, dans le groupe formé par les atomes d'halogène, tel que chloro ou bromo, comme dans, par exemple, le groupe 2-bromoéthyle ; les radicaux hydroxyle ; aryle tel que défini ci-dessus, soit un radical monocyclique ou constitué de cycles condensés carbocyclique ou hétérocyclique, étant entendu que les radicaux hétérocycliques tels que définis ci-dessus peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents, ce radical hétérocyclique pouvant être lié par un atome de carbone ou, le cas échéant, par un atome d'azote ; arylalkyle dans lequel le radical aryle est tel que défini ci-dessus ; cycloalkyle, par exemple cyclopropyle, cyclopentyle ou cyclohexyle ; cycloalkényle tel que par exemple le radical cyclohexényle peuvent être éventuellement substitués, parmi lesquels on peut citer le diméthyl-1,3 cyclohexène ; alkoxy, tel que défini ci-dessus par exemple méthoxy, éthoxy, n-propoxy ou iso-propoxy comme dans par exemple les groupes méthoxyméthyle ou 1-éthoxyéthyle ; alkoxy substitué tel que trihaloalkoxy comme, par exemple, trifluorométhoxy ; aryloxy, par exemple phénoxy ; aralkoxy, par exemple benzyloxy ; mercapto ; alkylthio, par exemple méthylthio ou éthylthio ; alkylthio substitué tel que trihaloalkylthio comme, par exemple, trifluorométhylthio ; arylthio ; aralkylthio ; amino comme dans, par exemple, le groupe 2-aminoéthyle ; amino substitué par un ou deux radicaux choisis par exemple parmi les radicaux alkyle, alkényle, aryle et arylalkyle tels que définis ci-dessus comme par exemple monoalkylamino dans, par exemple, méthylamino ou éthylamino, comme par exemple dialkylamino dans, par exemple, diméthylamino ; nitro ; cyano ; azido ; carboxy ; carboxy estérifié, par exemple méthoxycarbonyle ou éthoxycarbonyle ; formyle ; acyle, par exemple acétyle, propionyle ou benzoyle ; acyle substitué par exemple par un radical amino tel que défini ci-dessus ou par un radical cyclique lié au radical acyle par un atome d'azote, ce radical cyclique pouvant renfermer éventuellement un ou plusieurs hétéroatomes choisis parmi les atomes d'azote, d'oxygène ou de soufre et tel que défini ci-dessus ; acyloxy, par exemple acétoxy ou propionyloxy ; carbamoyle ; carbamoyle substitué par exemple un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle ; un groupe N-(hydroxyalkyl inférieur) carbamoyle, tel que N-(hydroxyméthyl) carbamoyle, N-(hydroxyéthyl) carbamoyle, un groupe carbamoylalkyle inférieur, tel que carbamoylméthyle, carbamoyléthyle ; phtalimido ; acylamido, par exemple acétamido ou ben-

zamido ; alkoxycarbonylamino, par exemple méthoxycarbonylamino ou éthoxycarbonylamino ; ou aralkoxycarbonylamino, par exemple benzyloxycarbonylamino.

Les radicaux aryle et alkoxy tels que définis ci-dessus et les radicaux aryles des radicaux arylalkyle et arylalkényle tels que définis ci-dessus, peuvent ne pas être substitués ou porter un ou plusieurs substituants choisis, par exemple, dans la liste indiquée ci-dessus pour les éventuels substituants des radicaux alkyle, alkényle et alkynyle tels que définis ci-dessus, comme par exemple pour donner le radical o-chlorophényle mais peuvent également être substitués par un ou plusieurs radicaux choisis dans le groupe formé par les radicaux alkyle, tel que alkyle inférieur, par exemple méthyle, éthyle, ou également isopropyle ou ter-butyle ; alkényle ; alkyle substitué tel que par exemple trihaloalkyle comme dans trifluorométhyle ; alkényle tel que, par exemple, vinyle ou allyle ; alkynyle tel que, par exemple, propargyle.

Les radicaux amino que peuvent représenter l'un ou plusieurs des éventuels substituants des radicaux définis dans les produits de formule (I) et dans ce qui suit et que peuvent représenter en particulier les radicaux

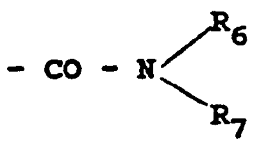 et 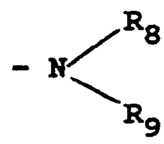

désigne des radicaux dans lesquels à l'atome d'azote sont liés deux radicaux, identiques ou différents, choisis parmi l'atome d'hydrogène ; les radicaux alkyles tels que définis ci-dessus pour donner de préférence les radicaux monoalkyl- ou dialkylamino dans lesquels les radicaux alkyles linéaires ou ramifiés renferment de 1 à 6 atomes de carbone et en particulier des radicaux méthyle, éthyle, isopropyle, trifluorométhyle, pentafluoroéthyle, hydroxyméthyle, hydroxyéthyle, méthoxyméthyle, méthoxyéthyle, éthoxyéthyle ; les radicaux alkényles tels que définis ci-dessus et représentés de préférence par les radicaux vinyle et allyle ; les radicaux aryles ou arylalkyles tels que définis ci-dessus, carbocycliques ou hétérocycliques et en particulier phényle, benzyle, phénéthyle, naphtyle, indolyle, indolinyle, thiényle, furyle, pyrrolyle, pyridyle, pyrrolidinyle, pipéridino, morpholino, pipérazinyle, ces radicaux pouvant être substitués par un ou plusieurs radicaux tels que définis ci-dessus comme par exemple dans méthylpipérazinyle, fluorométhylpipérazinyle, éthylpipérazinyle, propylpipérazinyle, phénylpipérazinyle ou benzylpipérazinyle.

Lorsque $R_6$ et $R_7$ d'une part ou $R_8$ et $R_9$ d'autre part forment ensemble avec l'atome d'azote auquel ils sont liés un hétérocycle, il s'agit, par exemple, d'un cycle pyrrolyle, imidazolyle, pyridyle, pyrazinyle, pyrimidyle, indolyle, indolinyle, purinyle, quinolyle, pyrrolidinyle, pipéridyle, pipéridino, morpholino, pipérazinyle ; ces radicaux peuvent être éventuellement substitués par les substituants déjà mentionnés précédemment et en particulier par un ou plusieurs radicaux choisis parmi les atomes de chlore et de fluor,les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment dans les radicaux aryle, arylalkyle et arylalkényle.

Les radicaux acyle que peuvent représenter $R_8$ et $R_9$ sont tels que définis précédemment et peuvent être choisis par exemple parmi les radicaux acétyle, propionyle, butyryle, valéryle ou carbamoyle, les radicaux alkyl cu arylsulfonyl que peuvent représenter $R_8$ ou $R_9$ sont choisis de préférence parmi les radicaux méthylsulfonyl, trifluorométhylsulfonyl ou paratolylsylfonyl.

Les radicaux $Y_1$ et $Y_2$ peuvent représenter les valeurs définies ci-dessus pour les radicaux aryles monocycliques ou constitués de cycles condensés, étant entendu que dans le cas où B représente une simple liaison $Y_2$ peut également représenter un atome d'hydrogène, un radical cyano ou un radical carboxy, libre, salifié ou estérifié, ce radical carboxy estérifié désignant de préférence un groupe alkoxy inférieur carbonyle tel que méthoxycarbonyle, éthoxycarbonyle ou benzyloxycarbonyle.

Les radicaux $Y_1$ ou $Y_2$, identiques ou différents, représentent un radical aryle éventuellement substitué par un ou plusieurs radicaux choisis, de préférence, parmi les atomes d'halogène, les radicaux hydroxyle, nitro, les radicaux alkyle, alkényle, alkoxy, acyle et carboxy libre, salifié ou estérifié, ces radicaux renfermant au plus 6 atomes de carbone et étant tels que définis ci-dessus.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique,l'acide alpha, bêta-éthanedisulfonique, les acides arylmonosul-

foniques tels que l'acide benzènesulfonique et les acides aryldisulfoniques.

Le ou les radicaux carboxy des produits de formule (I) peuvent être salifiés par des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Le radical $-(CH_2)_m-SO_2-X-R_{14}$ peut représenter par exemple les radicaux dans lesquels $(CH_2)_m$ représente les valeurs des radicaux alkylène, dérivés des radicaux alkyle linéaires indiqués ci-dessus telles que, par exemple, méthylène, éthylène, n-propylène ou butylène et $R_{14}$ peut représenter un radical alkyle ou alkényle choisi parmi les valeurs définies ci-dessus ou un radical aryle également choisi parmi les valeurs indiquées ci-dessus pour ce radical telles que par exemple phényle, biphényle, naphtyle, tétrazolyle ; le radical alkyle que peut représenter le radical $R_{14}$ peut éventuellement être notamment substitué par un radical aryle choisi parmi les valeurs définies ci-dessus pour former un radical aralkyle.

Ces radicaux alkyle, alkényle, aryle et aralkyle peuvent eux-mêmes être substitués ainsi qu'il est indiqué ci-dessus pour ces radicaux. Parmi les substituants que peut porter le radical $R_{14}$ lorsqu'il représente un radical alkyle, alkényle, aryle ou aralkyle. On peut notamment citer les radicaux suivants :

– $PO_3H$, -PO(OH)Alkyle, PO(OH)aryle, -PO(OH)Alkoxy, amino, mono ou dialkylamino, carboxy libre, estérifié ou salifié, nitro, halogène, alkylthio, alkoxy, hydroxy, mercapto.

On peut citer par exemple et de façon non exhaustive les radicaux :

$-SO_2-NH_2$, $-SO_2-NH-CH_3$, $-SO_2-NH-CF_3$, $-SO_2-NH-C_6H_5$,
$-SO_2-NH-CH_2-C_6H_5$,
$-CH_2-SO_2-NH_2$, $-CH_2-SO_2-NH-C_6H_5$,
$-SO_2-NH-CO-NH-CH_3$, $-SO_2-NH-CO-NH-C_6H_5$,
$-SO_2-NH-CO-NH-CF_3$, $-SO_2-NH-CO-NH-CH_2-C_6H_5$,
$-SO_2-NH-CO-NH-C_6H_4Cl$,

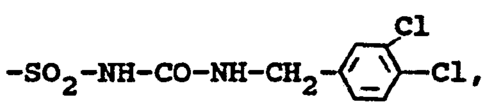

$-SO_2-NH-CO-NH-CH=CH-CH_3$,

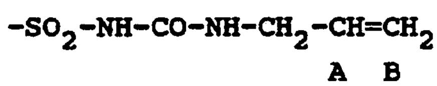

dans lequel A et B identiques ou différents sont choisis parmi l'atome d'hydrogène, les radicaux phényle, pyridyle et pyridinyle,

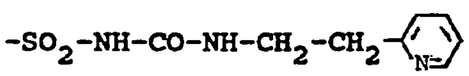 , 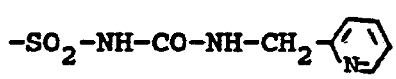 ,

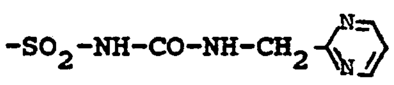 , 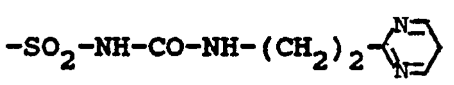 .

Le radical aryle que représente $Y_1$ ou $Y_2$ peut être substitué par un ou plusieurs radicaux choisis parmi les valeurs de $R_1$, $R_2$, $R_3$ et $R_4$ et en particulier par les radicaux

$-NH-(CH_2)_m-SO_2-X-R_{14}$ et

$-CO-NH-(CH_2)_m-SO_2-X-R_{14}$ dans lesquels le radical $(CH_2)_m-SO_22-X-R_{14}$ peut prendre par exemple les valeurs indiquées ci-dessus.

$Y_1$ ou $Y_2$ peut également être substitué par un radical carboxy libre, estérifié ou salifié, cyano formyle ou un radical tétrazolyle, tétrazolylealkyle, de préférence tétrazolyléthyle ou tétrazolylecarbamoyle.

Tous les radicaux indiqués ci-dessus sont de préférence situés en position ortho et sur le seul radical $Y_2$.

On peut citer par exemple et de façon non exhaustive les radicaux :

$-NH-SO_2-CH_3$, $-NH-SO_2-C_6H_5$, $-NH-SO_2-CF_3$,
$-NH-CH_2-SO_2-NH-C_6H_5$,

$-CO-NH-SO_2-C_2H_5$, $-CO-NH-SO_2-CH_3$,
$-CO-NH-SO_2-CH_2-C_6H_5$.

L'invention a notamment pour objet les produits de formule (I) telle que définie ci-dessus, caractérisés en ce que le ou les substituants, identiques ou différents que peuvent porter :

a) les radicaux alkyle, alkényle et alkynyle que peut représenter R,

b) les radicaux alkyle, alkényle, alkynyle, alkoxy et alkylthio que peuvent représenter $R_1$, $R_2$, $R_3$ et $R_4$,

c) les radicaux aryle, arylalkyle et arylalkényle que peuvent représenter $R_1$, $R_2$, $R_3$ et $R_4$

d) les radicaux alkyle, alkényle et aryle que peut représenter $R_{14}$,

sont choisis dans le groupe formé par :

– les atomes d'halogène, les radicaux hydroxyle, cyano, nitro, formyle, acyle ou acyloxy ayant au plus 6 atomes de carbone, benzoyle, carboxy libre, salifié ou estérifié par un radical alkyle renfermant au plus 6 atomes de carbone,

– les radicaux alkyle et alkényle renfermant au plus 6 atomes de carbone et éventuellement substitués par un ou plusieurs substituants choisis parmi les atomes d'halogène, le radical hydroxyle et les radicaux alkoxy renfermant au plus 6 atomes de carbone,

– les radicaux alkoxy linéaires et ramifiés renfermant au plus 6 atomes de carbone,

– les radicaux aryle et arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié,

– les radicaux

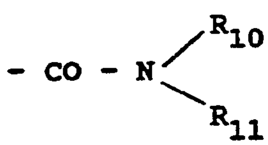

ou

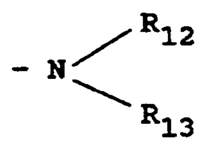

dans lesquels :

– ou bien $R_{10}$ et $R_{11}$ ou $R_{12}$ et $R_{13}$, identiques ou différents, représentent :

– un atome d'hydrogène,

– un radical alkyle ou alkényle renfermant au plus 6 atomes de carbone et éventuellement substitué par un ou plusieurs substituants choisis parmi les atomes d'halogène, le radical hydroxyle et les radicaux alkoxy renfermant au plus 6 atomes de carbone,

– un radical aryle ou arylalkyle dans lequel le radical alkyle linéaire ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène les radicaux hydroxyle, nitro, les radicaux alkyle, alkényle, alkoxy et axyle, ces radicaux renfermant au plus 6 atomes de carbone, les radicaux carboxy libre, salifié ou estérifié, tétralozyle, tétrazolylméthyle, tétrazolylcarbamoyle,

ou bien $R_{10}$ et $R_{11}$ ou $R_{12}$ et $R_{13}$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'alogène, les radicaux hydroxyle, nitro les radicaux alkyle, alkènyle, alkoxy et axyle, ces radicaux renfermant au plus 6 atomes de carbone, les radicaux carboxy libre, salifié ou estérifié, tétrazolyle, tétrazolylméthyle, tétrazolylcarbamoyle,

ou bien $R_{12}$ et $R_{13}$, identiques ou différents, représentent un radical acyle dérivé d'acide carboxylique renfermant au plus 6 atomes de carbone, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Parmi les substituants que peuvent comporter les radicaux alkyle, alkényle, alkynyle, alkoxy, alkylthio, aryle, arylalkyle et arylalkényle tels que définis ci-dessus, peuvent être cités plus particulièrement les atomes

d'halogène, tels que chloro et bromo ; les radicaux hydroxyle ; acyle tel que, par exemple, acétyle, propionyle, butyryle, valéryle, hexanoyle, acryloyle, crotonoyle ou carbamoyle ; benzoyle ; carboxy estérifié désignant de préférence un groupe alkoxy inférieur carbonyle tel que méthoxycarbonyle, éthoxycarbonyle ou benzyloxycarbonyle ; alkyle tel que méthyle ou éthyle ; amino ; amino substitué, tel que monoalkyl- et dialkylamino, par exemple méthylamino, éthylamino ou diméthylamino ; alkoxy, par exemple méthoxy, éthoxy ou isopropoxy ; aryle tel que phényle, biphényle, naphtyle, indényle, indolyle ou indolinyle ; aralkyle tels que, par exemple benzyle ou phénéthyle ; radicaux alkyle, alkoxy et aryle tels que définis ci-dessus pouvant eux-mêmes être substitués par un ou plusieurs radicaux, identiques ou différents, choisis, par exemple, dans le groupe formé par les radicaux hydroxy, alkyle et alkoxy linéaire ou ramifié, par exemple méthyle, éthyle, ter-butyle, méthoxy, éthoxy, isopropoxy ; amino substitué, tel que monoalkyl- et dialkylamino, par exemple méthylamino, éthylamino ou diméthylamino.

Comme exemples de radicaux dont A peut donc constituer un reste tel que défini ci-dessus, on peut citer, tout particulièrement :

– les radicaux monocycliques carbocycliques ou hétérocycliques renfermant 6 chaînons tels que les radicaux phényle, pyrannyle, pyridyle, pyrimidinyle, pyridazinyle, pyrazinyle, pipéridyle, pipérazinyle, pipéridino et morpholino ;

– les radicaux monocyclique carbocycliques ou hétérocycliques renfermant 5 chaînons, tel que par exemple le radical furyle, pyrrolyle, pyrrolinyle, imidazolyle ou pyrazolyle, isothiazolyle, isoxazolyle, pyrrolidinyle, imidazolidinyle, pyrazolidinyle.

– les radicaux constitués de cycles condensés carbocycliques ou hétérocycliques parmi lesquels par exemple les radicaux naphtyle, indolyle, quinolyle ou purinyle ainsi que leurs isomères de position du ou des hétéroatomes par exemple d'azote tels que par exemple le radical indazolyle ou isoquinolyle.

Quand l'hétérocycle que peut représenter A renferme un ou plusieurs atomes d'azote, ce ou ces atomes d'azote peuvent ne pas être substitués ou l'un ou plusieurs de ces atomes d'azote peuvent être substitués, par exemple, par un radical alkyle ou alkoxy linéaire ou ramifié renfermant de 1 à 5 atomes de carbone, tels que définis ci-dessus, par exemple méthyle, éthyle, isopropyle, tert-butyle, méthoxy ou éthoxy, un radical phényle ou benzyle, ces radicaux pouvant eux-même être substitués par les substituants déjà mentionnés ci-dessus pour les radicaux aryle et arylalkyle : on peut citer, comme exemples, les radicaux méthylpipérazinyle, éthylpipérazinyle, propylpipérazinyle, phénylpipérazinyle ou benzylpipérazinyle.

Parmi les valeurs préférées de A, on peut citer les radicaux phényle, naphtyle, pyridyle, pipérazinyle, pyrimidinyle, pyridazinyle et pyrazinyle.

L'invention a particulièrement pour objet les produits de formule (I) telle que définie ci-dessus, et répondant à la formule (Ia) :

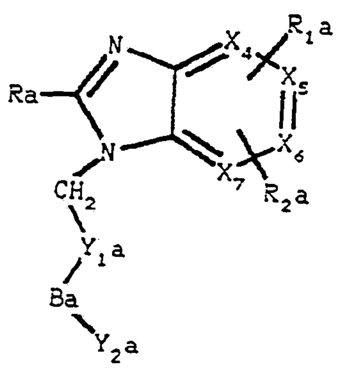

(Ia)

dans laquelle :

– $X_4$, $X_5$, $X_6$ et $X_7$ sont tels que :

soit ils représentent tous un radical méthine =CH-,

soit l'un ou deux quelconques d'entre eux représentent un atome d'azote et les autres représentent un radical méthine =CH-,

– $R_a$ représente un radical n-butyle ou buténуle,

– $R_{1a}$ et $R_{2a}$, identiques ou différents, sont choisis dans le groupe formé par :

. l'atome d'hydrogène,

. les atomes d'halogène,

. le radical hydroxyle, le radical mercapto,

. les radicaux alkoxy linéaires ou ramifiés renfermant au plus 6 atomes de carbone,

. les radicaux alkyle, alkényle, alkynyle et alkylthio linéaires ou ramifiés renfermant au plus 6 atomes de carbone et les radicaux aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alcoxy, alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkyle, alkoxy ou alkylthio renfermant au plus 6 atomes de carbone, mercapto, acyl, acyloxy, tétrazolyle,

. le radical carboxy libre,

. les radicaux carboxy estérifié par un radical alkyle linéaire ou ramifié renfermant au plus 4 atomes de carbone,

– $Y_{1a}$ représente un radical phényle,

– $B_a$ représente une simple liaison ou un radical -CO-NH-,

– $Y_{2a}$ est tel que :

soit, si $B_a$ représente une simple liaison ou un radical -CO-NH-, $Y_{2a}$ représente un radical phényle éventuellement substitué par un ou plusieurs radicaux choisis parmi les valeurs de $R_{1a}$ et $R_{2a}$,

soit, si $B_a$ représente une simple liaison, $Y_{2a}$ représente un radical cyano ou carboxy libre, salifié ou estérifié, ou tétrazolyle, étant entendu que si $B_a$ représente une simple liaison alors l'un au moins de $X_4$, $X_5$, $X_6$ et $X_7$ ne représente pas un radical méthine, lesdits produits de formule ($I_a$) étant sous toutes les formes isomères possibles racémiques, énantiomères et diastéréoisomères, ainsi que les sels d'addition avec les acides minéraux et organiques ou avec les bases minérales et organiques desdits produits de formule ($I_a$).

L'invention a plus particulièrement pour objet les produits de formule (I) telle que définie ci-dessus et répondant à la formule ($I_a$) dans laquelle $X_4$, $X_5$, $X_6$, $X_7$, $R_a$, $R_{1a}$ et $R_{2a}$ ont les significations indiquées ci-dessus et

– $Y_{1a}$ représente un radical phényle,

– $B_a$ représente une simple liaison ou un radical -CO-NH-,

– $Y_{2a}$ est tel que :

soit, si $B_a$ représente une simple liaison ou un radical -CO-NH-, $Y_{2a}$ représente un radical phényle éventuellement substitué par un radical $-(CH_2)_p-SO_2-X_a-R_{14a}$ dans lequel p représente les valeurs 0 et 1, $X_a$ représente les radicaux -NH-, -NH-CO-, -NH-CO-NH- ou une simple liaison et $R_{14a}$ représente un radical méthyle, éthyle, vinyle, allyle, pyridylméthyle, pyridyléthyle, pyridyle, phényle ou benzyle, par un radical carboxy libre, salifié ou estérifié, un radical tétrazolyle, tétrazolylalkyle ou tétrazolylcarbamoyle, dans lesquels le radical tétrazolyle est éventuellement substitué par un radical alkyle, alkényle, arylalkyle ou alcoxyalkyle,

soit, si $B_a$ représente une simple liaison, $Y_{2a}$ représente un radical cyano ou carboxy libre, salifié ou estérifié, ou tétrazolyle étant entendu que si $B_a$ représente une simple liaison alors l'un au moins de $X_4$, $X_5$, $X_6$ et $X_7$ ne représente pas un radical méthine, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

$R_{1a}$ et $R_{2a}$ peuvent prendre toutes les valeurs indiquées ci-dessus et par exemple les valeurs suivantes :

lorsque $R_{1a}$ ou $R_{2a}$ représente un radical alkoxy, ce radical peut représenter, par exemple, un radical méthoxy, éthoxy ou isopropoxy ;

lorsque $R_{1a}$ ou $R_{2a}$ représente un radical alkyle substitué, ce radical représente de préférence un radical méthyle, éthyle, isopropyle ou tert-butyle éventuellement substitué par un ou plusieurs radicaux choisis de préférence dans le groupe formé par les atomes d'halogène tel que brome, chlore ou fluor comme par exemple dans le radical trifluorométhyle ; les radicaux hydroxyle et alkoxy linéaire ou ramifié renfermant de 1 à 4 atomes de carbone, tel que, par exemple méthoxy, éthoxy ou isopropoxy ;

lorsque $R_{1a}$ ou $R_{2a}$ représente un radical carboxy estérifié, ce radical peut être, par exemple, méthoxycarbonyle ou éthoxycarbonyle.

Parmi les valeurs de A on peut citer les valeurs :

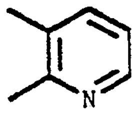 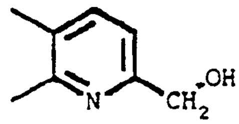

Parmi les différentes valeurs du cycle

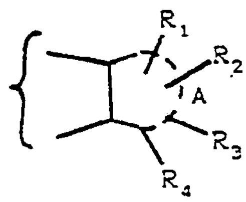

on peut citer notamment les valeurs suivantes :

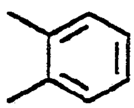 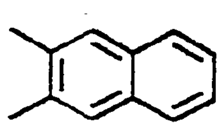 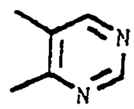

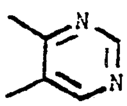 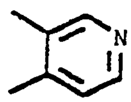 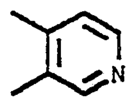

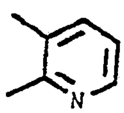 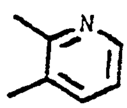 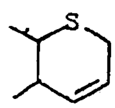 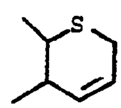

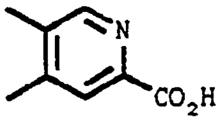 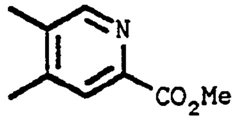 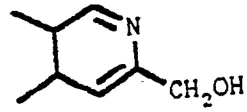

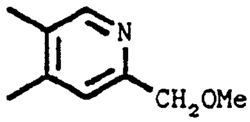 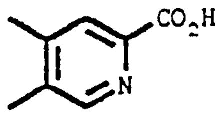 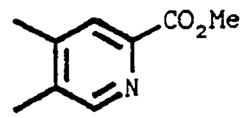

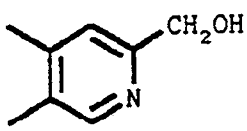 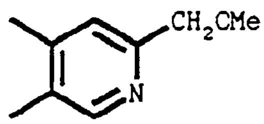 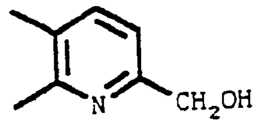

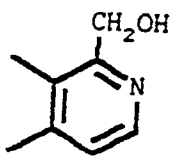 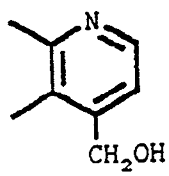 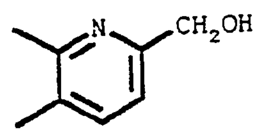

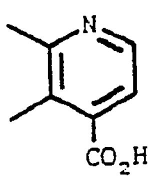 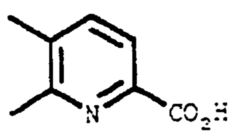 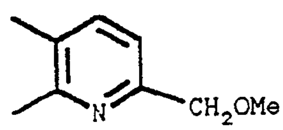

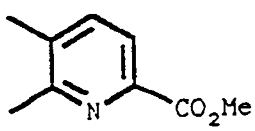 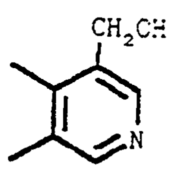 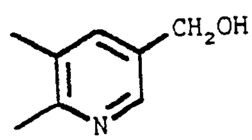

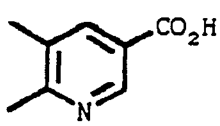 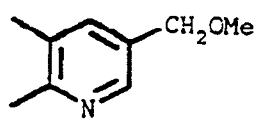 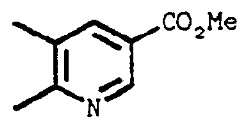

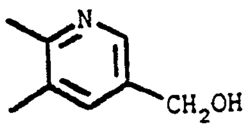
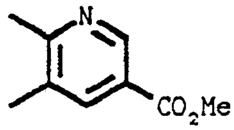
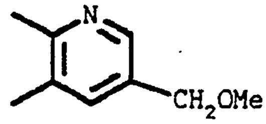

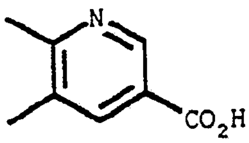
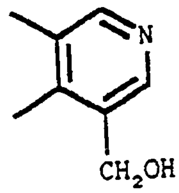
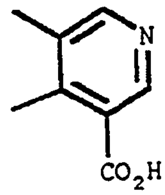

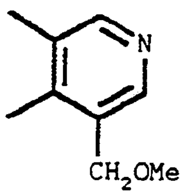
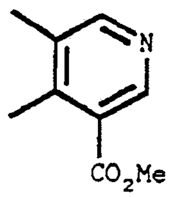
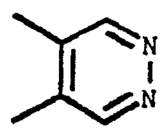

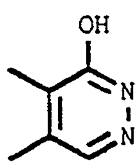
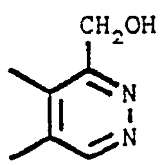
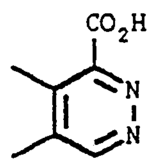

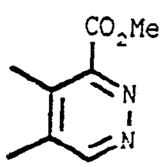
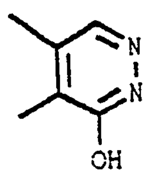
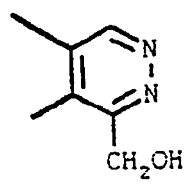

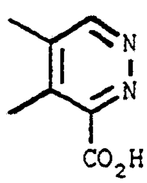
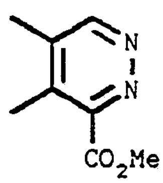
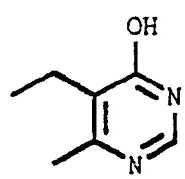

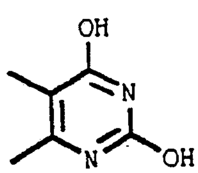
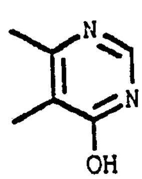
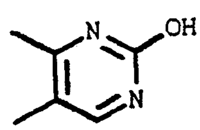

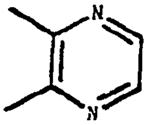
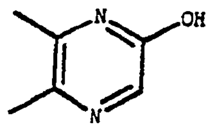
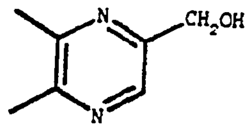
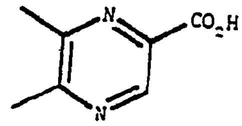
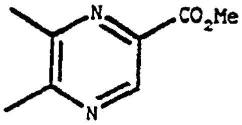
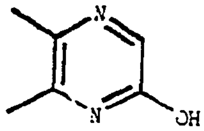
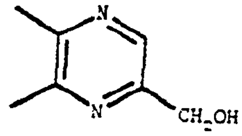
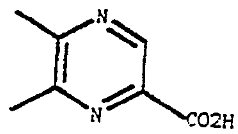
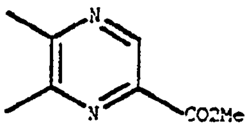
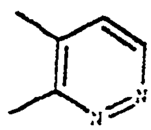
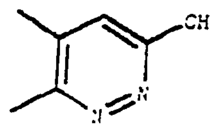
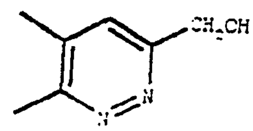
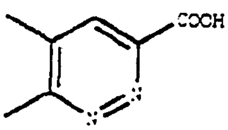
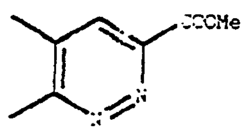
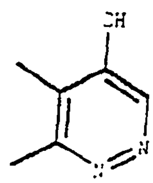
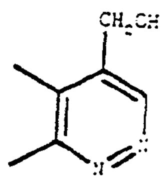
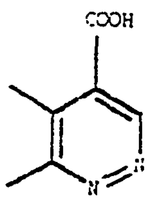
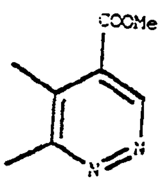
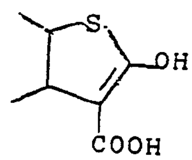
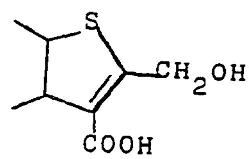
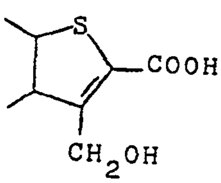
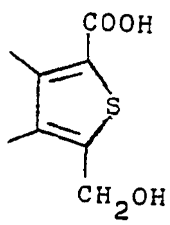
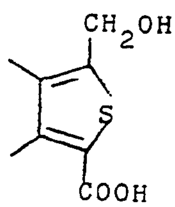
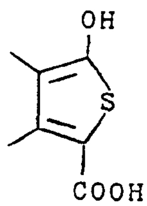
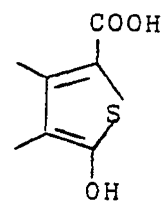

L'invention a tout particulièrement pour objet les produits de formule (I) telle que définie ci-dessus, dans laquelle A représente un radical phényle, naphtyle, pyridyle, pyrimidinyle ou thiényle,

R représente un radical n-butyle ou butèn-1-yle,

$R_1$, $R_2$, $R_3$ et $R_4$ sont tels que deux d'entre eux représentent un atome d'hydrogène et les deux autres, identiques ou différents, représentent un atome d'hydrogène, un radical hydroxyle, un radical alkyle renfermant au plus 4 atomes de carbone, un radical carboxy libre ou estérifié par un radical alkyle linéaire ou ramifié renfermant au plus 4 atomes de carbone,

$R_5$ représente un radical méthylène,

et Y représente le radical $-Y_1-B-Y_2$ dans lequel $Y_1$ représente un radical phényle, B représente une liaison simple carbonecarbone ou le radical -CO-NH- et $Y_2$ représente un radical cyano, carboxy libre ou estérifié par un radical alkyle linéaire ou ramifié renfermant au plus 4 atomes de carbone, un radical indolyle ou un radical phényle éventuellement substitué par un radical carboxy libre, salifié ou estérifié, un radical tétrazolyle, tétrazolylalkyle, tétrazolylcarbamoyle, dans lesquels le radical tétrazolyle est éventuellement substitué par un radical alkyle, alkényle ou alcoxyalkyle, ou par un radical $-(CH_2)_p-SO_2-X_a-R_{14a}$ dans lequel p représente les valeurs 0 et 1, $X_a$ représente les radicaux -NH-, NHCO-NH, -NH-CO- ou une simple liaison et $R_{14a}$ représente un radical méthyle, éthyle, vinyle, allyle, pyridylméthyle, pyridyléthyle, pyridyle, phényle ou benzyle, lesdits produits de formule (I) étant sous toutes les formes isomères possibles racémiques, énantiomères et diastéréoisomères, ainsi que les sels d'addition avec les acides minéraux et organiques ou avec les bases minérales et organiques

desdits produits de formule (I).

L'invention a plus particulièrement pour objet les produits de formule (I) telle que définie ci-dessus et répondant à la formule ($I_b$) :

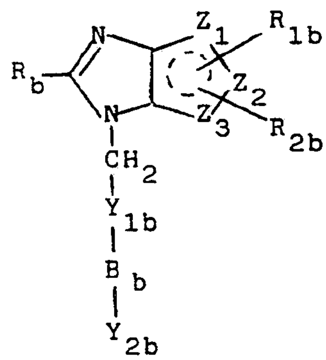

($I_b$)

dans laquelle $R_b$ représente un radical n-butyle ou butényle.

– $Z_1$, $Z_2$, $Z_3$ sont tels que :

l'un représente un atome de soufre

et les deux autres, identiques ou différents, représentent un radical méthine =CH-,

– $R_{1b}$ et $R_{2b}$, identiques ou différents, représentent :

. l'atome d'hydrogène,

. les atomes d'halogène,

. le radical hydroxyle, le radical mercapto,

. les radicaux alkoxy linéaires ou ramifiés renfermant au plus 6 atomes de carbone,

. les radicaux alkyle, alkényle, alkynyle et alkylthio, linéaires ou ramifiés renfermant au plus 6 atomes de carbone et les radicaux aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkoxy ou alkylthio renfermant au plus 4 atomes de carbone, mercapto, acyl, acyloxy,

. le radical carboxy libre,

. les radicaux carboxy estérifié par un radical alkyle linéaire ou ramifié renfermant au plus 4 atomes de carbone,

– $Y_{1b}$ représente un radical phényle,

– $B_b$ représente une simple liaison ou un radical -CO-NH-,

– $Y_{2b}$ est tel que :

soit, si $B_b$ représente une simple liaison ou un radical -CO-NH-, $Y_{2b}$ représente un radical phényle éventuellement substitué par un radical carboxy libre salifié ou estérifié un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, éthyle, vinyle, allyle, pyridylméthyle, pyridyléthyle, pyridyle, phényle ou benzyle,

soit, si $B_b$ représente une simple liaison, $Y_{2b}$ représente un radical cyano ou carboxy libre, salifié ou estérifié, lesdits produits de formule ($I_b$) étant sous toutes les formes isomères possibles racémiques, énantiomères et diastéréoisomères, ainsi que les sels d'addition avec les acides minéraux et organiques ou avec les bases minérales et organiques desdits produits de formule ($I_b$).

L'invention a également plus particulièrement pour objet les produits de formule (I) telle que définie ci-dessus répondant à la formule ($I_c$) :

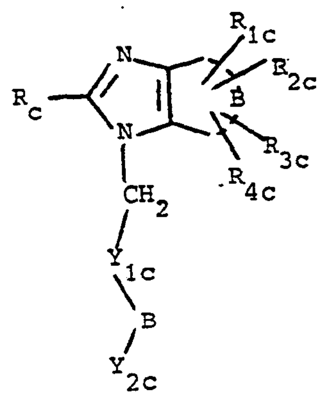

($I_c$)

dans laquelle

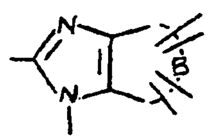

représente

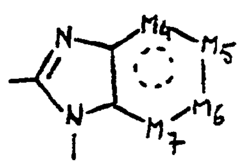

ou

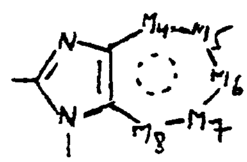

avec $M_4$, $M_5$, $M_8$, $M_7$ et $M_8$ identiques ou différents, représentent

- un radical méthine =CH- ou un atome d'azote éventuellement substitué par un radical choisi parmi les valeurs de $R_{1c}$, $R_{2c}$, $R_{3c}$ et $R_{4c}$,
- un radical méthylène $-CH_2-$ éventuellement substitué par un ou deux radicaux identiques ou différents choisis parmi les valeurs de $R_{1c}$, $R_{2c}$, $R_{3c}$ et $R_{4c}$,
- un radical -C=O,

un atome de soufre tels que l'un au moins de $M_4$, $M_5$, $M_6$, $M_7$ et $M_8$ représente un atome d'azote ou de soufre,

$R_c$ représente un radical alkyle ou alkényle renfermant au plus 4 atomes de carbone,

$R_{1c}$, $R_{2c}$, $R_{3c}$, $R_{4c}$ identiques ou différents, sont choisis parmi :

- l'atome d'hydrogène,
- le radical carboxy libre, salifié ou estérifié par un radical alkyle renfermant au plus 4 atomes de carbone ou par un radical aryle, ces radicaux alkyle et aryle étant eux-mêmes éventuellement substitués,
- les radicaux alkyle, alkényle et alkoxy renfermant au plus 4 atomes de carbone éventuellement substitués,
- les radicaux aryle,
- les radicaux amino et carbamoyle éventuellement subtitués par un ou deux radicaux identiques ou différents choisis parmi les radicaux alkyle et aryle eux-mêmes éventuellement substitués,

tous les radicaux alkyle, alkényle et aryle ci-dessus étant éventuellement substitués par un ou plusieurs radicaux choisis parmi les atomes d'halogène, les radicaux hydroxyle, carboxy libre, salifié ou estérifié par un radical alkyle renfermant au plus 4 atomes de carbone, amino, alkylamino, phényle, alkyle, alkényle et alkoxy renfermant au plus 4 atomes de carbone,

– $Y_{1c}$ représente un radical phényle,

– $B_c$ représente une simple liaison ou un radical -CO-NH-,

– $Y_{2c}$ est tel que :

soit, si $B_c$ représente une simple liaison ou un radical -CO-NH-, $Y_{2c}$ représente un radical phényle éventuellement substitué par un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, éthyle, n-propyle, vinyle, allyle, pyridylméthyle, pyridyléthyle, pyridyle, phényle, benzyle, pyrimidyle, tétrazolyle, thiazolyle, diazolyle, pipéridinyle ou tétrahydrofuranyle, ces radicaux étant éventuellement substitués par un radical méthyle, éthyle ou nitro,

soit, si $B_c$ représente une simple liaison, $Y_{2c}$ représente un radical cyano, formyle ou carboxy libre, salifié ou estérifié, lesdits produits de formule ($I_c$) étant sous toutes les formes isomères possibles racémiques, énantiomères et diastéréoisomères, ainsi que les sels d'addition avec les acides minéraux et organiques ou avec les bases minérales et organiques desdits produits de formule ($I_c$).

L'invention a encore plus particulièrement pour objet les produits de formule ($I_b$) telle que définie ci-dessus répondant à la formule ($I'_b$) :

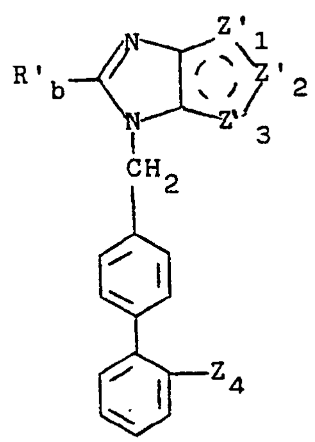

($I'_b$)

dans laquelle :

$R_{b'}$ représente un radical n-butyle,

$Z'_1$, $Z'_2$ et $Z'_3$ sont tels que :

l'un représente un atome de soufre,

et les deux autres, identiques ou différents, représentent un radical méthine =CH- éventuellement substitué par un radical hydroxyle ou un radical carboxy libre, salifié ou estérifié par un radical alkyle linéaire ou ramifié renfermant au plus 4 atomes de carbone,

et $Z_4$ représente un radical carboxy libre, salifié ou estérifié par un radical alkyle linéaire ou ramifié renfermant au plus 4 atomes de carbone ou un radical tétrazolyle, lesdits produits de formule ($I'_b$) étant sous toutes les formes isomères possibles racémiques, énantiomères et diastéréoisomères, ainsi que les sels d'addition avec les acides minéraux et organiques ou avec les bases minérales et organiques desdits produits de formule ($I'_b$).

Parmi les produits objet de l'invention, peuvent être cités tout particulièrement :

l'acide 2-butyl 1-[(4-carboxyphényl) méthyl] 1H-benzimidazole -6-carboxylique

l'acide 4-[(2-butyl-1H-benzimidazol-1-yl) méthyl] benzoïque le 4-[(2-butyl-1H-benzimidazol-1-yl) méthyl] N-(1H-indol-4-yl) benzamide

l'acide 4-[(2-butyl-1H-naphth(2,3-d)imidazol-1-yl) méthyl] benzoïque

l'acide 4-[(2-butyl-5,6-diméthyl-1H-benzimidazol-1-yl) méthyl] benzoïque

l'acide 4-[(2-butyl-3H-imidazo(4,5-c)pyridin-3-yl) méthyl] benzoïque

l'acide 4'-((2-butyl-3H-imidazo(4,5b) pyridin-3-yl) méthyl) (1,1'-biphényl) 2-carboxylique

le 2-butyl-1-((2'-carboxy-(1,1'-byphényl)-4-yl) méthyl)-6-hydroxy-1H-thiéno(2,3-d) imidazole-5-carboxylate de 1,1-diméthyléthyle et leurs sels.

L'invention a également pour objet un procédé de préparation de produits de formule (I) telle que définie ci-dessus, caractérisé en ce que :

soit l'on fait réagir un composé de formule (II) :

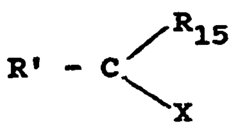

(II)

dans laquelle X représente un atome d'oxygène ou un radical =NH, $R_{15}$ représente un radical hydroxyle, alkoxy ou un atome d'halogène et $NH_2$, et R' a la signification indiquée ci-dessus, pour R dans laquelle les éventuelles fonctions réactives sont éventuellement protégées par des groupements protecteurs, avec un composé de formule (III) :

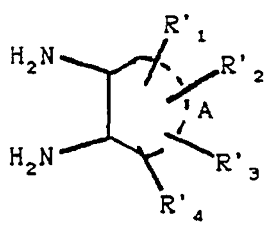

(III)

dans laquelle $R_1'$, $R_2'$, $R_3'$ et $R_4'$ ont les significations indiquées ci-dessus, respectivement pour $R_1$, $R_2$, $R_3$ et $R_4$ dans lesquelles les éventuelles fonctions réactives sont éventuellement protégées par des groupements protecteurs, pour obtenir un produit de formule IV, après isolement éventuel d'un intermédiaire de formule (IV') :

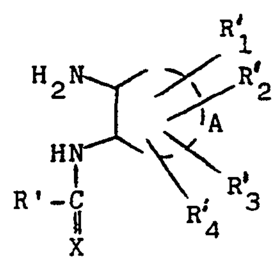

(IV')

dans laquelle R', $R'_1$, $R'_2$, $R'_3$, et $R'_4$ ont les significations indiquées ci-dessus, produit de formule (IV) :

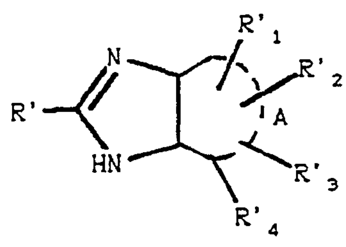

(IV)

dans laquelle R', $R_1'$, $R_2'$, $R_3'$ et $R_4'$ ont les significations indiquées ci-dessus, que l'on fait réagir avec un composé de formule (V) :

$$\text{Hal} - R_5 - Y' \qquad (V)$$

dans laquelle Hal représente un atome d'halogène, $R_5$ a la signification indiquée ci-dessus, et Y' a la signification indiquée ci-dessus, pour Y dans laquelle les éventuelles fonctions réactives sont éventuellement protégées par des groupements protecteurs, pour obtenir des produits de formule (IX) :

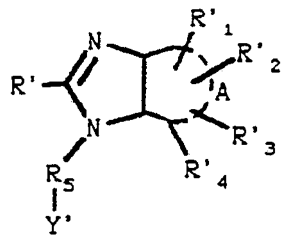

(IX)

dans laquelle R′, $R_1$′, $R_2$′, $R_3$′, $R_4$′, $R_5$ et Y′ ont les significations indiquées ci-dessus, soit l'on fait réagir un composé de formule (VI) :

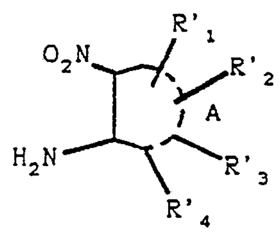

(VI)

dans laquelle $R_1$′, $R_2$′, $R_3$′ et $R_4$′ ont les significations indiquées ci-dessus, respectivement pour $R_1$, $R_2$, $R_3$ et $R_4$ dans lesquelles les éventuelles fonctions réactives sont éventuellement protégées par des groupements protecteurs,
ou bien avec le composé de formule (II′) :

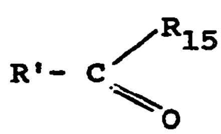

(II')

dans laquelle R′ et $R_{15}$ ont les significations indiquées ci-dessus, pour obtenir le produit de formule (X) :

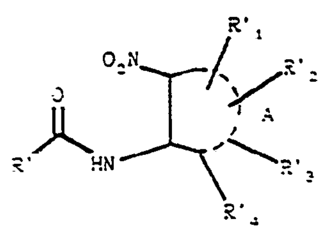

(X)

dans laquelle R′, $R_1$′, $R_2$′, $R_3$′ et $R_4$′ ont les significations précédentes, produit de formule (X) soit, que l'on réduit en produit de formule (X′) :

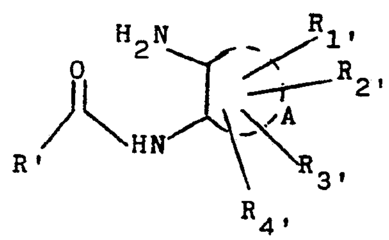

(X')

que l'on cyclise en produit de formule (IV) tel que défini ci-dessus que l'on traite ainsi qu'il est indiqué ci-dessus pour obtenir un produit de formule (IX) soit que l'on fait réagir le produit de formule (X) avec le composé de formule (V) telle que définie ci-dessus, pour obtenir un produit de formule (XI) :

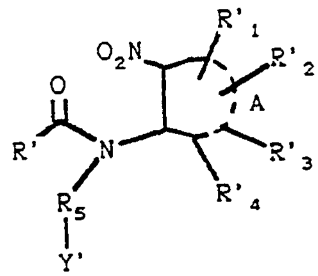

(XI)

dans laquelle R', $R_1$', $R_2$', $R_3$', $R_4$', $R_5$ et Y' ont les significations précédentes, que l'on soumet à une réaction de réduction sélective de la fonction nitro, pour obtenir le produit de formule (XII) :

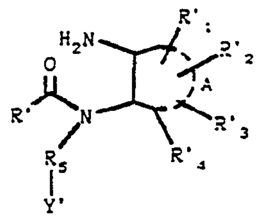

(XII)

dans laquelle R', $R_1$', $R_2$', $R_3$', $R_4$', $R_5$' et Y' ont les significations précédentes, que l'on soumet à une réaction de cyclisation pour obtenir des produits de formule (IX) telle que définie ci-dessus,
ou bien l'on fait réagir le composé de formule (VI) avec le composé de formule (V) telle que définie ci-dessus, pour obtenir des produits de formule (VII) :

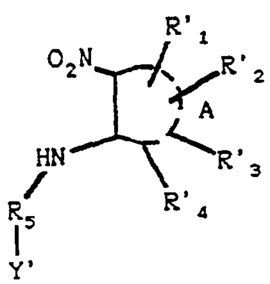

(VII)

dans laquelle $R_1$', $R_2$', $R_3$', $R_4$', $R_5$' et Y' ont les significations indiquées ci-dessus, que :
ou bien l'on fait réagir avec le composé de formule (II') tel que défini ci-dessus, pour obtenir un produit de formule (XI) tel que défini ci-dessus que l'on traite ensuite comme indiqué ci-dessus,
ou bien l'on soumet à une réaction de réduction du radical nitro en radical amino pour obtenir des produits de formule (VIII) :

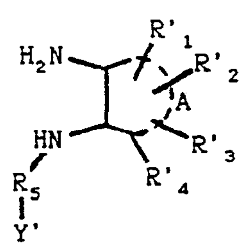

(VIII)

dans laquelle $R_1$', $R_2$', $R_3$', $R_4$', $R_5$' et Y' ont les significations indiquées ci-dessus, que l'on fait réagir avec le produit de formule (II) telle que définie ci-dessus, pour obtenir un produit de formule (IX) telle que définie ci-dessus, produit de formule (IX) que l'on traite, si désiré et si nécessaire, à l'une ou plusieurs des réactions suivantes, dans un ordre quelconque :

– une réaction d'élimination des groupements protecteurs que peuvent porter les fonctions réactives protégées,

– une réaction de salification par un acide minéral ou organique ou par une base pour obtenir le sel correspondant,

– une réaction d'estérification ou salification de fonction acide,

– une réaction de saponification de fonction ester en fonction acide,

– une réaction de transformation de fonction alkoxy en fonction hydroxyle,

– une réaction de transformation de la fonction cyano en fonction acide,

– une réaction de réduction de la fonction carboxy en fonction alcool,

– une réaction de substitution de fonction hydroxyle ou mercapto par un atome d'halogène,

– une réaction de substitution sur un atome d'halogène,

– une réaction de dédoublement des formes racémiques en produits dédoublés,

lesdits produits de formule (I) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :

Le produit de formule (IV) peut être obtenu par addition du produit de formule (II) sur une fonction amine libre du produit de formule (III) et cyclisation sur la deuxième fonction amine libre du produit ainsi formé.

Le produit de formule (IV) peut ainsi être obtenu par action du composé de formule (II) sur le composé de formule (III) dans diverses conditions réactionnelles notamment le composé de formule (II) peut être condensé sur le composé de formule (III) de préférence dans un solvant organique tel que par exemple le tétrahydrofuranne ou le diméthylformamide au reflux ou à une température comprise environ entre 20°C et 200°C.

Le composé de formule (II) peut être, par exemple, quand X représente =NH, le pentanimidoate d'éthyle et quand X représente =O, par exemple, l'acide valérique.

Le composé de formule (II) qui peut être par exemple tel que X représente un atome d'oxygène et $R_{15}$ représente un radical hydroxyle et peut par exemple représenter l'acide valérique peut encore êre mis en présence du composé de formule (III), qui peut être par exemple un dérivé de la pyridine, à une température d'environ 170°C de préférence sous agitation pendant environ 18 heures.

Les produits de formule (IV) ainsi obtenus peuvent être condensés avec un composé de formule (V) pour obtenir des produits de formule (IX).

La réaction de condensation des produits de formule (IV) avec un composé de formule (V) dans laquelle l'atome d'halogène représente de préférence un atome de brome peut être réalisée, par exemple, dans un solvant organique tel que le diméthylformamide ou le tétrahydrofuranne : ainsi le dérivé halogéné peut être condensé sur l'anion de l'imidazole de formule (IV) préparé par exemple par action d'une base forte telle que l'hydrure de sodium ou de potassium ou encore d'un alcoolate de sodium ou de potassium tel que par exemple le méthylate de sodium ou le carbonate de potassium dans le diméthylformamide.

La réaction d'addition du composé de formule (II') sur la fonction amine libre du composé de formule (VI) pour obtenir les produits de formule (X) peut être réalisée par simple chauffage à une température d'environ 120°C à 170°C : le composé de formule (II') peut être par exemple l'acide valérique, utilisé alors de préférence en excés par rapport au composé de formule (VI).

La réaction d'addition du composé de formule (V) sur la fonction amine libre du composé de formule (X) pour obtenir les produits de formule (XI) peut être réalisée à la température ambiante ou par chauffage à une température d'environ 20°C à 150°C, de préférence en présence d'une base telle que par exemple la triéthylamine, la soude, le méthylate ou éthylate de sodium ou encore l'hydrure de sodium dans un solvant tel que par exemple le tétrahydrofuranne ou le diméthylformamide.

La réduction du radical nitro des produits de formule (XI) en radical amino pour obtenir les produits de formule (XII) ainsi que la réduction des produits de formule (X) en produits de formule (X') peut être réalisée selon les méthodes usuelles connues de l'homme de métier notamment par une hydrogénation catalytique en présence d'hydroxyde de palladium dans un solvant tel que par exemple l'éthanol ou par du zinc dans un solvant tel que par exemple l'acide acétique en présence d'acétate de sodium ou encore par du borohydrure de sodium.

La réaction de cyclisation du produit de formule (XII) pour obtenir des produits de formule (IX) peut être réalisée par simple chauffage ou en présence d'un catalyseur tel que par exemple le chlorure de thionyle, le pentachlorure de phosphore ou encore l'anhydride phosphorique dans un solvant tel que par exemple le tétrahydrofuranne ou le diméthylformamide.

La réaction d'addition du composé de formule (V) sur la fonction amine libre du composé de formule (VI) pour obtenir les produits de formule (VII) peut être réalisée dans les mêmes conditions que celles décrites ci-dessus pour l'addition du composé de formule (V) sur les produits de formule (IV).

La réaction du produit de formule (VII) avec le composé de formule (II') est effectuée dans les mêmes conditions que la réaction du produit de formule (VI) avec le composé de formule (II').

La réduction du radical nitro des produits de formule (VII) en radical amino pour obtenir les produits de formule (VIII) peut être réalisée selon les méthodes usuelles connues de l'homme de métier notamment dans les mêmes conditions que celles décrites ci-dessus pour la réduction du radical nitro des produits de formule (XI) en radical amino pour obtenir les produits de formule (XII).

La réaction d'addition du composé de formule (II) sur le radical amine libre des produits de formule (VIII) suivie de la cyclisation des produits ainsi obtenus peut être réalisée dans les mêmes conditions que celles décrites ci-dessus pour l'addition du composé de formule (II) sur le composé de formule (III).

Ainsi qu'il est indiqué ci-dessus, les produits de formule (IX) peuvent donc être obtenus :

– soit par réaction des produits de formule (IV) avec un composé de formule (V),

– soit par une réaction de cyclisation des produits de formule (XII),

– soit par réaction de produit de formule (VIII) avec le composé de formule (II).

Selon les valeurs de R′, $R_1$′, $R_2$′, $R_3$′, $R_4$′ et Y′, les produits de formule (IX) constituent ou non des produits de formule (I).

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :

– les groupements hydroxyle peuvent être protégés par exemple par les radicaux alkyle, triméthylsilyle, dihydropyranne, méthoxyméthyle ou tétrahydropyrannyle,

– les groupements amino peuvent être protégés par exemple par les radicaux acétyle, trityle, benzyle, tert-butoxycarbonyle, phtalimido ou d'autres radicaux connus dans la chimie des peptides,

– les groupements acyles tel que le groupement formyle peuvent être protégés par exemple sous forme de cétals cycliques ou non cycliques tels que le diméthyl ou diéthylcétal ou l'éthylène dioxycétal,

– les fonctions acide des produits décrits ci-dessus peuvent être, si désiré, amidifiées par une amine primaire ou secondaire par exemple en présence de chorure de méthylène dans du chlorhydrate de 1-éthyl-3-(diméthylaminopropyl) carbodiimide à la température ambiante :

– les fonctions acide peuvent être protégés par exemple sous forme d'esters formés avec les esters facilement clivables tels que les esters benzyliques ou ter butyliques ou des esters connus dans la chimie des peptides.

L'élimination de ces groupements protecteurs est effectuée dans les conditions usuelles connues de l'homme de métier notamment l'hydrolyse acide effectuée avec un acide tel que l'acide chlorhydrique, benzène sulfonique ou para-toluène sulfonique, formique ou trifluoroacétique.

Le groupement phtalimido est éliminé par l'hydrazine. On trouvera une liste de différents groupements protecteurs utilisables par exemple dans le brevet BF 2 499 995.

Les produits décrits ci-dessus peuvent, si désiré, faire l'objet de réactions de salification par un acide minéral ou organique selon les méthodes usuelles connues de l'homme de métier.

Les produits décrits ci-dessus peuvent, si désiré, faire l'objet, sur les éventuelles fonctions carboxy, de réactions de salification par une base minérale ou organique ou d'estérification : ces réactions d'estérification et de salification peuvent être réalisées selon les méthodes usuelles connues de l'homme de métier.

Les éventuelles fonctions carboxy estérifiées des produits décrits ci-dessus peuvent être, si désiré, réduites en fonction alcool par les méthodes connues de l'homme de métier et notamment par de l'hydrure de lithium et d'aluminium dans un solvant tel que par exemple le tétrahydrofuranne ou encore le dioxanne ou l'éther éthylique.

Les éventuelles transformations de fonctions ester en fonction acide des produits décrits ci-dessus peuvent être, si désiré, réalisées dans les conditions usuelles connues de l'homme de métier notamment par hydrolyse acide ou alcaline par exemple par de la soude ou de la potasse en milieu alcoolique tel que, par exemple, dans du méthanol ou encore par de l'acide chlorhydrique ou sulfurique.

Les éventuelles fonctions cyano des produits décrits ci-dessus peuvent être, si désiré, transformées en fonction acide dans les conditions usuelles connues de l'homme de métier par exemple par une hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions alkoxy telles que notamment méthoxy des produits décrits ci-dessus peuvent être, si désiré, transformées en fonction alcool dans les conditions usuelles connues de l'homme de métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.

Les éventuelles réactions de substitution par un atome d'halogène et notamment les réactions de substitution de fonction hydroxyle ou mercapto par un atome d'halogène peuvent être, si désiré, réalisées dans les conditions usuelles connues de l'homme de métier telles que, par exemple par le chlorure de thionyle, le pentachlorure de phosphore ($PCl_5$), l'oxychlorure de phosphore $POCl_3$ dans un solvant tel que l'éther, le chlorure de méthylène ou le tétrahydrofuranne en présence ou non d'une base telle que la pyridine, ou par un tétrahalogène de méthane tel que le tétrachlorure ou le tétrabromure et la triphénylphosphine.

Les éventuelles formes optiquement actives des produits de formule (I) peuvent être préparées par dédoublement des racémiques selon les méthodes usuelles.

L'invention a également pour objet un procédé de préparation de produits de formule ($I_c$) :

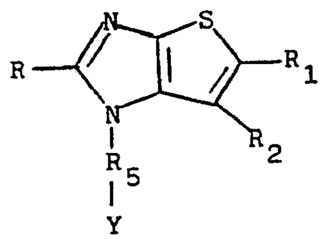

($I_C$)

dans laquelle R, $R_1$, $R_2$, $R_5$ et Y ont les significations indiquées ci-dessus caractérisé en ce que :
soit, l'on soumet un composé de formule (XIII) :

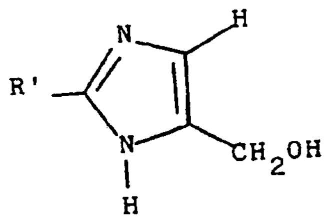

(XIII)

dans laquelle R' a la signification indiquée ci-dessus pour R dans laquelle les fonctions éventuellement réactives sont éventuellement protégées à une réaction d'halogénation pour obtenir un composé de formule (XIV) :

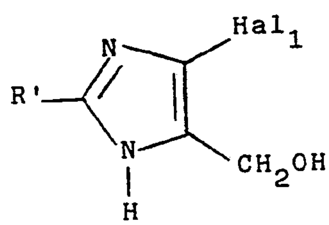

(XIV)

dans laquelle R' a la signification indiquée ci-dessus, et $Hal_1$ représente un atome d'halogène, de préférence le brome, que l'on soumet à une réaction d'oxydation pour obtenir le composé de formule (XV) :

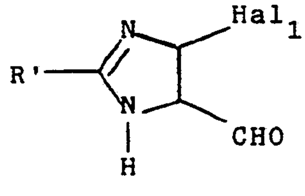

(XV)

dans laquelle R' et $Hal_1$ ont les significations indiquées ci-dessus, que l'on fait réagir avec un composé de formule (V) :

$$Hal\text{-}R_5\text{-}Y' \qquad (V)$$

dans laquelle Hal, $R_5$ et Y' ont les significations indiquées ci-dessus pour obtenir un composé de formule (XVI) :

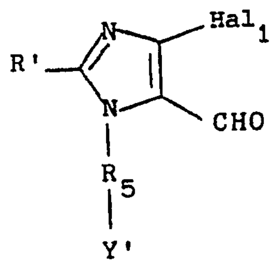

(XVI)

dans laquelle $Hal_1$, R', $R_5$ et Y' ont les significations indiquées ci-dessus que l'on fait réagir avec un composé de formule (XVII) :

$HS\text{-}CH_2\text{-}R_1'$ (XVII)

dans laquelle $R_1'$ a la signification indiquée ci-dessus, pour $R_1$ dans laquelle les fonctions éventuellement réactives sont éventuellement protégées pour obtenir après cyclisation un composé de formule ($I'_c$) :

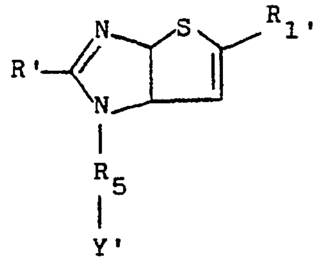

($I'_c$)

correspondant aux produits de formule ($I_c$) dans laquelle $R_2$ représente un atome d'hydrogène,
<u>soit</u>, l'on fait réagir un composé de formule (XVIII) :

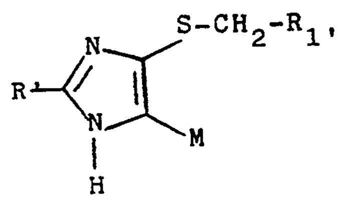

(XVIII)

dans laquelle R' et $R_1'$ ont les significations indiquées ci-dessus dans lesquelles les fonctions éventuellement réactives sont éventuellement protégées,
et M représente le radical cyano ou le radical

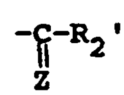

dans dans lequel $R_2'$ a la signification indiquée ci-dessus pour $R_2$ dans laquelle les éventuelles fonctions réactives sont éventuellement protégées et Z représente l'atome d'oxygène ou l'atome de soufre, avec le composé de formule (V) tel que défini ci-dessus pour obtenir le produit de formule (XIX) :

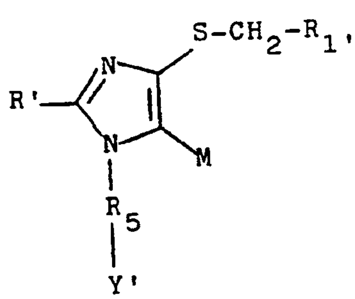

(XIX)

dans laquelle R′, $R_1$′, $R_5$′ et Y′ ont les significations indiquées ci-dessus, que l'on soumet à une réaction de cyclisation pour obtenir le composé de formule ($I''_c$) :

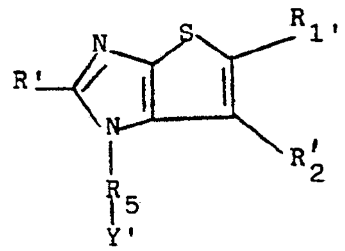

($I''_c$)

dans laquelle R′, $R_1$′, $R'_2$, $R_5$ et Y′ ont les significations indiquées ci-dessus et $R_2''$ a la signification indiquée ci-dessus pour $R_2'$ produits de formule ($I_c'$) et ($I_c''$) que l'on traite, si désire et si nécessaire, à l'une ou plusieurs des réactions suivantes, dans un ordre quelconque :

- une réaction d'élimination des groupements protecteurs que peuvent porter les fonctions réactives protégées,
- une réaction de salification par un acide minéral ou organique ou par une base pour obtenir le sel correspondant,
- une réaction d'estérification ou salification de fonction acide,
- une réaction de saponification de fonction ester en fonction acide,
- une réaction de transformation de fonction alkoxy en fonction hydroxyle,
- une réaction de transformation de la fonction cyano en fonction acide,
- une réaction de réduction de la fonction carboxy en fonction alcool,
- une réaction de substitution de fonction hydroxyle ou mercapto par un atome d'halogène,
- une réaction de substitution sur un atome d'halogène,
- une réaction de dédoublement des formes racémiques en produits dédoublés,

lesdits produits de formule ($I_c$) ainsi obtenus étant sous toutes les formes isomères possibles racémiques, énantiomères et diastéréoisomères.

Les étapes du procédé indiqué ci-dessus peuvent être réalisées, le cas échéant, selon les méthodes usuelles connues de l'homme de métier.

Dans des conditions préférentielles de mise en oeuvre de l'invention, le procédé ci-dessus peut être réalisé de la manière suivante :

La réaction d'halogénation du composé de formule (XIII) en composé de formule (XIV) peut être réalisée selon les méthodes usuelles connues de l'homme de métier et par exemple en présence d'un agent halogénant tel que le N-bromo succinimide dans un solvant tel que le dioxanne ou un alcool tel que l'éthanol ou le N-halo-succinimide dans le dioxanne ou ainsi qu'il est indiqué par exemple dans la demande de brevet EP 0253.310.

La réaction d'oxydation du composé de formule (XIV) en composé de formule (XV), soit d'une fonction alcool en fonction aldéhyde, peut être réalisée, par exemple, par action du dioxyde de manganèse dans un solvant tel que le dioxanne ou le dichlorométhane ou le pyridinium chlorochromate ou pyridinium dichromate par exemple dans le dichlorométhane ou le diméthyl sulfoxyde/chlorure d'oxalyle par exemple dans le dichlorométhane.

La réaction d'addition du composé de formule (V) sur le composé de formule (XV) pour obtenir un composé de formule (XVI) peut être réalisée par exemple ainsi qu'il est indiqué ci-dessus dans la réaction d'addition de ce composé de formule (V) avec le composé de formule (IV) pour obtenir un produit de formule (IX), soit, dans un solvant tel que par exemple le diméthylformamide en présence de bicarbonate de potassium ou de sodium ou encore le carbonate de sodium ou le carbonate de potassium dans le diméthylformamide ou le diméthylsulfoxyde, ou la soude ou la potasse dans le diméthylformamide, ou un alcoolate alcalin comme le méthylate ou l'éthylate de sodium ou de potassium dans le diméthylformamide ou le tétrahydrofuranne, ou par exemple ainsi qu'il est indiqué dans la demande européenne EP 0253.310.

La réaction d'addition du composé de formule (XVII) sur le composé de formule (XVI) suivie de cyclisation pour obtenir un composé de formule ($I_c'$) peut être réalisée, par exemple, en présence d'un alcoolate de sodium ou de potassium tel que l'éthylate de sodium dans un solvant tel que par exemple un alcool tel que le méthanol ou l'éthanol.

Les composés de formule ($I_c'$) correspondent à des produits de formule (I) dans laquelle $R_2$ représente un atome d'hydrogène.

La réaction d'addition du composé de formule (V) sur le composé de formule (XVIII) pour obtenir un

composé de formule (XIX) peut être réalisée, par exemple, dans les mêmes conditions que celles indiquées ci-dessus pour l'addition du composé de formule (V) avec le composé de formule (XV).

La réaction de cyclisation du composé de formule (XIX) en composé de formule ($I_c$") peut être réalisée par exemple dans une solution de (bis triméthyl silyl) amide de lithium ou de sodium, d'hydrure de sodium ou de potassium ou encore de diisopropyl amidure de lithium dans un solvant tel que le tétrahydrofuranne, le diméthylformamide ou le diméthoxyéthane.

Les composés de formule ($I_c$") peuvent représenter l'ensemble des produits de formule (I) telle que définie ci- dessus lorsque le radical M dans les composés de formule (XVIII) représente le radical

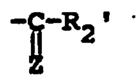

tel que défini ci-dessus

Dans le cas où le radical M dans les composés de formule (XVIII) représente le radical cyano, on obtient des produits de formule ($I_c$") représentant des produits de formule (I) telle que définie ci-dessus dans laquelle $R_2$ représente un radical amino. Ce radical amino peut être éventuellement substitué par les méthodes usuelles connues de l'homme de métier en un dérivé de ce radical amino pour donner un produit de formule (I).

Les produits de formule ($I_c$') et ($I_c$") qui représentent des produits de formule (I) peuvent être soumis à l'une ou plusieurs des réactions indiquées ci-dessus qui peuvent être réalisées dans les conditions usuelles connues de l'homme de métier telles que, par exemple, dans les conditions définies ci-dessus pour les produits de formule (IX).

Les composés de formule (I) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits tels que définis par la formule (I) ci-dessus, lesdits produits de formule (I) étant sous toutes les formes isomères possibles racémiques ou optiquement actives, ainsi que les sels d'addition avec les acides minéraux ou organiques pharmaceutiquement acceptables desdits produits de formule (I).

L'invention a particulièrement pour objet à titre de médicaments, les produits de formule (I) telle que définie ci-dessus, et répondant à la formule ($I_a$) :

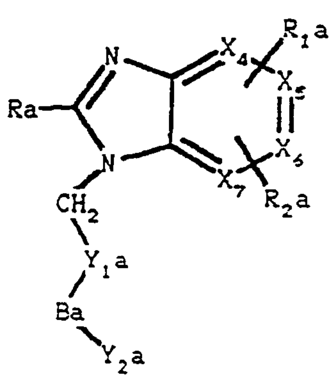

($I_a$)

dans laquelle :

– $X_4$, $X_5$, $X_6$ et $X_7$ sont tels que :

soit ils représentent tous un radical méthine =CH-,

soit l'un ou deux quelconques d'entre eux représentent un atome d'azote et les autres représentent un radical méthine =CH-

– $R_a$ représente un radical n-butyle ou butényle,

– $R_{1a}$ et $R_{2a}$, identiques ou différents, sont choisis dans le groupe formé par :

. l'atome d'hydrogène,

. les atomes d'halogène,

. le radical hydroxyle, le radical mercapto,

. les radicaux alkoxy linéaires ou ramifiés renfermant au plus 6 atomes de carbone,

. les radicaux alkyle, alkényle, alkynyle et alkylthio linéaires ou ramifiés renfermant au plus 6 atomes de carbone et les radicaux aryle, arylalkyle ou arylalkényle dans lesquels les radicaux alkyle et alkényle, linéaires ou ramifiés, renfermant au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alcoxy, alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkyle, alkoxy ou alkylthio renfermant au plus 6 atomes de carbone, mercapto, acyl, acyloxy, tétrazolyle,

. le radical carboxy libre,

. les radicaux carboxy estérifié par un radical alkyle linéaire ou ramifié renfermant au plus 4 atomes de carbone,

– $Y_{1a}$ représente un radical phényle,

– $B_a$ représente une simple liaison ou un radical -CO-NH-,

– $Y_{2a}$ est tel que :

soit, si $B_a$ représente une simple liaison ou un radical -CO-NH-, $Y_{2a}$ représente un radical phényle éventuellement substitué par un ou plusieurs radicaux choisis parmi les valeurs de $R_{1a}$ et $R_{2a}$,

soit, si $B_a$ représente une simple liaison, $Y_{2a}$ représente un radical cyano ou carboxy libre, salifié ou estérifié, ou tétrazolyle étant entendu que si $B_a$ représente une simple liaison alors l'un au moins de $X_4$, $X_5$, $X_6$, et $X_7$ ne représente pas un radical méthine, lesdits produits de formule (I), étant sous toutes les formes isomères possibles racémiques, énantiomères et diastéréoisomères, ainsi que les sels d'addition avec les acides minéraux et organiques ou avec les bases minérales et organiques desdits produits de formule (I), ainsi que les sels d'addition avec les acides minéraux et organiques ou avec les bases minérales et organiques pharmaceutiquement acceptables desdits produits de formule (I).

L'invention a plus particulièrement pour objet à titre de médicaments les produits de formule (I) telle que définie ci-dessus et répondant à la formule ($I_a$) dans laquelle $X_4$, $X_5$, $X_6$, $X_7$, $R_a$, $R_{1a}$, et $R_{2a}$ ont les significations indiquées ci-dessus et

– $Y_{1a}$ représente un radical phényle,

– $B_a$ représente une simple liaison ou un radical -CO-NH-,

– $Y_{2a}$ est tel que :

soit, si $B_a$ représente une simple liaison ou un radical -CO-NH-, $Y_{2a}$ représente un radical phényle éventuellement substitué par un radical carboxy libre, salifié ou estérifié, un radical tétrazolyle, tétrazolylalkyle ou tétrazolylcarbamoyle, dans lesquels le radical tétrazolyle est éventuellement substitué par un radical alkyle, alkényle, arylalkyle ou alcoxy alkyle,

soit, si $B_a$ représente une simple liaison, $Y_{2a}$ représente un radical cyano ou carboxy libre, salifié ou estérifié ou tétrazolyle étant entendu que si $B_a$ représente une simple liaison alors l'un au moins de $X_4$, $X_5$, $X_6$ et $X_7$ ne représente pas un radical méthine, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I), ainsi que les sels d'addition avec les acides minéraux et organiques ou avec les bases minérales et organiques pharmaceutiquement acceptables desdits produits de formule (I).

L'invention a tout particulièrement pour objet à titre de médicaments, les produits de formule (I) telle que définie ci-dessus, dans laquelle :

A représente un radical phényle, naphtyle, pyridyle, pyrimidinyle ou thiényle,

R représente un radical n-butyle ou butèn-1-yle,

$R_1$, $R_2$, $R_3$ et $R_4$ sont tels que deux d'entre eux représentent un atome d'hydrogène et les deux autres, identiques ou différents, représentent un atome d'hydrogène, un radical hydroxyle, un radical alkyle renfermant au plus 4 atomes de carbone, un radical carboxy libre ou estérifié par un radical alkyle linéaire ou ramifié renfermant au plus 4 atomes de carbone,

$R_5$ représente un radical méthylène,

et Y représente le radical $-Y_1$-B-$Y_2$ dans lequel $Y_1$ représente un radical phényle, B représente une liaison simple carbonecarbone ou le radical -CO-NH- et $Y_2$ représente un radical cyano, carboxy libre ou estérifié par un radical alkyle linéaire ou ramifié renfermant au plus 4 atomes de carbone, un radical indolyle ou un radical phényle éventuellement substitué par un radical carboxy libre, salifié ou estérifié, un radical tétrazolyle, tétrazolylalkyle, tétrazolylcarbamoyle, dans lesquels le radical tétrazolyle est éventuellement substitué par un radical alkyle, alkényle ou alcoxyalkyle, ou par un radical $-(CH_2)_p-SO_2-X_a-R_{14a}$ dans lequel p représente les valeurs 0 et 1, $X_a$ représente les radicaux -NH-, NHCO-NH-, -NH-CO- ou une simple liaison et $R_{14a}$ représente un radical

méthyle, phényle ou benzyle, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I) ainsi que les sels d'addition avec les acides minéraux et organiques ou avec les bases minérales et organiques pharmaceutiquement acceptables desdits produits de formule (I).

L'invention a plus particulièrement pour objet les produits de formule (I) telle que définie ci-dessus et répondant à la formule ($I_b$) :

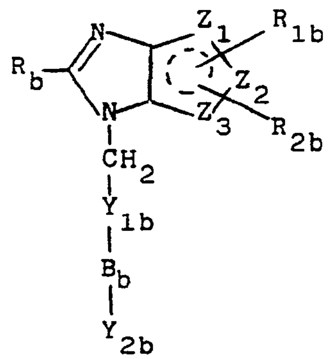

($I_b$)

dans laquelle $R_b$ représente un radical n-butyle ou butényle.

– $Z_1$, $Z_2$, $Z_3$ sont tels que :
l'un représente un atome de soufre
et les deux autres, identiques ou différents, représentent un radical méthine =CH-,

– $R_{1b}$ et $R_{2b}$, identiques ou différents, représentent :
. l'atome d'hydrogène,
. les atomes d'halogène,
. le radical hydroxyle, le radical mercapto,
. les radicaux alkoxy linéaires ou ramifiés renfermant au plus 6 atomes de carbone,
. les radicaux alkyle, alkényle, alkynyle et alkylthio, linéaires ou ramifiés renfermant au plus 6 atomes de carbone et les radicaux aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkoxy ou alkylthio renfermant au plus 4 atomes de carbone, mercapto, acyl, acyloxy,
. le radical carboxy libre,
. les radicaux carboxy estérifié par un radical alkyle linéaire ou ramifié renfermant au plus 4 atomes de carbone,

– $Y_{1b}$ représente un radical phényle,

– $B_b$ représente une simple liaison ou un radical -CO-NH-,

– $Y_{2b}$ est tel que :
soit, si $B_b$ représente une simple liaison ou un radical -CO-NH-, $Y_{2b}$ représente un radical phényle éventuellement substitué par un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, phényle ou benzyle,
soit, si $B_b$ représente une simple liaison, $Y_{2b}$ représente un radical cyano ou carboxy libre, salifié ou estérifié, et encore plus particulièrement les produits de formule ($I_b$) telle que définie ci-dessus répondant à la formule ($I_{b'}$) :

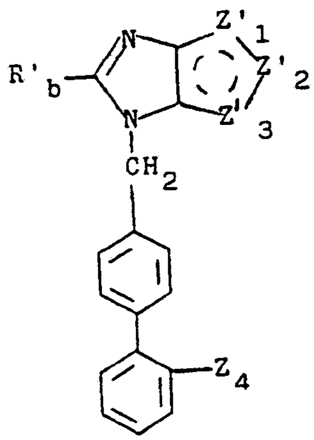

(Ib')

dans laquelle :

$R_b'$ représente un radical n-butyle,

$Z_1'$, $Z_2'$ et $Z_3'$ sont tels que :

l'un représente un atome de soufre,

et les deux autres, identiques ou différents, représentent un radical méthine =CH- éventuellement substitué par un radical hydroxyle ou un radical carboxy libre, salifié ou estérifié par un radical alkyle linéaire ou ramifié renfermant au plus 4 atomes de carbone,

et $Z_4$ représente un radical carboxy libre, salifié ou estérifié par un radical alkyle linéaire ou ramifié renfermant au plus 4 atomes de carbone ou un radical tétrazolyle, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I) ainsi que les sels d'addition avec les acides minéraux et organiques ou avec les bases minérales et organiques pharmaceutiquement acceptables desdits produits de formule (I).

L'invention a plus particulièrement pour objet, à titre de médicaments, les produits décrits ci-après dans les exemples et notamment les produits de formule (I) suivants :

l'acide 2-butyl 1-[(4-carboxyphényl) méthyl] 1H-benzimidazole-6-carboxylique

l'acide 4-[(2-butyl-1H-benzimidazol-1-yl) méthyl] benzoïque

l'acide 4-[(2-butyl-1H-benzimidazol-1-yl) méthyl] N-(1H-indol-4-yl) benzamide

l'acide 4-[(2-butyl-1H-naphth(2,3-d)imidazol-1-yl) méthyl] benzoïque

l'acide 4-[(2-butyl-5,6-diméthyl-1H-benzimidazol-1-yl) méthyl] benzoïque

l'acide 4-[(2-butyl-3H-imidazo(4,5-c)pyridin-3-yl) méthyl] benzoïque

l'acide 4'-((2-butyl-3H-imidazo(4,5b) pyridin-3-yl) méthyl) (1,1'-biphényl) 2-carboxylique

le 2-butyl-1-((2'-carboxy-(1,1'-byphényl)-4-yl) méthyl)-6-hydroxy-1H-thiéno-(2,3-d) imidazole-5-carboxylate de 1,1-diméthyléthyle

ainsi que leurs sels d'addition avec les acides minéraux ou organiques pharmaceutiquement acceptables.

Les médicaments, objet de l'invention, peuvent être utilisés dans le traitement de l'hypertension artérielle, des insuffisances cardiaques, des insuffisances rénales et dans la prévention des resténoses post-angioplastie.

Ils peuvent également être utilisés dans le traitement de certains désordres gastro-intestinaux, gynécologiques et en particulier pour un effet relaxant au niveau de l'utérus.

L'invention s'étend aux compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par

exemple, de 1 à 100 mg par jour chez l'adulte, par voie orale.

Les composés de départ de formules (II), (II'), (III), (V), (VI), (XIII), (XVII) et (XVIII) peuvent être disponibles dans le commerce ou peuvent être préparées selon les méthodes usuelles connues de l'homme de métier.

Certains composés de formule (III) peuvent être trouvés dans le commerce comme par exemple le 3,4 diaminobenzoate de méthyle que l'on peut trouver, par exemple, sous forme de produit commmercialisé par exemple par LANCASTER.

De nombreux exemples de préparation des composés de formule (III) sont décrits dans la littérature et des exemples en sont donnés notamment dans les références suivantes :

Bull. S.O.C. chim (1957), pp 2197-2201
Beil. 22, (2), 394
Beil. 22, (2), 395
Beil. 24, 324
Beil. 13, 270
Beil. 13, 207
Beil. 13, 179
Beil. 25, 481
Beil. 13, 1
Beil. 24, 469.

Parmi les composés de formule (II) et (II') se trouve par exemple l'acide valérique ou le pentanimidoate d'éthyle qui peut être préparé, par exemple, par action d'acide chlorhydrique gazeux dans l'éthanol sur le valéronitrile que l'on peut trouver, par exemple, sous forme de produit commmercialisé par LONZA.

Un exemple de préparation de ces composés de formule (II) est donné par exemple dans la référence : J.A.C.S. (1942), 64, pp 1827

Un procédé de préparation de certains produits de formule (V) telle que définie ci-dessus peut consister à soumettre le composé de formule (Va) :

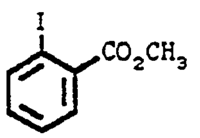

(Va)

soit le iodobenzoate de méthyle que l'on peut trouver, par exemple, sous forme de produit commmercialisé par JANSSEN, à l'action d'un composé de formule (Vb) :

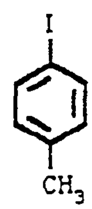

(Vb)

soit du iodotoluène que l'on peut trouver, par exemple, sous forme de produit commmercialisé par FLUKA, la réaction se réalisant par exemple en présence de cuivre en poudre à une température d'environ 100°C à 300°C, pour obtenir un produit de formule (Vc) :

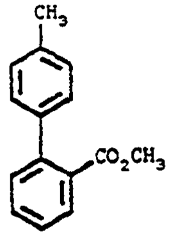

(Vc)

dont le radical carboxy estérifié peut, si désiré, être libéré du radical alkyle par les méthodes classiques connues

de l'homme de métier ou indiquées ci-dessus, par exemple d'hydrolyse acide ou alcaline, que l'on peut soumettre à une réaction de bromation sur le radical méthyle par les méthodes classiques connues de l'homme de métier par exemple par action du n-bromosuccinimide dans le tétrachlorure de carbone.

Des exemples de préparation de composés de formule (V) sont décrits dans la littérature et des exemples en sont donnés notamment dans le brevet US 4,880,804 et dans la demande de brevet européenne EP 0400.974.

Le composé de formule (VI) peut être par exemple l'orthonitroaniline que l'on peut trouver, par exemple, sous forme de produit commmercialisé par UCB.

Des exemples de préparation de composés de formule (VI) sont décrits dans la littérature et des exemples en sont donnés notamment dans les références suivantes :

Canadian journal of chemistry, 1977, 55, (10), pp 1653-1657

Beil. 22, (1), 631

Beil. 13, (2), 191

Beil. 14, (1), 583

Les composés de formule (XIII) peuvent être préparés ainsi qu'il est indiqué dans la demande de brevet européenne EP 0253.310.

Certains composés de formule (XVII) peuvent être trouvés dans le commerce comme par exemple le thioglycolate de méthyle, le benzylmercaptan ou encore le n-butylmercaptan ou le thioacétal que l'on peut trouver, par exemple, sous forme de produit commercialisé par exemple par ALDRICH.

De nombreux exemples de préparation des composés de formule (XVII) sont décrits dans la littérature et des exemples en sont donnés notamment dans la référence suivante :

ORGANICS SYNTHESIS COLL. VOL. 4, p. 296.

La présente invention a enfin pour objet à titre de produits industriels nouveaux, et notamment à titre de produits intermédiaires nécessaire à la préparation des produits de formule (I), les composés de formule (IV), (VII), (VIII), (XVI) (XVIII) et (XIX).

En plus des produits décrits dans les exemples qui illustrent l'invention sans toutefois la limiter, les produits suivants constituent des produits pouvant être obtenus dans le cadre de la présente invention : les substituants $R_a$, $X_4$, $X_5$, $X_6$, $X_7$, $R_{1a}$, $R_{2a}$, $Y_{1a}$, $B_a$ et $Y_{2a}$ sont ceux indiqués dans la formule ($I_a$).

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | -CH= | -CH= | H | OH | 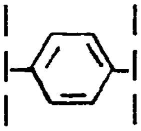 | - | COOH |
| " | " | " | " | " | OH en 4 | " | " | " | " |
| " | " | " | " | " | OH en 5 | " | " | " | " |
| " | " | " | " | " | OH en 6 | " | " | " | " |
| " | " | " | " | " | OH en 7 | " | " | " | " |
| " | " | " | " | " | $CH_2OH$ en 4 | " | " | " | " |
| " | " | " | " | " | $CH_2OH$ en 5 | " | " | " | " |
| " | " | " | " | " | $CH_2OH$ en 6 | " | " | " | " |
| " | " | " | " | " | $CH_2OH$ en 7 | " | " | " | " |
| " | " | " | " | " | COOH en 4 | " | " | " | " |
| " | " | " | " | " | COOH en 5 | " | " | " | " |
| " | " | " | " | " | COOH en 6 | " | " | " | " |
| " | " | " | " | " | COOH en 7 | " | " | " | " |
| " | " | " | " | " | COOMe en 4 | " | " | " | " |
| " | " | " | " | " | COOMe en 5 | " | " | " | " |
| " | " | " | " | " | COOMe en 6 | " | " | " | " |
| " | " | " | " | " | COOMe en 7 | " | " | " | " |
| " | " | " | " | " | $CH_3$ en 4 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | -CH= | -CH= | $CH_3$ en 5 | OH | 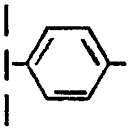 | - | COOH |
| " | " | " | " | " | $CH_3$ en 6 | " | " | " | " |
| " | " | " | " | " | $CH_3$ en 7 | " | " | " | " |
| " | " | " | " | " | $CH_2OH$ en 6 | H | " | " | " |
| " | " | " | " | " | $CH_2OH$ en 5 | " | " | " | " |
| " | " | " | " | " | $OCH_3$ en 6 | H | " | " | " |
| " | " | " | " | " | Cl en 6 | " | " | " | " |
| " | " | " | " | " | " | " | " | " | 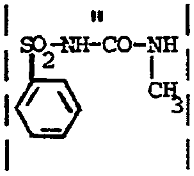 |
| " | " | " | " | " | COOH en 6 | " | " | " | " |
| " | " | " | " | " | COOH en 5 | " | " | " | " |
| " | " | " | " | " | $CH_2OH$ en 6 | " | " | " | " |
| " | " | " | " | " | $CH_2OH$ en 5 | " | " | " | " |
| " | " | " | " | " | OH en 6 | " | " | " | " |
| " | " | " | " | " | OH en 5 | " | " | " | " |
| " | " | " | " | " | $CO_2CH_3$ en 6 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| " | " | " | " | " | $CO_2CH_5$ en 5 | " | " | " | " |
| " | " | " | " | " | Cl en 6 | " | " | " | " |
| " | " | " | " | " | COOH en 6 | " | " | " | 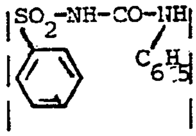 |
| " | " | " | " | " | COOH en 5 | " | " | " | " |
| " | " | " | " | " | $CH_2OH$ en 6 | H | " | " | " |
| " | " | " | " | " | $CH_2OH$ en 5 | " | " | " | " |
| " | " | " | " | " | OH en 5 | H | " | " | " |
| " | " | " | " | " | OH en 6 | H | " | " | " |
| " | " | " | " | " | $CO_2CH_3$ en 6 | " | " | " | " |
| " | " | " | " | " | $CO_2CH_3$ en 5 | " | " | " | " |
| " | " | " | " | " | Cl en 3 | " | " | " | " |
| " | " | " | " | " | COOH en 3 | " | " | " | 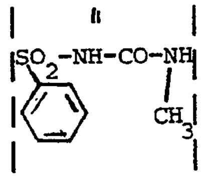 |
| " | " | " | " | " | COOH en 4 | " | " | " | " |
| " | " | " | " | " | $CH_2OH$ en 3 | " | " | " | " |
| " | " | " | " | " | $CH_2OH$ en 4 | " | " | " | " |
| " | " | " | " | " | OH en 3 | " | " | " | " |
| " | " | " | " | " | OH en 4 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| " | " | " | " | " | $CO_2CH_3$ en 3 | " | " | " | " |
| " | " | " | " | " | $CO_2CH_3$ en 4 | " | " | " | " |
| " | " | " | " | " | Cl en 3 | " | " | " | 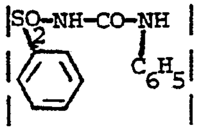 |
| " | " | " | " | " | COOH en 3 | " | " | " | " |
| " | " | " | " | " | COOH en 4 | " | " | " | " |
| " | " | " | " | " | $CH_2OH$ en 3 | " | " | " | " |
| " | " | " | " | " | $CH_2OH$ en 4 | " | " | " | " |
| " | " | " | " | " | OH en 3 | " | " | " | " |
| " | " | " | " | " | OH en 4 | " | " | " | " |
| " | " | " | " | " | $CO_2CH_3$ en 3 | " | " | " | " |
| " | " | " | " | " | $CO_2CH_3$ en 4 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nbu | N | -CH= | -CH= | -CH= | H | H | " | " | COOH |
| " | " | " | " | " | $CH_2OH$ en 7 | " | " | " | " |
| " | " | " | " | " | $CH_2OH$ en 5 | " | " | " | " |
| " | " | " | " | " | COOH en 7 | " | " | " | " |
| " | " | " | " | " | $CH_2OH$ en 6 | " | " | " | " |
| " | " | " | " | " | $CO_2CH_3$ en 6 | " | " | " | " |
| " | " | " | " | " | $CH_2OCH_3$ en 6 | " | " | " | " |
| " | " | " | " | " | COOH en 6 | " | " | " | " |
| " | " | " | " | " | COOH en 5 | " | " | " | " |
| " | " | " | " | " | $CO_2CH_3$ en 7 | " | " | " | " |
| " | " | " | " | " | $CH_2OCH_3$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | N | -CH= | -CH= | -CH= | H | H | 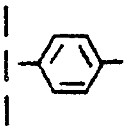 | - | 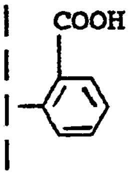 |
| " | " | " | " | " | $CH_2OH$ en 7 | " | " | " | " |
| " | " | " | " | " | $CH_2OH$ en 5 | " | " | " | " |
| " | " | " | " | " | COOH en 7 | " | " | " | " |
| " | " | " | " | " | $CH_2OH$ en 6 | " | " | " | " |
| " | " | " | " | " | $CO_2CH_3$ en 6 | " | " | " | " |
| " | " | " | " | " | $CH_2OCH_3$ en 6 | " | " | " | " |
| " | " | " | " | " | COOH en 6 | " | " | " | " |
| " | " | " | " | " | H | H | " | " | 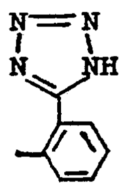 |
| " | " | " | " | " | $CH_2OH$ en 7 | " | " | " | " |
| " | " | " | " | " | $CH_2OH$ en 5 | " | " | " | " |
| " | " | " | " | " | COOH en 7 | " | " | " | " |
| " | " | " | " | " | $CH_2OH$ en 6 | " | " | " | " |
| " | " | " | " | " | $CO_2CH_3$ en 6 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | N | -CH= | -CH= | -CH= | $CO_2CH_3$ en 7 | H | | - | |
| " | " | " | " | " | $CH_2OCH_3$ en 7 | " | " | " | " |
| " | " | " | " | " | $CH_2OCH_3$ en 6 | " | " | " | " |
| " | " | " | " | " | COOH en 6 | " | " | " | " |
| nBu | -CH= | -CH= | N | -CH= | H | H | " | " | COOH |
| " | " | " | " | " | COOH en 5 | " | " | " | " |
| " | " | " | " | " | $CO_2CH_3$ en 5 | " | " | " | " |
| " | " | " | " | " | $CH_2OH$ en 5 | " | " | " | " |
| " | " | " | " | " | $CH_2OCH_3$ en 5 | " | " | " | " |
| " | " | " | " | " | $CH_2OH$ en 4 | " | " | " | " |
| " | " | " | " | " | COOH en 7 | " | " | " | " |
| " | " | " | " | " | $CO_2CH_3$ en 7 | " | " | " | " |
| " | " | " | " | " | $CH_2OH$ en 7 | " | " | " | " |
| " | " | " | " | " | $CH_2OCH_3$ en 7 | " | " | " | " |
| " | " | " | " | " | H | H | " | " | 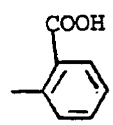 |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | N | -CH= | COOH en 5 | H | | - | 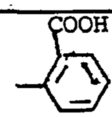 |
| " | " | " | " | " | $CO_2CH_3$ en 5 | " | " | " | " |
| " | " | " | " | " | $CH_2OH$ en 5 | " | " | " | " |
| " | " | " | " | " | $CH_2OCH_3$ en 5 | " | " | " | " |
| " | " | " | " | " | $CH_2OH$ en 4 | " | " | " | " |
| " | " | " | " | " | COOH en 7 | " | " | " | " |
| " | " | " | " | " | $CO_2CH_3$ en 7 | " | " | " | " |
| " | " | " | " | " | $CH_2OH$ en 7 | " | " | " | " |
| " | " | " | " | " | $CH_2OCH_3$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | N | -CH= | H | H | 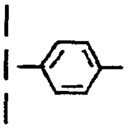 | - | 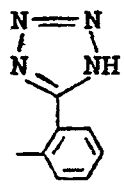 |
| " | " | " | " | " | COOH en 5 | " | " | " | " |
| " | " | " | " | " | $CO_2CH_3$ en 5 | " | " | " | " |
| " | " | " | " | " | $CH_2OH$ en 5 | " | " | " | " |
| " | " | " | " | " | $CH_2OCH_3$ en 5 | " | " | " | " |
| " | " | " | " | " | $CH_2OH$ en 4 | " | " | " | " |
| " | " | " | " | " | COOH en 7 | " | " | " | " |
| " | " | " | " | " | $CO_2CH_3$ en 7 | " | " | " | " |
| " | " | " | " | " | $CH_2OH$ en 7 | " | " | " | " |
| " | " | " | " | " | $CH_2OCH_3$ en 7 | " | " | " | " |
| " | " | " | " | " | COOH en 4 | " | " | " | 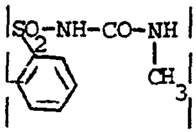 |
| " | " | " | " | " | COOH en 5 | " | " | " | " |
| " | " | " | " | " | $CH_2OH$ en 4 | " | " | " | " |
| " | " | " | " | " | $CH_2OH$ en 5 | " | " | " | " |
| " | " | " | " | " | OH en 4 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| " | " | " | " | " | OH en 5 | " | " | " | " |
| " | " | " | " | " | $CO_2CH_3$ en 4 | " | " | " | " |
| " | " | " | " | " | $CO_2CH_3$ en 5 | " | " | " | " |
| " | " | " | " | " | COOH en 4 | " | " | " | 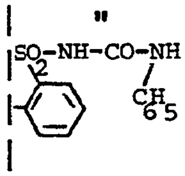 |
| " | " | " | " | " | COOH en 5 | " | " | " | " |
| " | " | " | " | " | $CH_2OH$ en 4 | " | " | " | " |
| " | " | " | " | " | $CH_2OH$ en 5 | " | " | " | " |
| " | " | " | " | " | OH en 4 | " | " | " | " |
| " | " | " | " | " | OH en 5 | " | " | " | " |
| " | " | " | " | " | $CO_2CH_3$ en 4 | " | " | " | 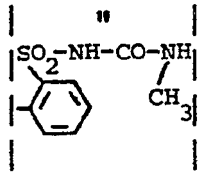 |
| " | " | " | " | " | $CO_2CH_3$ en 5 | " | " | " | " |
| " | " | " | " | " | COOH en 7 | " | " | " | " |
| " | " | " | " | " | $CH_2OH$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| " | " | " | " | " | OH en 7 | " | " | " | 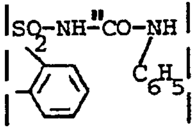 |
| " | " | " | " | " | $CO_2CH_3$ en 7 | " | " | " | |
| " | " | " | " | " | COOH en 7 | " | " | " | " |
| " | " | " | " | " | $CH_2OH$ en 7 | " | " | " | " |
| " | " | " | " | " | OH en 7 | " | " | " | " |
| " | " | " | " | " | $CO_2CH_3$ en 7 | " | " | " | " |
| nBu | -CH= | -CH= | -CH= | N | H | H | H | " | COOH |
| " | " | " | " | " | $CH_2OH$ en 6 | " | " | " | " |
| " | " | " | " | " | COOH en 6 | " | " | " | " |
| " | " | " | " | " | $CH_2OCH_3$ en 6 | " | " | " | " |
| " | " | " | " | " | $CO_2CH_3$ en 6 | " | " | " | " |
| " | " | " | " | " | $CH_2OH$ en 5 | " | " | " | " |
| " | " | " | " | " | COOH en 5 | " | " | " | " |
| " | " | " | " | " | $CH_2OCH_3$ en 5 | " | " | " | " |
| " | " | " | " | " | $CO_2CH_3$ en 5 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | -CH= | N | H | H | 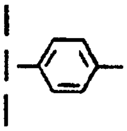 | - | 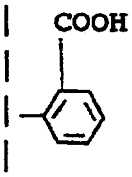 |
| " | " | " | " | " | $CH_2OH$ en 6 | " | " | " | " |
| " | " | " | " | " | COOH en 6 | " | " | " | " |
| " | " | " | " | " | $CH_2OCH_3$ en 6 | " | " | " | " |
| " | " | " | " | " | $CO_2CH_3$ en 6 | " | " | " | " |
| " | " | " | " | " | $CH_2OH$ en 5 | " | " | " | " |
| " | " | " | " | " | COOH en 5 | " | " | " | " |
| " | " | " | " | " | $CH_2OCH_3$ en 5 | " | " | " | " |
| " | " | " | " | " | $CO_2CH_3$ en 5 | " | " | " | " |
| " | " | " | " | " | H | H | " | " | 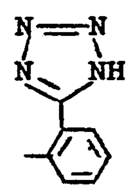 |
| " | " | " | " | " | $CH_2OH$ en 6 | " | " | " | " |
| " | " | " | " | " | COOH en 6 | " | " | " | " |
| " | " | " | " | " | $CH_2OCH_3$ en 6 | " | " | " | " |
| " | " | " | " | " | $CO_2CH_3$ en 6 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | -CH= | N | $CH_2OH$ en 5 | H | 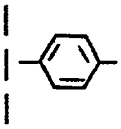 | - | 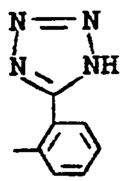 |
| " | " | " | " | " | COOH en 5 | " | " | " | " |
| " | " | " | " | " | $CH_2OCH_3$ en 5 | " | " | " | " |
| " | " | " | " | " | $CO_2CH_3$ en 5 | " | " | " | " |
| nBu | -CH= | N | -CH= | -CH= | H | H | " | " | COOH |
| " | " | " | " | " | COOH en 6 | " | " | " | " |
| " | " | " | " | " | $CO_2CH_3$ en 6 | " | " | " | " |
| " | " | " | " | " | $CH_2OH$ en 6 | " | " | " | " |
| " | " | " | " | " | $CH_2OCH_3$ en 6 | " | " | " | " |
| " | " | " | " | " | $CH_2OH$ en 4 | " | " | " | " |
| " | " | " | " | " | $CH_2OH$ en 7 | " | " | " | " |
| " | " | " | " | " | COOH en 7 | " | " | " | " |
| " | " | " | " | " | $CH_2OCH_3$ en 7 | " | " | " | " |
| " | " | " | " | " | $CO_2CH_3$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | N | -CH= | -CH= | H | H | 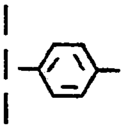 | - | 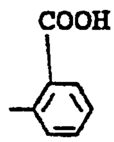 |
| " | " | " | " | " | COOH en 6 | " | " | " | " |
| " | " | " | " | " | $CO_2CH_3$ en 6 | " | " | " | " |
| " | " | " | " | " | $CH_2OH$ en 6 | " | " | " | " |
| " | " | " | " | " | $CH_2OCH_3$ en 6 | " | " | " | " |
| " | " | " | " | " | $CH_2OH$ en 4 | " | " | " | " |
| " | " | " | " | " | $CH_2OH$ en 7 | " | " | " | " |
| " | " | " | " | " | COOH en 7 | " | " | " | " |
| " | " | " | " | " | $CH_2OCH_3$ en 7 | " | " | " | " |
| " | " | " | " | " | $CO_2CH_3$ en 7 | " | " | " | " |
| " | " | " | " | " | H | H | " | " | 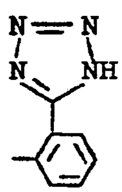 |
| " | " | " | " | " | COOH en 6 | " | " | " | " |
| " | " | " | " | " | $CO_2CH_3$ en 6 | " | " | " | " |
| " | " | " | " | " | $CH_2OH$ en 6 | " | " | " | " |
| " | " | " | " | " | $CH_2OCH_3$ en 6. | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | N | -CH= | -CH= | $CH_2OH$ en 4 | H | 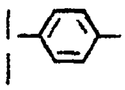 | - | 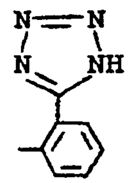 |
| " | " | " | " | " | $CH_2OH$ en 7 | " | " | " | " |
| " | " | " | " | " | COOH en 7 | " | " | " | " |
| " | " | " | " | " | $CH_2OCH_3$ en 7 | " | " | " | " |
| " | " | " | " | " | $CO_2CH_3$ en 7 | " | " | " | " |
| nBu | N | -CH= | N | -CH= | H | H | " | " | COOH |
| " | " | " | " | " | OH en 7 | " | " | " | " |
| " | " | " | " | " | OH en 5 | " | " | " | " |
| " | " | " | " | " | H | H | " | " | 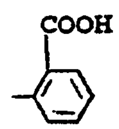 |
| " | " | " | " | " | OH en 7 | " | " | " | " |
| " | " | " | " | " | OH en 5 | " | " | " | " |
| " | " | " | " | " | H | H | " | " | 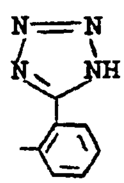 |
| " | " | " | " | " | OH en 7 | " | " | " | " |
| " | " | " | " | " | OH en 5 | " | " | " | " |
| " | " | " | " | " | COOH en 7 | " | " | " | " |
| " | " | " | " | " | $CH_2OH$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | N | -CH= | N | H | H | 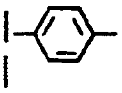 | - | COOH |
| " | " | " | " | " | OH en 4 | H | " | " | " |
| " | " | " | " | " | OH en 4 | OH en 6 | " | " | " |
| " | " | " | " | " | H | H | " | " | 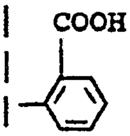 |
| " | " | " | " | " | OH en 4 | H | " | " | " |
| " | " | " | " | " | OH en 4 | OH en 6 | " | " | " |
| " | " | " | " | " | H | H | " | " | 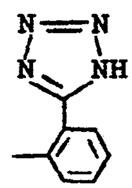 |
| " | " | " | " | " | OH en 4 | H | " | " | " |
| " | " | " | " | " | OH en 4 | OH en 6 | " | " | " |
| nBu | -CH= | N | N | -CH= | H | H | " | " | COOH |
| " | " | " | " | " | OH en 4 | " | " | " | " |
| " | " | " | " | " | $CH_2OH$ en 4 | " | " | " | " |
| " | " | " | " | " | COOH en 4 | " | " | " | " |
| " | " | " | " | " | $CO_2CH_3$ en 4 | " | " | " | " |
| " | " | " | " | " | OH en 7 | " | " | " | " |
| " | " | " | " | " | $CH_2OH$ en 7 | " | " | " | " |
| " | " | " | " | " | COOH en 7 | " | " | " | " |
| " | " | " | " | " | $CO_2CH_3$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | N | N | -CH= | H | H | 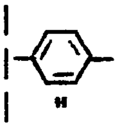 | - | 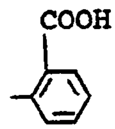 |
| " | " | " | " | " | OH en 4 | " | " | " | " |
| " | " | " | " | " | $CH_2OH$ en 4 | " | " | " | " |
| " | " | " | " | " | COOH en 4 | " | " | " | " |
| " | " | " | " | " | $CO_2CH_3$ en 4 | " | " | " | " |
| " | " | " | " | " | OH en 7 | " | " | " | " |
| " | " | " | " | " | $CH_2OH$ en 7 | " | " | " | " |
| " | " | " | " | " | COOH en 7 | " | " | " | " |
| " | " | " | " | " | $CO_2CH_3$ en 7 | " | " | " | " |
| " | " | " | " | " | H | H | " | " | 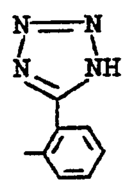 |
| " | " | " | " | " | OH en 4 | " | " | " | " |
| " | " | " | " | " | $CH_2OH$ en 4 | " | " | " | " |
| " | " | " | " | " | COOH en 4 | " | " | " | " |
| " | " | " | " | " | $CO_2CH_3$ en 4 | " | " | " | " |
| " | " | " | " | " | OH en 7 | " | " | " | " |
| " | " | " | " | " | $CH_2OH$ en 7 | " | " | " | " |
| " | " | " | " | " | COOH en 7 | " | " | " | " |
| " | " | " | " | " | $CO_2CH_3$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | N | N | -CH= | -CH= | H | H | 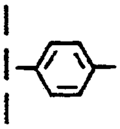 | - | COOH |
| " | " | " | " | " | OH en 6 | " | " | " | " |
| " | " | " | " | " | $CH_2OH$ en 6 | " | " | " | " |
| " | " | " | " | " | COOH en 6 | " | " | " | " |
| " | " | " | " | " | $CO_2CH_3$ en 6 | " | " | " | " |
| " | " | " | " | " | OH en 7 | " | " | " | " |
| " | " | " | " | " | $CH_2OH$ en 7 | " | " | " | " |
| " | " | " | " | " | COOH en 7 | " | " | - | " |
| " | " | " | " | " | $CO_2CH_3$ en 7 | " | " | " | " |
| " | " | " | " | " | H | H | " | " | 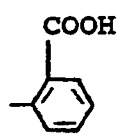 |
| " | " | " | " | " | OH en 6 | " | " | " | " |
| " | " | " | " | " | $CH_2OH$ en 6 | " | " | " | " |
| " | " | " | " | " | COOH en 6 | " | " | " | " |
| " | " | " | " | " | $CO_2CH_3$ en 6 | " | " | " | " |
| " | " | " | " | " | OH en 7 | " | " | " | " |
| " | " | " | " | " | $CH_2OH$ en 7 | " | " | " | " |
| " | " | " | " | " | COOH en 7 | " | " | " | " |
| " | " | " | " | " | $CO_2CH_3$ en 7 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | N | N | -CH= | -CH= | H | H | 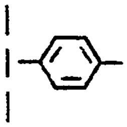 | - | 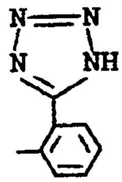 |
| " | " | " | " | " | OH en 6 | " | " | " | " |
| " | " | " | " | " | $CH_2OH$ en 6 | " | " | " | " |
| " | " | " | " | " | COOH en 6 | " | " | " | " |
| " | " | " | " | " | $CO_2CH_3$ en 6 | " | " | " | " |
| " | " | " | " | " | OH en 7 | " | " | " | " |
| " | " | " | " | " | $CH_2OH$ en 7 | " | " | " | " |
| " | " | " | " | " | COOH en 7 | " | " | " | " |
| " | " | " | " | " | $CO_2CH_3$ en 7 | " | " | " | " |
| nBu | -CH= | -CH= | N | N | H | H | " | " | COOH |
| " | " | " | " | " | OH en 4 | " | " | " | " |
| " | " | " | " | " | $CH_2OH$ en 4 | " | " | " | " |
| " | " | " | " | " | COOH en 4 | " | " | " | " |
| " | " | " | " | " | $CO_2CH_3$ en 4 | " | " | " | " |
| " | " | " | " | " | OH en 5 | " | " | " | " |
| " | " | " | " | " | $CH_2OH$ en 5 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | N | N | COOH en 5 | H | 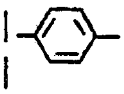 | - | COOH |
| " | " | " | " | " | $CO_2CH_3$ en 5 | " | " | " | " |
| " | " | " | " | " | H | H | " | " | 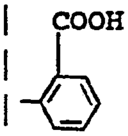 |
| " | " | " | " | " | OH en 4 | " | " | " | " |
| " | " | " | " | " | $CH_2OH$ en 4 | " | " | " | " |
| " | " | " | " | " | COOH en 4 | " | " | " | " |
| " | " | " | " | " | $CO_2CH_3$ en 4 | " | " | " | " |
| " | " | " | " | " | OH en 5 | " | " | " | " |
| " | " | " | " | " | $CH_2OH$ en 5 | " | " | " | " |
| " | " | " | " | " | COOH en 5 | " | " | " | " |
| " | " | " | " | " | $CO_2CH_3$ en 5 | " | " | " | " |
| " | " | " | " | " | H | H | " | " | 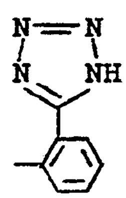 |
| " | " | " | " | " | OH en 4 | " | " | " | " |
| " | " | " | " | " | $CH_2OH$ en 4 | " | " | " | " |
| " | " | " | " | " | COOH en 4 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | N | N | $CO_2CH_3$ en 4 | H | 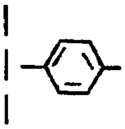 | - | 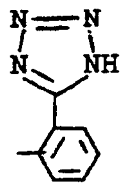 |
| " | " | " | " | " | OH en 5 | " | " | " | " |
| " | " | " | " | " | $CH_2OH$ en 5 | " | " | " | " |
| " | " | " | " | " | COOH en 5 | " | " | " | " |
| " | " | " | " | " | $CO_2CH_3$ en 5 | " | " | " | 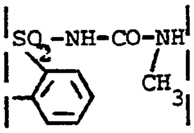 |
| " | " | " | " | " | COOH en 4 | " | " | " | " |
| " | " | " | " | " | COOH en 5 | " | " | " | " |
| " | " | " | " | " | $CH_2OH$ en 4 | " | " | " | " |
| " | " | " | " | " | $CH_2OH$ en 5 | " | " | " | " |
| " | " | " | " | " | OH en 4 | " | " | " | " |
| " | " | " | " | " | OH en 5 | " | " | " | " |
| " | " | " | " | " | $CO_2CH_3$ en 4 | " | " | " | " |
| " | " | " | " | " | $CO_2CH_3$ en 5 | " | " | " | 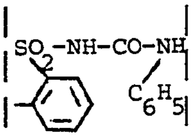 |
| " | " | " | " | " | COOH en 4 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | -CH= | -CH= | N | N | COOH en 5 | H | | - | 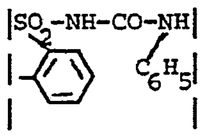 |
| " | " | " | " | " | $CH_2OH$ en 4 | " | " | " | " |
| " | " | " | " | " | $CH_2OH$ en 5 | " | " | " | " |
| " | " | " | " | " | OH en 4 | " | " | " | " |
| " | " | " | " | " | OH en 5 | " | " | " | " |
| " | " | " | " | " | $CO_2CH_3$ en 4 | " | " | " | " |
| " | " | " | " | " | $CO_2CH_3$ en 5 | " | " | " | " |
| nBu | N | -CH= | -CH= | N | H | H | " | " | COOH |
| " | " | " | " | " | OH en 5 | " | " | " | " |
| " | " | " | " | " | $CH_2OH$ en 5 | " | " | " | " |
| " | " | " | " | " | COOH en 5 | " | " | " | " |
| " | " | " | " | " | $CO_2CH_3$ en 5 | " | " | " | " |
| " | " | " | " | " | OH en 6 | " | " | " | " |
| " | " | " | " | " | $CH_2OH$ en 6 | " | " | " | " |
| " | " | " | " | " | COOH en 6 | " | " | " | " |
| " | " | " | " | " | $CO_2CH_3$ en 6 | " | " | " | " |
| " | " | " | " | " | H | H | " | " | 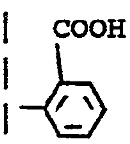 |
| " | " | " | " | " | OH en 5 | " | " | " | " |
| " | " | " | " | " | $CH_2OH$ en 5 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | N | -CH= | -CH= | N | COOH en 5 | H | 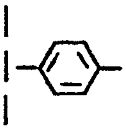 | - | 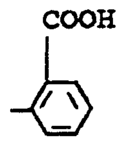 |
| " | " | " | " | " | $CO_2CH_3$ en 5 | " | " | " | " |
| " | " | " | " | " | OH en 6 | " | " | " | " |
| " | " | " | " | " | $CH_2OH$ en 6 | " | " | " | " |
| " | " | " | " | " | COOH en 6 | " | " | " | " |
| " | " | " | " | " | $CO_2CH_3$ en 6 | " | " | " | " |
| " | " | " | " | " | H | H | " | " | 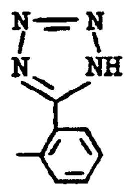 |
| " | " | " | " | " | OH en 5 | " | " | " | " |
| " | " | " | " | " | $CH_2OH$ en 5 | " | " | " | " |
| " | " | " | " | " | COOH en 5 | " | " | " | " |
| " | " | " | " | " | $CO_2CH_3$ en 5 | " | " | " | " |
| " | " | " | " | " | OH en 6 | " | " | " | " |
| " | " | " | " | " | $CH_2OH$ en 6 | " | " | " | " |
| " | " | " | " | " | COOH en 6 | " | " | " | " |
| " | " | " | " | " | $CO_2CH_3$ en 6 | " | " | " | " |

| $R_a$ | $X_4$ | $X_5$ | $X_6$ | $X_7$ | $R_{1a}$ | $R_{2a}$ | $Y_{1a}$ | $B_a$ | $Y_{2a}$ |
|---|---|---|---|---|---|---|---|---|---|
| nBu | N | -CH= | -CH= | N | H | H | 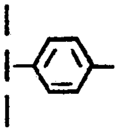 | - | 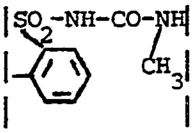 |
| " | " | " | " | " | COOH en 6 | " | " | " | " |
| " | " | " | " | " | COOH en 5 | " | " | " | " |
| " | " | " | " | " | $CH_2OH$ en 6 | " | " | " | " |
| " | " | " | " | " | $CH_2OH$ en 5 | " | " | " | " |
| " | " | " | " | " | OH en 6 | " | " | " | " |
| " | " | " | " | " | OH en 5 | " | " | " | " |
| " | " | " | " | " | $CO_2CH_3$ en 6 | " | " | " | " |
| " | " | " | " | " | $CO_2CH_3$ en 5 | " | " | " | " |
| | | | | | | | | | 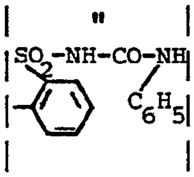 |
| " | " | " | " | " | COOH en 6 | " | " | " | " |
| " | " | " | " | " | COOH en 5 | " | " | " | " |
| " | " | " | " | " | $CH_2OH$ en 6 | " | " | " | " |
| " | " | " | " | " | $CH_2OH$ en 5 | " | " | " | " |
| " | " | " | " | " | OH en 6 | " | " | " | " |
| " | " | " | " | " | OH en 5 | " | " | " | " |
| " | " | " | " | " | $CO_2CH_3$ en 6 | " | " | " | " |
| " | " | " | " | " | $CO_2CH_3$ en 5 | " | " | " | " |

En plus des produits décrits dans les exemples qui illustrent l'invention sans toutefois la limiter, et des produits indiqués dans les tableaux ci-dessus répondant à la formule ($I_a$), les produits suivants constituent des

produits pouvant être obtenus dans le cadre de la présente invention : les substituants $R_b$, $Z_1$, $Z_2$, $Z_3$, $R_{1b}$, $R_{2b}$, $Y_{1b}$, $B_b$ et $Y_{2b}$ sont ceux indiqués dans la formule ($I_b$).

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| nBu | S | -CH= | -CH= | H | H | 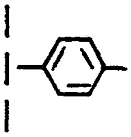 | - | 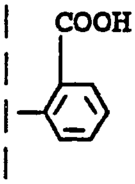 |
| " | " | " | " | $CH_2OH$ en 5 | " | " | " | " |
| " | " | " | " | COOH en 5 | " | " | " | " |
| " | " | " | " | $CH_2OH$ en 6 | " | " | " | " |
| " | " | " | " | $CO_2CH_3$ en 6 | " | " | " | " |
| " | " | " | " | $CH_2OCH_3$ en 6 | " | " | " | " |
| " | " | " | " | COOH en 6 | " | " | " | " |
| " | " | " | " | $CO_2CH_3$ en 5 | " | " | " | " |
| " | " | " | " | OH en 6 | " | " | " | " |
| " | " | " | " | OH en 5 | " | " | " | " |
| " | " | " | " | H | H | | - | COOH |
| " | " | " | " | $CH_2OH$ en 5 | " | " | " | " |
| " | " | " | " | COOH en 5 | " | " | " | " |
| " | " | " | " | $CH_2OH$ en 6 | " | " | " | " |
| " | " | " | " | $CO_2CH_3$ en 6 | " | " | " | " |
| " | " | " | " | $CH_2OCH_3$ en 6 | " | " | " | " |
| " | " | " | " | COOH en 6 | " | " | " | " |
| " | " | " | " | $CO_2CH_3$ en 6 | " | " | " | " |

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| " | " | " | " | $CO_2CH_3$ en 5 | " | " | " | " |
| " | " | " | " | OH en 6 | " | " | " | " |
| " | " | " | " | OH en 5 | " | " | " | " |
| nBu | S | -CH= | -CH= | $CH_2OCH_3$ en 6 | H | | - | 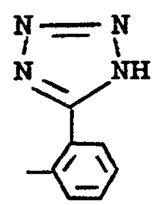 |
| " | " | " | " | COOH en 6 | " | " | " | " |
| " | " | " | " | H | H | " | " | " |
| " | " | " | " | OH en 5 | " | " | " | " |
| " | " | " | " | $CO_2CH_3$ en 5 | " | " | " | " |
| " | " | " | " | OH en 6 | " | " | " | " |
| " | " | " | " | $CH_2OH$ en 5 | " | " | " | " |
| " | " | " | " | COOH en 5 | " | " | " | " |
| " | " | " | " | $CH_2OH$ en 6 | " | " | " | " |
| " | " | " | " | $CO_2CH_3$ en 6 | " | " | " | 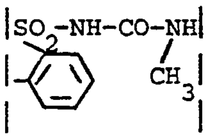 |
| " | " | " | " | COOH en 6 | " | " | " | " |
| " | " | " | " | COOH en 5 | " | " | " | " |

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| " | " | " | " | $CH_2OH$ en 6 | H | " | " | " |
| " | " | " | " | $CH_2OH$ en 5 | " | " | " | " |
| " | " | " | " | OH en 6 | " | " | " | " |
| " | " | " | " | OH en 5 | " | " | " | " |
| " | " | " | " | $CO_2CH_3$ en 6 | " | " | " | " |
| " | " | " | " | $CO_2CH_3$ en 5 | " | " | " | " |
| " | " | " | " | COOH en 6 | " | " | " | 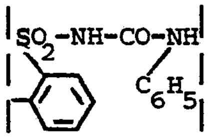 |
| " | " | " | " | COOH en 5 | " | " | " | " |
| " | " | " | " | $CH_2OH$ en 6 | " | " | " | " |
| " | " | " | " | $CH_2OH$ en 5 | " | " | " | " |
| " | " | " | " | OH en 6 | " | " | " | " |
| " | " | " | " | OH en 5 | " | " | " | " |
| " | " | " | " | $CO_2CH_3$ en 6 | " | " | " | " |
| " | " | " | " | $CO_2CH_3$ en 5 | " | " | " | " |
| nBu | -CH= | S | -CH= | H | H | | - | 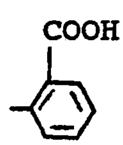 |
| " | " | " | " | OH en 4 | " | " | " | " |
| " | " | " | " | OH en 4 | OH en 6 | " | " | " |
| " | " | " | " | COOH en 6 | " | " | " | " |

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| " | " | " | " | $CO_2CH_3$ en 6 | " | " | " | " |
| " | " | " | " | $CH_2OH$ en 6 | " | " | " | " |
| " | " | " | " | $CH_2OCH_3$ en 6 | " | " | " | " |
| " | " | " | " | $CH_2OH$ en 4 | " | " | " | " |
| " | " | " | " | H | H | " | " | 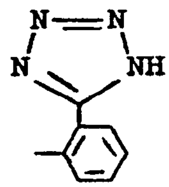 |
| " | " | " | " | COOH en 4 | " | " | " | |
| " | " | " | " | COOH en 6 | " | " | " | " |
| " | " | " | " | OH en 4 | " | " | " | " |
| " | " | " | " | $CO_2CH_3$ en 6 | " | " | " | " |
| " | " | " | " | OH en 4 | OH en 6 | " | " | " |
| " | " | " | " | $CH_2OH$ en 6 | " | " | " | " |
| " | " | " | " | $CH_2OCH_3$ en 6 | " | " | " | " |
| " | " | " | " | $CH_2OH$ en 4 | " | " | " | " |
| nBu | -CH= | S | -CH= | H | H | " | " | COOH |
| " | " | " | " | OH en 4 | " | " | " | " |
| " | " | " | " | OH en 4 | OH en 6 | " | " | " |
| " | " | " | " | COOH en 6 | H | " | " | " |
| " | " | " | " | $CO_2CH_3$ en 6 | " | " | " | " |

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| nBu | -CH= | S | -CH= | $CH_2OH$ en 6 | H | | - | COOH |
| " | " | " | " | $CH_2OCH_3$ en 6 | " | " | " | " |
| " | " | " | " | $CH_2OH$ en 4 | " | " | " | " |
| " | " | " | " | COOH en 6 | " | " | " | 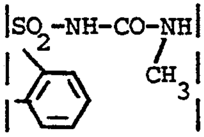 |
| " | " | " | " | COOH en 4 | " | " | " | " |
| " | " | " | " | $CH_2OH$ en 6 | " | " | " | " |
| " | " | " | " | $CH_2OH$ en 4 | " | " | " | " |
| " | " | " | " | OH en 6 | " | " | " | " |
| " | " | " | " | OH en 4 | " | " | " | " |
| " | " | " | " | $CO_2CH_3$ en 6 | " | " | " | " |
| " | " | " | " | $CO_2CH_3$ en 4 | " | " | " | " |
| " | " | " | " | COOH en 6 | " | " | " | 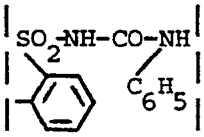 |
| " | " | " | " | COOH en 4 | " | " | " | " |
| " | " | " | " | $CH_2OH$ en 6 | " | " | " | " |
| " | " | " | " | $CH_2OH$ en 4 | " | " | " | " |
| " | " | " | " | OH en 6 | " | " | " | " |
| " | " | " | " | OH en 4 | " | " | " | " |

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| nBu | -CH= | S | -CH= | $CO_2CH_3$ en 6 | H | | - | |
| " | " | " | " | $CO_2CH_3$ en 4 | " | " | " | " |
| nBu | -CH= | -CH= | S | H | H | 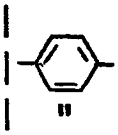 | - | 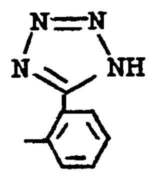 |
| " | " | " | " | COOH en 4 | " | " | " | |
| " | " | " | " | COOH en 5 | " | " | " | " |
| " | " | " | " | $CO_2CH_3$ en 5 | " | " | " | " |
| " | " | " | " | $CH_2OH$ en 5 | " | " | " | " |
| " | " | " | " | $CH_2OCH_3$ en 5 | " | " | " | " |
| " | " | " | " | $CH_2OH$ en 4 | " | " | " | " |
| " | " | " | " | $CO_2CH_3$ en 4 | H | " | " | " |
| " | " | " | " | OH en 4 | " | " | " | " |
| " | " | " | " | OH en 5 | " | " | " | " |
| " | " | " | " | H | H | " | " | COOH |
| " | " | " | " | OH en 4 | " | " | " | " |
| " | " | " | " | OH en 5 | " | " | " | " |
| " | " | " | " | COOH en 5 | " | " | " | " |
| " | " | " | " | $CO_2CH_3$ en 5 | " | " | " | " |
| " | " | " | " | $CH_2OH$ en 5 | " | " | " | " |

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| nBu | -CH= | S | -CH= | $CH_2OCH_3$ en 5 | H | | - | COOH |
| " | " | " | " | $CH_2OH$ en 4 | " | " | " | " |
| " | " | " | " | COOH en 4 | " | " | " | " |
| " | " | " | " | $CO_2CH_3$ en 4 | " | " | " | " |
| " | " | " | " | H | H | " | " | 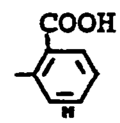 |
| " | " | " | " | OH en 4 | " | " | " | " |
| " | " | " | " | COOH en 5 | " | " | " | " |
| " | " | " | " | $CO_2CH_3$ en 5 | " | " | " | " |
| " | " | " | " | $CH_2OH$ en 5 | " | " | " | " |
| " | " | " | " | $CH_2OCH_3$ en 5 | " | " | " | " |
| " | " | " | " | $CH_2OH$ en 4 | " | " | " | " |
| " | " | " | " | COOH en 4 | " | " | " | " |
| " | " | " | " | $CO_2CH_3$ en 4 | " | " | " | " |
| " | " | " | " | OH en 5 | " | " | " | " |
| " | " | " | " | $CH_2OH$ en 5 | OH | " | " | " |
| " | " | " | " | COOH en 5 | " | " | " | " |

| $R_b$ | $Z_1$ | $Z_2$ | $Z_3$ | $R_{1b}$ | $R_{2b}$ | $Y_{1b}$ | $B_b$ | $Y_{2b}$ |
|---|---|---|---|---|---|---|---|---|
| nBu | -CH= | S | -CH= | $CO_2CH_3$ en 5 | OH | 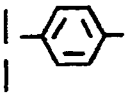 | - | 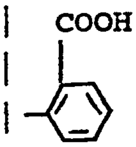 |
| " | " | " | " | COOH en 4 | " | " | " | 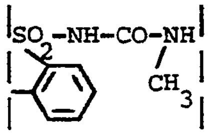 |
| " | " | " | " | COOH en 5 | " | " | " | " |
| " | " | " | " | $CH_2OH$ en 4 | " | " | " | " |
| " | " | " | " | $CH_2OH$ en 5 | " | " | " | " |
| " | " | " | " | OH en 4 | " | " | " | " |
| " | " | " | " | OH en 5 | " | " | " | " |
| " | " | " | " | $CO_2CH_3$ en 4 | " | " | " | " |
| " | " | " | " | $CO_2CH_3$ en 5 | " | " | " | " |
| " | " | " | " | COOH en 4 | " | " | " | 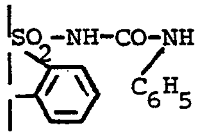 |
| " | " | " | " | COOH en 5 | " | " | " | " |
| " | " | " | " | $CH_2OH$ en 4 | " | " | " | " |
| " | " | " | " | $CH_2OH$ en 5 | " | " | " | " |
| " | " | " | " | OH en 4 | " | " | " | " |
| " | " | " | " | OH en 5 | " | " | " | " |
| " | " | " | " | $CO_2CH_3$ en 4 | " | " | " | " |
| " | " | " | " | $CO_2CH_3$ en 5 | " | " | " | " |

Dans les tableaux indiqués ci-dessus, les produits peuvent comporter pour $Y_{2a}$ ou $Y_{2b}$ un noyau phényle substitué par un autre groupement de formule $-(CH_2)_m-SO_2-X-R_{14}$ dont des exemples sont donnés ci-dessus

dans une liste non exhaustive et constituer ainsi également des produits pouvant être obtenus dans le cadre de la présente invention.

De plus, parmi les produits de formule (I) répondant à la formule ($I_c$), les formules (I) et ($I_c$) étant telles que définies ci-dessus, on peut citer plus précisément les produits suivants qui constituent des produits préférés de l'invention répondant à la formule ($I'_d$) :

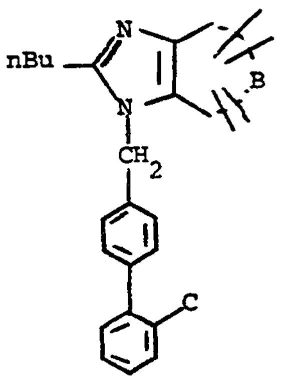

($I'_d$)

dans laquelle C représente les radicaux formyle, cyano, carboxy libre, salifié ou estérifié, tétrazole éventuellement substitué ou salifié et les radicaux $SO_2$-$X_b$-$R_{14b}$ tels que définis ci-dessus et notamment :

$-SO_2-NH-CO-NH-CH_2-CH=CH_2$

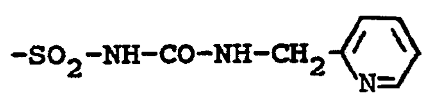

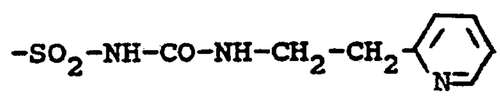

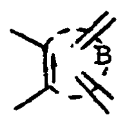

et dans laquelle le reste de cycle B renferme 4 à 5 chaînons, identiques ou différents, choisis parmi

- l'atome de soufre,
- l'atome d'azote éventuellement substitué par un radical formyle ou carboxy libre, salifié ou estérifié,
- le radical oxo >=O
- le radical méthine ≧CH éventuellement substitué,
- le radical méthylène $>CH_2$ éventuellement susbtitué par un ou deux radicaux, identiques ou différents,

les éventuels substituants des radicaux méthine et méthylène étant choisis parmi les radicaux :

- alkyle renfermant au plus 4 atomes de carbone tel que par exemple méthyle, éthyle, propyle, isopropyle, n-butyle, éventuellement substitué tel que notamment dans le radical hydroxy méthyle,
- carboxy libre, salifié ou estérifié,
- aryle tel que notamment phényle, pyridyle, pyrimidyle,
- aralkyle tel que notamment benzyle, pyridylméthyle,
- amino et carbamoyle éventuellement substitué par un ou deux radicaux alkyle ou par un radical acyle.

On peut ainsi notamment et de façon non exhaustive citer les radicaux suivants qui renferment :

a) un atome de soufre et 4 à 5 chaînons tels que :

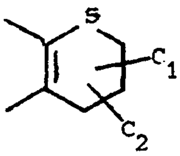 ; 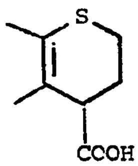 ; 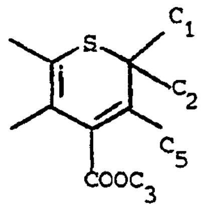 ; 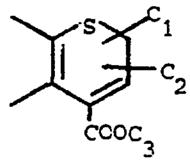 ;

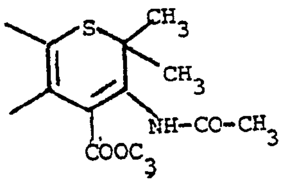 ; 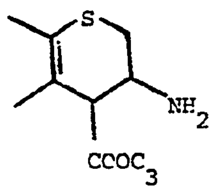 ; 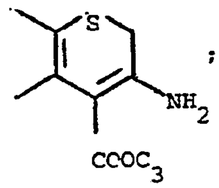 ; 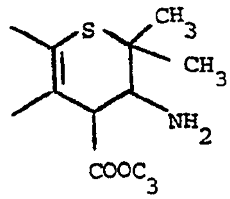 ;

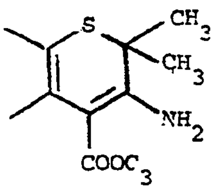 ; 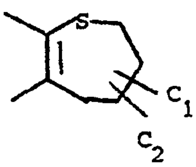 ; 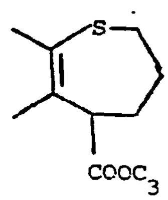 ; 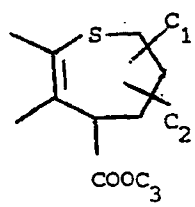 ;

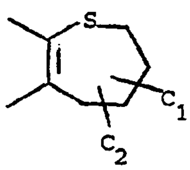 ; 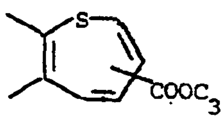 ; 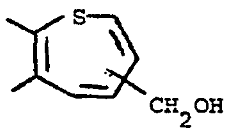

b) deux atomes de soufre et 4 à 5 chaînons tels que :

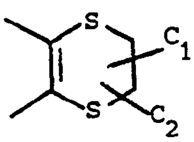 ; 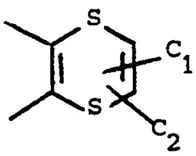 ; 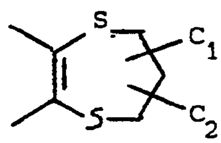 ;

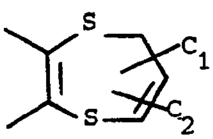 ; 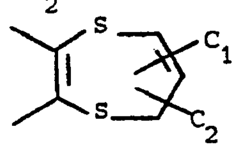 ;

c) un atome de soufre et un atome d'azote et 4 à 5 chaînons tels que :

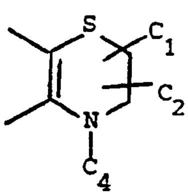 ; 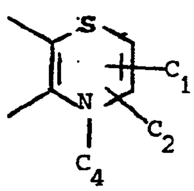 ; 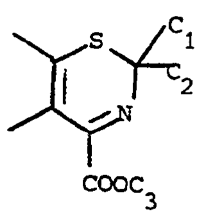 ; 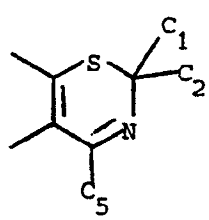 ;

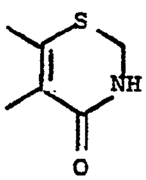 ; 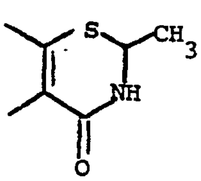 ; 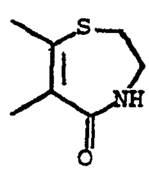 ; 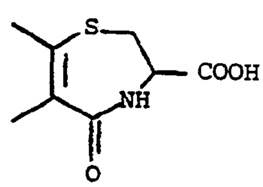

d) deux atomes de soufre et un atome d'azote et 4 chaînons tels que :

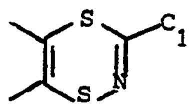

Dans les formules précédentes, $C_1$, $C_2$ et $C_5$ sont choisis parmi les valeurs $-CH_2OH$, $CO_2H$, alkyle $C_{1-4}$ et aryle notamment phényle, $C_3$ est choisi parmi l'atome d'hydrogène, les radicaux alkyle $C_{1-4}$ et aryle notamment phényle, $C_4$ est choisi parmi l'atome d'hydrogène et les radicaux formyle et $CO_2C_3$ dans lequel $C_3$ a les définitions précédentes.

Les exemples suivants illustrent l'invention sans toutefois la limiter.

**EXEMPLE 1 : 2-butyl 1-((4-cyanophényl) méthyl) 1H-benzimidazol 6-carboxylate de méthyle et son homologue 5-carboxylate de méthyle**

STADE A : 2-butyl-1H-benzimidazole 5-carboxylate de méthyle

A une solution de 5 g de 3,4-diaminobenzoate de méthyle dans 60 $cm^3$ de tétrahydrofuranne, on ajoute 5,47 g de chlorhydrate de pentanimidoate d'éthyle (préparation selon J.A.C.S. 64, 1827 (1942)), on agite 3 heures 30 à 90°C. On évapore le tétrahydrofuranne ajoute 50 $cm^3$ d'une solution saturée de bicarbonate de sodium et extrait avec du chlorure de méthylène. On lave à l'eau, sèche et évapore à sec sous pression réduite. On recueille 9 g de produit que l'on cristallise dans l'éther isopropylique. On obtient 6,65 g de produit recherché. F = 114°C.

Analyse pour $C_{13}H_{16}N_2O_2$ = 232,27

| | C | H | N |
|---|---|---|---|
| % calculés | 67,22 | 6,94 | 12,06 |
| % trouvés | 67,3 | 6,8 | 11,3 |

Spectre IR : $CHCl_3$

| | |
|---|---|
| =C-NH | 3456 $cm^{-1}$ |
| >=O | 1712 $cm^{-1}$ |
| $CH_3$ de $COOCH_3$ | 1438 $cm^{-1}$ |
| Aromatiques + système conjugué | 1588 - 1576 - 1546 $cm^{-1}$ |

STADE B : 2-butyl 1-((4-cyanophényl) méthyl) 1H-benzimidazole 6-carboxylate de méthyle (produit A) et son isomère 5-carboxylate de méthyle (produit B)

A une solution de 4,64 g du produit obtenu au stade A ci-dessus, dans 46 $cm^{-1}$ de diméthyl formamide, on ajoute 960 mg d'hydrure de sodium en dispersion à 50 % dans l'huile. On agite 1 heure 30 à température ambiante et ajoute 4,4 g de 4-bromométhyl benzonitrile. On agite 30 minutes puis ajoute lentement 100 $cm^3$ d'eau, on agite 30 minutes, essore, lave à l'eau et sèche à 100°C sous pression réduite. On obtient : 7,4 g de produit brut. F = 140°C.

Isolement du produit A :

On dissout 7,4 g du produit ci-dessus dans 400 $cm^3$ d'acétate d'éthyle au reflux, filtre la solution à chaud et concentre à un volume total de 100 $cm^3$. On agite 1 heure à température ambiante. On obtient après essorage 3,4 g de produit recherché.
F = 200-205°C.

Isolement du produit B :

On évapore à sec les liqueurs mères du produit A et cristallise le résidu dans l'éther isopropylique, on recueille 3,35 g du produit attendu. F = 120°C.

Contrôle :

Analyse pour $C_{21}H_{21}N_3O_2$ = 347,4

| | C | H | N |
|---|---|---|---|
| % calculés | 72,06 | 6,09 | 12,10 |
| % trouvés Isomère A | 72,7 | 5,9 | 12,1 |
| % trouvés Isomère B | 72,4 | 5,8 | 12,0 |

Spectre de RMN : ($CDCl_3$, 400 MHz)

| | Isomère A | Isomère B |
|---|---|---|
| $\underline{CH_3}-(CH_2)_3-$ | 13,7 | 13,7 |
| $CH_3-\underline{CH_2}-(CH_2)_2-$ | 22,5 | 22,5 |
| $CH_3-CH_2-\underline{CH_2}-$ | 27,4 | 27,3 |
| $CH_3-(CH_2)_2-\underline{CH_2}-$ | 29,4 | 29,4 |
| $>N-\underline{CH_2}-C_6H_4$ | 46,5 | 46,6 |
| $C \equiv N$ | 188,1 | 118,1 |
| $>N-C<$ | 134,8 | 138,2 |
| $=N-C\leq$ | 146,2 | 142,3 |
| $COOCH_3$ | 52,1 | 52,0 |
| aromatiques | 111,1 à 140,8 (10H) | 108,7 à 140,8 (10H) |

EXEMPLE 2 : **Acide 2-butyl 1-((4-carboxyphényl) méthyl) 1H-benzimidazol 6-carboxylique**

On agite pendant 19 heures au reflux une solution de 500 mg du produit A obtenu à l'exemple 1, dans 2,5 cm³ d'une solution constituée à parts égales d'acide sulfurique, d'acide acétique et d'eau. On refroidit, ajoute 30 g de glace et alcalinise avec de la lessive de soude. On ajuste ensuite le pH à 6 avec de l'acide acétique, agite 15 minutes à température ambiante, essore, lave à l'eau et sèche à 90°C sous pression réduite. On obtient 480 mg de produit. F $\simeq$ 290°C.

Purification : 556 mg de produit obtenu comme ci-dessus sont dissous dans 30 cm³ de méthanol au reflux, on concentre à 10 cm³, ajoute 2 cm³ d'eau puis après amorçage de la cristallisation, on ajoute 20 cm³ d'eau. Après 3 heures, on essore, lave avec 3 fois 5 cm³ de mélange méthanol-eau (1-1). On obtient 490 mg de produit. F = 290°C.

On dissout ces 490 mg de produit dans 40 cm³ d'isopropanol au reflux, concentre à 15 cm³. On maintient 16 heures à température ambiante, essore et obtient 430 mg de produit recherché. F = 290°C.

Analyse pour $C_{20}H_{20}N_2O_4$ = 352,38

| | C | H | N |
|---|---|---|---|
| % calculés | 68,17 | 5,72 | 7,95 |
| % trouvés | 68,4 | 5,8 | 8,0 |

Spectre IR : Nujol
C = O 1725 $cm^{-1}$
Spectre de RMN : DMSO, 400 MHz
$CH_3$ 0,86 (t)

| | |
|---|---|
| 2 $CH_2$ centraux | 1,35 (m) et 1,71 (m) |
| l'autre $CH_2$ de la chaîne | 2,87 (m) |
| N-$CH_2$- | 5,70 (s) |
| aromatiques | 7,16 (d) et 7,91 (d) 4H |
| Les autres aromatiques | 7,67 (d), 7,82 (d), 8,05 (s) |
| H mobile du diacide | 12,88 (m) 2H. |

**EXEMPLE 3 : Acide 2-butyl 1-((4-carboxyphényl) méthyl) 1H-benzimidazol 5-carboxylique**

On opère comme à l'exemple 2 à partir de 500 mg de produit B obtenu à l'exemple 1. On obtient 390 mg de produit brut. F = 200°C.

On dissout le produit brut dans 10 $cm^3$ d'éthanol à 60°C, ajoute 10 $cm^3$ d'eau et laisse reposer 2 heures à température ambiante. On essore et recueille 260 mg de produit que l'on dissout dans 150 $cm^3$ d'acétate d'éthyle au reflux, concentre à un volume total de 30 $cm^3$, laisse reposer 16 heures à température ambiante et essore. On obtient 220 mg de produit attendu. F $\simeq$ 225°C.

Analyse pour $C_{20}H_{20}N_2O_4$ = 352,38

| | C | H | N |
|---|---|---|---|
| % calculés | 68,17 | 5,72 | 7,95 |
| % trouvés | 68,2 | 5,7 | 7,9 |

Spectre IR : Nujol
C = O 1717 et 1685 $cm^{-1}$

**EXEMPLE 4 : 4-((2-butyl-1H-benzimidazol-1-yl) méthyl) benzonitrile**

On opère comme au stade B de l'exemple 1 à partir de 3,5 g de 2-butyl-1H-benzimidazol (préparé selon POOL et Al. Am. Soc. 59, 178 (1937)) et 4,3 g de 4-bromométhyl benzonitrile. On obtient 6,8 g de produit recherché (F = 130°C) que l'on dissout dans le chlorure de méthylène, traite avec du charbon actif, filtre et évapore à sec, on recueille 6 g de produit (F = 130°C) que l'on recristallise dans l'éther isopropylique. On obtient 4,7 g du produit attendu F = 148°C, l'échantillon analytique a été obtenu en recristallisant deux fois successives 1,2 g du produit ci-dessus dans l'éther éthylique pour recueillir 700 mg de produit purifié F = 150°C.

Analyse pour $C_{19}H_{19}N_3$ = 289,381

| | C | H | N |
|---|---|---|---|
| % calculés | 78,86 | 6,62 | 14,52 |
| % trouvés | 79,0 | 6,6 | 14,6 |

**EXEMPLE 5 : Acide 4-((2-butyl-1H-benzimisazol-1-yl) méthyl benzoïque**

On opère comme à l'exemple 2 à partir de 1 g du produit obtenu à l'exemple 4. On obtient 1 g du produit recherché que l'on recristallise dans 10 $cm^3$ d'acétate d'éthyle. On obtient 900 mg de produit attendu. F = 235°C.

L'échantillon analytique a été obtenu par deux recristallisations successives dans l'isopropanol, on recueille ainsi 500 mg de produit pur F = 235°C.

Analyse pour $C_{19}H_{20}N_2O_2$ = 308,88

| | C | H | N |
|---|---|---|---|
| % calculés | 73,99 | 6,54 | 9,08 |
| % trouvés | 74,0 | 6,6 | 9,3 |

Spectre IR : (Nujol)

Absorption complexe région OH/NH

| | |
|---|---|
| >= O | 1690 $cm^{-1}$ |
| Aromatiques | 1610 $cm^{-1}$ |
| + | 1579 $cm^{-1}$ |
| Hétéroatome | 1508 $cm^{-1}$ |

**EXEMPLE 6 : 4-((2-butyl-1H-benzimidazol-1-yl) méthyl) N-(1H-indol-4-yl) benzamide**

A une suspension de 180 mg du produit obtenu à l'exemple 5, dans 4 $cm^3$ de chlorure de méthylène, on ajoute 192 mg de chlorhydrate de 1-éthyl-3-(3-diméthylaminopropyl) carbodiimide. On agite 10 minutes à température ambiante et ajoute 53 mg de 4-amino indole. On agite 18 heures, on extrait au chlorure de méthylène 2 fois 25 $cm^3$, lave à l'eau, sèche et évapore à sec sous pression réduite. On obtient 230 mg de résidu que l'on chromatographie sur silice (éluant : chlorue de méthylène-méthanol (95-5)). On obtient 115 mg de produit recherché (F = 196°C). Après recristallisation dans l'acétate d'éthyle on recueille 87 mg de produit recherché F = 196°C.

Analyse pour $C_{27}H_{26}N_4O$ = 422,51

| | C | H | N |
|---|---|---|---|
| % calculés | 76,76 | 6,20 | 13,26 |
| % trouvés | 77,2 | 6,2 | 13,3 |

Spectre IR : (Nujol)

| | |
|---|---|
| C = O | 1640 $cm^{-1}$ |
| Aromatique | 1611 $cm^{-1}$ |
| hétéroatome | 1575 $cm^{-1}$ |
| amide II | 1528 $cm^{-1}$ |
| | 1492 $cm^{-1}$ |

**EXEMPLE 7 : 2-butyl-1-((4-cyanophényl) méthyl) 1H-benzimidazol 4-carboxylate de méthyle**

STADE A : 2-butyl-1H-benzimidazole 4-carboxylate de méthyle.

On opère comme au stade A de l'exemple 1, à partir de 2,5 g de 2,3-diaminobenzoate de méthyle (préparé selon J. Chem. Soc. 117 (1920) p. 775 et CAN. J. Chem. 55 (1977 p. 1653-1657) en utilisant 2,73 g de chlorhydrate de pentanimidoate d'éthyle (préparé selon J.A.C.S. 64, 1827 (1942)). On obtient 3,05 g de produit recherché, cristallisé de l'essence G. F ≃ 85°C. L'échantillon analytique est obtenu par deux recristallisations successives de 280 mg de produit, dans l'éther isopropylique, on recueille 100 mg de produit F = 97°C.

Analyse pour $C_{13}H_{16}N_2O_2$ = 232,29

| | C | H | N |
|---|---|---|---|
| % calculés | 67,22 | 6,94 | 12,06 |
| % trouvés | 67,1 | 7,0 | 12,0 |

Spectre IR : ($CHCl_3$)

| | |
|---|---|
| =C-NH | 3448 $cm^{-1}$ |
| >= O complexe | 1728 $cm^{-1}$ |
| | 1699 $cm^{-1}$ |
| Système conjugué | 1626 $cm^{-1}$ |
| + | 1606 $cm^{-1}$ |
| aromatique | 1522 $cm^{-1}$ |
| | 1495 $cm^{-1}$ |

STADE B : 2-butyl-1-((4-cyanophényl) méthyl) 1H-benzimidazol 4-carboxylate de méthyle

On opère comme au stade B de l'exemple 1 à partir de 1,86 g de produit obtenu au stade A précédent en utilisant 1,88 g de 4-bromométhyl benzonitrile. On obtient 3,5 g de produit que l'on chromatographie sur silice (éluant : acétate d'éthyle-cyclohexane (6-4)). On obtient 2,05 g de produit recherché recristallisé de l'éther éthylique. F = 113°C.
Spectre de RMN : ($CDCl_3$, 250 MHz)

| | |
|---|---|
| $\underline{CH_3}-(CH_2)_2$ | 0,92 ppm (t) |
| $CH_2$ centraux | 1,43 - 1,82 ppm (m) |
| $>-\underline{CH_2}-CH_2$ | 2,92 ppm (t) |
| $CO_2CH_3$ | 4,04 ppm (s) |
| $N-\underline{CH_2}-C_6H_5$ | 5,44 ppm |
| benzonitrile | 7,09 - 7,61 ppm (d) |
| $H_5$ | 7,95 ppm (dd) |
| $H_6$, $H_7$ | ≃ 7,27 ppm (m). |

**EXEMPLE 8 : Acide 2-butyl 1-((4-carboxyphényl) méthyl) 1H-benzimidazol 4-carboxylique**

On opère comme à l'exemple 2 à partir de 1 g du produit obtenu à l'exemple 7. On obtient 1 g du produit recherché F = 250°C.

On a obtenu l'échantillon analytique après 2 recristallisation successives dans le mélange chlorure de méthylèneméthanol en recueillant 345 mg de produit F = 250°C.

Analyse pour $C_{20}H_{20}N_2O_4$ = 352,39

| | C | H | N |
|---|---|---|---|
| % calculés | 68,17 | 5,72 | 7,95 |
| % trouvés | 68,3 | 5,8 | 8,1 |

Spectre de RMN : (DMSO, 250 MHz)
$\underline{CH_3}-(CH_2)$ 0,87 ppm (t)

| | |
|---|---|
| $CH_3-(CH_2)_2-$ | 1,37 - 1,71 ppm (m) |
| $=C-CH_2$ | 2,93 ppm (t) |
| $N-\underline{CH_2}-C_6H_5$ | 5,71 ppm (s) |
| $-C_6H_4-CO$ | 7,21 - 7,91 ppm (dl) |
| $H_6$ | 7,36 ppm (t) |
| $H_5$, $H_7$ | 7,80 ppm (dd). |

**EXEMPLE 9 : 4-((2-butyl-1H-napht(2,3-d) imidazol-1-yl) méthyl) benzonitrile**

STADE A : 2-butyl-1H-napht(2,3-d) imidazole

On opère comme au stade A de l'exemple 1 à partir de 2,7 g de 2,3-diaminonaphtalène en utilisant 3,4 g de chlorhydrate de pentanimidoate d'éthyle (préparé selon J.A.C.S. 64 1827 (1942)) et le dichloréthane comme solvant. On obtient 4,5 g de produit brut que l'on dissout dans 100 $cm^3$ d'acétate d'éthyle au reflux, concentre à un volume total de 30 $cm^3$, laisse en repos 16 heures, essore et obtient 2,37 g de produit recherché. F = 188°C.

Un échantillon analytique a été obtenu par recristallisation successives de 500 mg du produit obtenu ci-dessus, dans l'acétate d'éthyle puis l'isopropanol. On a recueilli 280 mg de produit attendu. F = 190°C.

Analyse pour $C_{15}H_{16}N_2$ = 224,29

| | C | H | N |
|---|---|---|---|
| % calculés | 80,32 | 7,19 | 12,49 |
| % trouvés | 80,1 | 7,2 | 12,4 |

Spectre de RMN : (DMSO, 250 MHz)

| | |
|---|---|
| $\underline{CH_3}-(CH_2)$ | 0,94 ppm (t) |
| $CH_3-(\underline{CH_2})-(\underline{CH_2})-$ | 1,41 - 1,83 (m) |
| $>C-\underline{CH_2}-$ | 2,91 (t) |
| aromatiques | 7,35 (m) 2H - 7,97 (m) 4H |
| 1H mobile | 12,29 (sl) |

STADE B : 4-((2-butyl-1H-napht(2,3-d) imidazol-1-yl) méthyl) benzonitrile

On opère comme au stade B de l'exemple 1 à partir de 449 mg du produit obtenu au stade A ci-dessus, en utilisant 392 mg de 4-bromométhyl benzonitrile. On obtient 460 mg de produit recherché F = 138°C, après recristallisation dans l'éther éthylique.

**EXEMPLE 10 : Acide 4-((2-butyl-1H-napht(2,3-d) imidazol-1-yl) méthyl) benzoïque**

On porte au reflux pendant 21 heures une suspension de 420 mg du composé obtenu à l'exemple 9 avec 4 $cm^3$ d'éthanol et 7,5 $cm^3$ de soude 2N. On refroidit, ajoute 5 $cm^3$ de glace et neutralise à pH 7 avec 7,5 $cm^3$ d'acide chlorhydrique 2N, on ajoute 30 $cm^3$ de méthanol, chauffe au reflux et concentre à 20 $cm^3$. Après essorage, on obtient 420 mg de produit attendu.

On recristallise les 420 mg du produit ci-dessus, successivement dans l'isopropanol puis dans l'acétate d'éthyle, on recueille 260 mg de produit. F = 220°C.

Analyse pour $C_{23}H_{22}N_2O_2$ = 358,44

| | C | H | N |
|---|---|---|---|
| % calculés | 77,07 | 6,19 | 7,82 |
| % trouvés | 76,8 | 6,0 | 7,6 |

Spectre de RMN : (DMSO, 400 MHz)

| | |
|---|---|
| $\underline{CH_3}$-$CH_2$ | 0,87 ppm (t) |
| $CH_3$-$\underline{CH_2}$-$\underline{CH_2}$ | 1,38 et 1,76 ppm (m) |
| $\underline{CH_2}$-C< | 2,89 ppm (t) |
| N-$\underline{CH_2}$-$C_6H_5$ | 5,68 ppm (s) |
| C-$C_6H_4$-C< | 7,22 et 7,89 ppm (d) |
| aromatiques | 7,36 (m) 2H - 7,89 (m) 2H - 7,99 (m) 1H - 8,14 (s) 1H |
| H mobile | 12,96 (s) |

**<u>EXEMPLE 11</u> : 4-((2-butyl-3H-imidazo(4,5-d) pyrimidin-3-yl) méthyl) benzonitrile (produit A) et 4-((2-butyl-3h-imidazo(5,6-d) pyrimidine-3-yl) méthyl) benzonitrile (produit B)**

<u>STADE A</u> : 2-butyl-3H-imidazo(4,5-d) pyrimidine

On opère comme à l'exemple 1 à partir de 5 g de 4,5-diaminopyrimidine en utilisant 10 g de chlorhydrate de pentanimidoate d'éthyle (J.A.C.S. <u>64</u>, 1827 (1942)) et en utilisant le diméthyl formamide comme solvant. On obtient 2,1 g de produit recherché, cristallisé de l'acétate d'éthyle. F = 166°C. On recristallise le produit ci-dessus dans l'acétate d'éthyle et obtient 1,8 g du produit attendu F = 168°C. 200 mg d'échantillon analytique ont été obtenu par une recristallisation supplémentaire dans l'acétate d'éthyle, de 300 mg du produit ci-dessus. F = 168°C.

Analyse pour $C_9H_{12}N_4$ = 176,22

| | C | H | N |
|---|---|---|---|
| % calculés | 61,34 | 6,86 | 31,8 |
| % trouvés | 61,3 | 6,9 | 31,9 |

Spectre de RMN : ($CDCl_3$, 250 MHz)

| | |
|---|---|
| $CH_3$- | 1,01 ppm (t) |
| $CH_3$-$\underline{CH_2}$-$\underline{CH_2}$ | 1,53 et 1,99 (m) |
| $\underline{CH_2}$-C< | 3,12 (t) |
| aromatiques | 8,99 et 9,13 (s) |
| H mobile | 13,45 (sl) |

<u>STADE B</u> : 4-((2-butyl-3H-imidazo(4,5-d) pyrimidin-3-yl) méthyl) benzonitrile (Produit A) et 4-((2-butyl-3H-imidazo-(5,6-d) pyrimidin-3-yl) méthyl) benzonitrile (Produit B).

On opère comme au stade B de l'exemple 1 à partir de 352 mg du composé obtenu au stade A ci-dessus, en utilisant 431 mg de 4-bromométhyl benzonitrile, on obtient 630 mg de produit brut que l'on chromatographie sur silice, (éluant : chlorure de méthylène-méthanol (9-1)), on recueille 130 mg de produit A, cristallisé de l'éther éthylique (F = 112°C) et 35 mg de produit B F = 85°C, cristallisé d'un mélange méthyl éthyl céthone éther.

Produit A :

Analyse pour $C_{17}H_{17}N_5$ = 291,35

| | C | H | N |
|---|---|---|---|
| % calculés | 70,08 | 5,88 | 24,04 |
| % trouvés | 70,0 | 5,8 | 23,9 |

Spectre de RMN : ($CDCl_3$, 300 MHz ppm)

| | |
|---|---|
| $CH_3-$ | 0,93 (t) |
| $CH_3-\underline{CH_2}-\underline{CH_2}$ | 1,42 et 1,82 (m) |
| $\underline{CH_2}-<$ | 2,80 (m) |
| $N-\underline{CH_2}-$ | 5,52 (s) |
| aromatiques | 7,27 - 7,65 (d,l) |
| hétérocycle | 8,94 et 9,08 (s). |

Produit B :

Spectre de RMN : ($CDCl_3$, 300 MHz ppm)

| | |
|---|---|
| $CH_3-$ | 0,94 (t) |
| $CH_3-\underline{CH_2}-\underline{CH_2}$ | 1,44 et 1,88 (m) |
| $>C-\underline{CH_2}-$ | 2,90 (m) |
| $-N-\underline{CH_2}-$ | 5,49 (s) |
| aromatiques | 7,18 - 7,68 (d,l) |
| hétérocycle | 8,60 et 9,09 (s). |

**EXEMPLE 12 : Acide 4-((2-butyl-3H-imidazo(4,5-d) pyrimidin-3-yl) méthyl) benzoïque**

On agite 2 heures au reflux 400 mg du produit A obtenu comme à l'exemple 11 avec 8 $cm^3$ d'éthanol à 10 % eau et 1 $cm^3$ de lessive de soude. On ajoute 20 $cm^3$ d'eau, neutralise à pH 5-6 avec de l'acide acétique, extrait avec du chlorure de méthylène, lave, sèche et évapore à sec sous pression réduite. On obtient 280 mg de produit attendu, cristallisé d'un mélange acétate d'éthyle-éther (F = 165°C).

On recristallise deux fois successives, le produit obtenu ci-dessus, dans de la méthyl éthyl cétone. On obtient 185 mg du composé recherché F = 173°C.

Analyse pour $C_{17}H_{18}N_4O_2$ = 310,35

| | C | H | N |
|---|---|---|---|
| % calculés | 65,79 | 5,85 | 18,05 |
| % trouvés | 65,8 | 5,9 | 18,0 |

Spectre de RMN : (DMSO, 250 MHz ppm)

| | |
|---|---|
| $CH_3$- | 0,84 (t) |
| $CH_3$-$\underline{CH_2}$-$\underline{CH_2}$ | 1,34 et 1,68 (m) |
| >C-$\underline{CH_2}$- | 2,86 (t) |
| N-$\underline{CH_2}$- | 5,62 (s) |
| hétérocycle | 8,90 et 9,09 (sl) |
| aromatiques | 7,28 et 7,92 (d,l) |
| H mobile | 12,88 (m). |

**EXEMPLE 13 : Acide 4-((2-butyl-3H-imidazo(5,6-d) pyrimidin-3-yl) méthyl) benzoïque**

En opérant comme à l'exemple 12 à partir du produit B obtenu à l'exemple 11, on a obtenu le composé attendu.
PF = 180°C.

**EXEMPLE 14 : 4-((2-butyl-5,6-diméthyl-1H-benzimidazol-1-yl) méthyl) benzonitrile**

STADE A : 2-butyl-5,6-diméthyl-1H-benzimidazole

On opère comme au stade A de l'exemple 1 à partir de 2,04 g de 4,5-diméthyl 1,2-phénylène diamine en utilisant 3,73 g de chlorhydrate de pentanimidoate d'éthyle (J.A.C.S. 64, 1827 (1942)). On obtient 3,48 g de produit brut que l'on chromatographie sur silice, (éluant : chlorure de méthylèneméthanol (9-1)) on obtient 2,27 g du composé attendu
F = 110°C.

Un échantillon analytique a été préparé par recristallisation de 120 mg du produit ci-dessus dans l'éther isopropylique. On recueille 88 mg de produit F = 110°C.

Analyse pour $C_{17}H_{18}N_4O_2$ = 310,35

| | C | H | N |
|---|---|---|---|
| % calculés | 65,79 | 5,85 | 18,05 |
| % trouvés | 65,8 | 5,9 | 18,0 |

Spectre IR : ($CHCl_3$)

| | |
|---|---|
| =C-NH | 3470 $cm^{-1}$ |
| C=N | 1634 $cm^{-1}$ |
| aromatique | 1585 $cm^{-1}$ |
| | 1538 $cm^{-1}$ |

STADE B : 4-((2-butyl-5,6-diméthyl-1H-benzimidazol-1-yl) méthyl) benzonitrile

On opère comme au stade B de l'exemple 1 à partir de 2,02 g de produit obtenu au stade A ci-dessus, en utilisant 2,21 g de 4-bromométhyl benzonitrile. On obtient 2,88 g du composé recherché cristallisé de l'éther.
F = 159°C.

L'échantillon analytique a été préparé par recristallisation de 476 mg de produit dans l'isopropanol puis dans l'acétate d'éthyle, on recueille 132 mg de produit attendu.

F = 160°C.

Analyse pour $C_{21}H_{23}N_3$ = 317,43

| | C | H | N |
|---|---|---|---|
| % calculés | 79,46 | 7,30 | 13,24 |
| % trouvés | 79,7 | 7,5 | 13,1 |

Spectre de RMN : ($CDCl_3$, 250 MHz ppm)

| | |
|---|---|
| $CH_3$- | 0,90 (t) |
| $CH_3$-$\underline{CH_2}$-$\underline{CH_2}$ | 1,40 - 1,78 (m) |
| >C-$\underline{CH_2}$- | 2,75 (m) |
| 2 $\underline{CH_3}$-$C_6H_3$ | 2,31 - 2,36 (s) |
| N-$\underline{CH_2}$-$C_6H_4$ | 5,34 (s) |
| 2H | 6,87 - 7,63 (s) |

**EXEMPLE 15 : Acide 4-((2-butyl-5,6-diméthyl-1H-benzimidazol-1-yl) éthyl) benzoïque**

On opère comme à l'exemple 12 à partir de 395,7 mg de produit obtenu comme à l'exemple 14. On obtient 367 mg de produit attendu. F = 220°C. L'échantillon analytique a été obtenu en recristallisant deux fois dans l'isopropanol le produit ci-dessus. On recueille 198 mg de produit pur. F = 254°C.

Analyse pour $C_{21}H_{24}N_2O_2$ = 336,44

| | C | H | N |
|---|---|---|---|
| % calculés | 74,97 | 7,19 | 8,33 |
| % trouvés | 74,8 | 7,3 | 8,3 |

Spectre de RMN : (DMSO, 250 MHz ppm)

| | |
|---|---|
| $CH_3$- | 0,89 (t) |
| $CH_3$-$\underline{CH_2}$-$\underline{CH_2}$ | 1,33 et 1,66 (m) |
| >C-$\underline{CH_2}$- | 2,75 (t) |
| les $CH_3$-C< | 2,26 et 2,29 (s) |
| le N-$\underline{CH_2}$-$C_6H_4$ | 5,51 (sl) |
| les aromatiques | 7,17 - 7,85 (d) |
| les autres aromatiques | 7,11 - 7,36 (s) |

**EXEMPLE 16 : 1H-benzimidazol méthyl benzonitrile**

On opère comme au stade B de l'exemple 1 à partir de 951 mg de benzimidazole en utilisant 1,97 g de 4-bromométhyl benzonitrile dans le diméthylformamide. On obtient 2,48 g de produit brut que l'on chromatographie sur silice (éluant : chlorure de méthylène-méthanol (95-5)), on obtient 1,34 g du produit recherché, F = 94°C, après cristallisation dans l'éther.

Un échantillon analytique a été préparé par deux recristallisations successives de 422 mg du produit obtenu ci-dessus, dans l'éther puis l'éther isopropylique. On recueille 167 mg de produit pur. F = 94°C.

| Analyse pour $C_{15}H_{11}N_3$ = 233,28 | C | H | N |
|---|---|---|---|
| % calculés | 77,23 | 4,75 | 18,02 |
| % trouvés | 77,5 | 4,6 | 17,9 |

Spectre de RMN : (DMSO, 250 MHz ppm)

| | |
|---|---|
| N-<u>CH</u>=N | 8,45 (s) |
| aromatiques (benzimidazole) | 7,21 (m) 2H<br>7,46 (m) 1H<br>7,69 (m) 1H |
| les autres aromatiques | 7,45 (d)<br>7,83 (d) |
| N-<u>CH</u>$_2$-$C_6H_4$ | 5,64 |

**<u>EXEMPLE 17</u> : Acide 4-((1H-benzimidazol-1-yl) méthyl) benzoïque**

On opère comme à l'exemple 12 à partir de 921 mg du composé obtenu à l'exemple 16. On obtient 888 mg du produit recherché. F > 260°C.

On a préparé un échantillon analytique en recristallisant deux fois le produit ci-dessus, dans l'isopropanol puis dans l'acétate d'éthyle. On recueille 390 mg de produit pur, F > 260°C.

| Analyse pour $C_{15}H_{12}N_2O_2$ = 252,28 | C | H | N |
|---|---|---|---|
| % calculés | 71,42 | 4,79 | 11,11 |
| % trouvés | 71,2 | 4,7 | 11,1 |

Spectre de RMN : (DMSO, ppm)

| | |
|---|---|
| N-<u>CH</u>$_2$-$C_6H_4$ | 5,62 (s) |
| N-<u>CH</u>- | 8,46 |
| aromatiques | 7,22 (m) 2H<br>7,50 (m) 1H<br>7,70 (m) 1H |
| les autres aromatiques | 7,39 (d)<br>7,93 (d) |
| H mobile | 13,02 |

**<u>EXEMPLE 18</u> : 4-((2-butyl-1H-imidazo(4,5-c) pyridin-1-yl) méthyl) benzonitrile (produit A) et 4-((2-butyl-1H-imidazo(3,4-c) pyridin-1-yl) méthyl) benzonitrile (produit B)**

<u>STADE A</u> : 2-butyl-1H-imidazo(4,5-c) pyridine

On chauffe à 170°C pendant 18 heures un mélange de 3 g de 3,4-diaminopyridine et 8,28 g d'acide valérique. On chromatographie le milieu réactionnel sur silice, (éluant : acétate d'éthyle-méthanol (8-2)).

On obtient 4,8 g de produit attendu utilisé tel quel pour le stade suivant.

STADE B : 4-((2-butyl-1H-imidazo(4,5-c) pyridin-1-yl) méthyl) benzonitrile (produit A) et 4-((2-butyl-1H-imidazo(3,4-c) pyridin-1-yl) méthyl)benzonitrile (produit B)

On opère comme au stade B de l'exemple 1, à partir de 4,09 g du produit obtenu au stade A ci-dessus en utilisant 4,58 g de 4-bromométhyl benzonitrile. On obtient 8,5 g de produit brut que l'on chromatographie sur silice (éluant : chlorure de méthylène-méthanol (9-1)). On obtient 290 mg de produit B (cristallisé de l'éther) F = 130°C et 380 mg de produit A (cristallisé de l'éther) F = 164°C.

| Analyse : | Produit A | Produit B |
|---|---|---|
| RMN $CDCl_3$ 250 MHz | | |
| $CH_3$- | 0,93 (t) | 0,94 (t) |
| $CH_3$-$\underline{CH_2}$-$\underline{CH_2}$ | 1,44 - 1,84 (m) | 1,43 et 1,85 (m) |
| >C-$\underline{CH_2}$- | 2,82 (m) | 2,85 (m) |
| N-$\underline{CH_2}$-$C_6H_4$ | 5,41 (s) | 5,48 (s) |
| hétéroatome : | 9,08 (s) | 7,68 (d,l,j=6) |
| | 8,38 (d,j=6) | 8,45 (d,j=6) |
| | 7,10 (d) masqué | |
| aromatiques | 7,13-7,64 (d,l,j=8) | 7,16-7,65 (d,l) |

EXEMPLE 19 : Acide 4-((2-butyl-1H-imidazo(4,5c) pyridin-1-yl) méthyl) benzoïque

On opère comme à l'exemple 12 à partir de 320 mg du produit A, obtenu à l'exemple 18, on obtient 320 mg de produit brut F - 210°C, on recristallise ce produit dans l'isopropanol à 40 % d'eau, on obtient 250 mg de produit recherché F = 246°C. Ce produit est recristallisé une nouvelle fois dans 10 cm³ d'isopropanol, on recueille 180 mg de composé recherché F = 246°C.

Analyse pour $C_{18}H_{19}N_3O_2$ = 309,37

| | C | H | N |
|---|---|---|---|
| % calculés | 69,88 | 6,19 | 13,58 |
| % trouvés | 70,0 | 6,2 | 13,6 |

Spectre de RMN : (DMSO, 250 MHz ppm)

| | |
|---|---|
| $CH_3$- | 0,85 (t) |
| $CH_3$-$\underline{CH_2}$-$\underline{CH_2}$ | 1,35 - 1,71 (m) |
| -$\underline{CH_2}$-C< | 2,85 (t) |
| >N-$\underline{CH_2}$-$C_6H_4$ | 5,64 (s) |
| hétérocycle | 8,90 (s) 8,29 (d) 7,56 (d) |
| aromatique | 7,20 et 7,92 (d) |

EXEMPLE 20 : Acide 4-((2-butyl-3H-imidazo(4,5c) pyridin-3-yl) méthyl) benzoïque

On opère comme à l'exemple 12 à partir du produit B obtenu à l'exemple 18. On obtient 170 mg de produit F = 200°C, après la recristallisation dans l'isopropanol 216°C.

Analyse pour $C_{18}H_{19}N_3O_2$ = 309,37

| | C | H | N |
|---|---|---|---|
| % calculés | 69,88 | 6,19 | 13,58 |
| % trouvés | 70,1 | 6,2 | 13,5 |

Spectre de RMN : (DMSO, 250 MHz ppm)

| | |
|---|---|
| $CH_3$- | 0,85 (t) |
| $CH_3$-<u>$CH_2$</u>-<u>$CH_2$</u> | 1,35 - 1,71 (m) |
| -<u>$CH_2$</u>-C< | 2,87 (t) |
| >N-<u>$CH_2$</u>-$C_6H_4$ | 5,69 (s) |
| hétérocycle | 7,60 (d) 8,30 (d) 8,80 (s) |
| aromatique | 7,22 (d) - 7,91 (d) |
| H mobile | 12,95 |

**<u>EXEMPLE 21</u> : 4-((2-(1-buténYl) 1H-benzimidazol-1-yl) méthyl) benzonitrile**

<u>STADE A</u> : 4-((2-(1-bromobutyl) 1H-benzimidazol-1-yl) méthyl) benzonitrile

On agite pendant 2 heures à 60°C, sous irradiation d'une lampe de 60 watts une suspension composée de : 1,16 g du produit obtenu à l'exemple 4, avec 36 cm³ de tétrachlorure de carbone, 712 mg de N-bromosuccinimide et quelques cristaux de peroxyde de benzyle. On refroidit, extrait au chlorure de méthylène, lave avec une solution saturée de bicarbonate de sodium, puis à l'eau, sèche et évapore à sec sous pression réduite. On obtient 2,1 g de résidu que l'on chromatographie sur silice (éluant : acétate d'éthyle-cyclohexane (1-1). On obtient 600 mg de produit recherché F = 132°C. Un échantillon analytique a été préparé par recristallisation de 100 mg du produit obtenu ci-dessus, dans 20 cm³ d'éther. On obtient 58 mg du produit attendu F = 135°C.

Spectre de RMN : ($CDCl_3$, 250 MHz)

| | |
|---|---|
| $CH_3$- | 0,91 (t) |
| <u>$CH_2$</u>-$CH_3$ | 1,49 (m) |
| <u>$CH_2$</u>-$CH_2$-$CH_3$ | 2,50 (m) |
| $CH_2$-CH-C=<br>X | 4,95 (d,d,j=7 et 7,5) |
| N-$CH_2$ | 5,56 (AB) J = 17,5) |
| aromatiques | 7,19 (d,m)<br>7,63 (d,m)<br>7,85 (d,d) |
| autres aromatiques | 7,10 à 7,37 (m) |

Analyse pour $C_{19}H_{18}BrN_3$ = 368,27

| | Br |
|---|---|
| % calculés | 21,7 |
| % trouvés | 21,4 |

STADE B : 4-((2-(1-buténiyl) 1H-benzimidazol-1-yl) méthyl) benzonitrile

A une solution de 1,10 g de produit obtenu comme au stade A, dans 5 $cm^3$ de diméthylformamide, on ajoute 1,33 g de carbonate de lithium et 1,56 g de bromure de lithium. On agite pendant 15 minutes au reflux. On évapore le diméthylformamide et chromatographie le résidu sur silice (éluant : cyclohexaneacétate d'éthyle (1-1)). On obtient 480 mg de produit attendu. F = 125°C, cristallisé de l'éther.

Spectre de RMN : ($CDCl_3$, 250 MHz)

| | |
|---|---|
| $CH_3$- | 1,10 (t) |
| $\underline{CH_2}$-$CH_3$ | 2,31 (m) |
| N-$CH_2$ | 5,44 (s) |
| -$\underline{CH}$=CH-$CH_2$ (delta E) | 6,31 (dt,J= 15,5 et 1,5) |
| aromatiques | 7,19 (d,m)<br>7,61 (d,m)<br>7,77 (d,1) |
| 3 autres aromatiques et l'autre -CH= | 7,11 à 7,32 (m) |

**EXEMPLE 22 : Acide 4-((2-(1-buténiyl) 1H-benzimidazol-1-yl) méthyl) benzoïque**

On opère comme à l'exemple 12 à partir de 430 mg de produit obtenu à l'exemple 21. On obtient 450 mg de produit recherché. F = 210°C puis 225°C.

Un échantillon analytique a été obtenu par recristallisations successives dans le méthanol aqueux, l'acétate d'éthyle et enfin le méthanol aqueux légèrement acidifié à l'acide acétique. On recueille ainsi 150 mg de produit attendu.
F = 240°C.

Analyse pour $C_{19}H_{18}N_2O_2$ = 306,35

| | C | H | N |
|---|---|---|---|
| % calculés | 74,49 | 5,92 | 9,15 |
| % trouvés | 74,6 | 6,0 | 9,0 |

Spectre de RMN : (DMSO, 250 MHz)

| | | |
|---|---|---|
| $CH_3$- | 1,06 (t) | |
| $\underline{CH_2}$-$CH_3$ | 2,28 (m) | |
| N-$\underline{CH_2}$ | 5,67 (s) | |
| $CH_3$-$\underline{CH_2}$-$\underline{CH}$=CH- | 6,67 (d,j=16 Hz) | delta E |
| $CH_3$-$CH_2$-CH=$\underline{CH}$- | 7,05 (d,t) J = 16 et 6 Hz | |
| H en ortho de COO | 7,89 (d) | |
| autres aromatiques | 7,48 (m) - 7,60 (m) - 7,1 à 7,3 (m) | |
| Hydrogène mobile | 12,95 | |

**EXEMPLE 23 : 4-((2-butyl-3H-imidazo(4,5-b) pyridin-1-yl) méthyl) benzonitrile (Produit A) et 4-((2-butyl-3H-imidazo-(4,5-b) pyridin-3-yl) méthyl) benzonitrile (Produit B)**

STADE A : 2-butyl-3H-imidazo(4,5-b) pyridin

On opère comme au stade A de l'exemple 1 à partir de 3,27 g de 2,3-diaminopyridine en utilisant 6,5 $cm^3$ d'acide valérique. Après chromatographie sur silice (éluant : acétate d'éthyle méthanol (8-2)), on obtient 5,7 g de produit que l'on traite avec du charbon actif dans l'éther éthylique et que l'on cristallise dans 20 $cm^3$ d'éther isopropylique. On obtient 3,9 g de produit recherché. F = 104°C. Un échantillon analytique a été obtenu par recristallisation de 300 mg du produit, ci-dessus, dans l'éther éthylique, on obtient 240 mg du produit recherché. F = 104°C.

Analyse pour $C_{10}H_{13}N_3$ = 175,235

| | C | H | N |
|---|---|---|---|
| % calculés | 68,54 | 7,48 | 23,98 |
| % trouvés | 68,3 | 7,5 | 23,7 |

STADE B : 4-((2-butyl-3H-imidazo(4,5-b) pyridin-1-yl) méthyl) benzonitrile (Produit A) et 4-((2-butyl-3H-imidazo(4,5-b) pyridin-3-yl) méthyl) benzonitrile (Produit B)

On opère comme au stade B de l'exemple 1 à partir de 700 mg de produit obtenu au stade A ci-dessus, en utilisant 200 mg d'hydrure de sodium à 50 % dans l'huile et 800 mg de 4-bromobenzonitrile.

Après chromatographie sur silice (éluant : chlorure de méthylène-méthanol (9-1)), on obtient :

Fraction A : 510 mg cristallisé de l'éther éthylique (F = 130°C) que l'on recristallise dans 3 $cm^3$ d'acétate d'éthyle pour obtenir 280 mg de produit recherché.
F = 130°C.

Fraction B : 170 mg cristallisé de l'éther éthylique F = 103°C. Après recristallisation dans 3 $cm^3$ d'éther éthylique, on recueille 155 mg du produit recherché. F = 103°C.

Analyse fraction A pour $C_{18}H_{18}N_4$ = 290,37

| | C | H | N |
|---|---|---|---|
| % calculés | 74,46 | 6,25 | 19,29 |
| % trouvés | 74,2 | 6,2 | 19,1 |

Analyse fraction B pour $C_{18}H_{18}N_4$ = 290,37

| | C | H | N |
|---|---|---|---|
| % calculés | 74,46 | 6,25 | 19,29 |
| % trouvés | 74,4 | 6,2 | 19,2 |

**EXEMPLE 24 : Acide 4-((2-butyl) 1H-imidazo(4,5b) pyridin-1-yl) méthyl) benzoïque**

On opère comme à l'exemple 12 à partir de 250 mg du produit obtenu au stade précédent, fraction A, en utilisant 0,7 $cm^3$ d'hydroxyde de sodium. On obtient 210 mg du produit attendu, F = 190°C, que l'on recristallise deux fois successives dans l'isopropanol pour obtenir 140 mg de produit recherché. F = 200°C.

Analyse pour $C_{18}H_{19}N_3O_2$ = 309,37

| | C | H | N |
|---|---|---|---|
| % calculés | 69,88 | 6,19 | 13,58 |
| % trouvés | 69,7 | 6,2 | 13,3 |

Spectre IR (Nujol)
Absorption OH/NH

| | |
|---|---|
| >C=O | 1699 $cm^{-1}$ |
| Système conjugué | 1614 $cm^{-1}$ |
| + | 1580 $cm^{-1}$ |
| aromatique | 1508 $cm^{-1}$ |

**EXEMPLE 25 : Acide 4-((2-butyl 3H-imidazo(4,5b) pyridin-3-yl) méthyl) benzoïque**

On opère comme à l'exemple 12 à partir de 130 mg de la fraction B obtenue à l'exemple 23. On obtient 110 mg de produit attendu, F = 180°C, cristallisé d'un mélange isopropanol-eau. Produit que l'on recristallise dans 2 $cm^3$ d'éther éthylique, on recueille 80 mg de produit recherché. F = 180°C.

Analyse pour $C_{18}H_{19}N_3O_2$ = 309,37

| | C | H | N |
|---|---|---|---|
| % calculés | 69,88 | 6,19 | 13,58 |
| % trouvés | 69,6 | 6,2 | 13,4 |

Spectre IR Nujol
Absorption région OH/NH

| | |
|---|---|
| >C=O | 1698 $cm^{-1}$ |
| C=C + | 1604 $cm^{-1}$ |
| C=N + | 1598 $cm^{-1}$ |
| aromatique | 1578 $cm^{-1}$ |
| | 1502 $cm^{-1}$ |

**EXEMPLE 26 : 4'-((2-butyl-3H-imidazo(4,5b) pyridin-3-yl) méthyl) (1,1'-biphényl) 2-carboxylate de méthyle (fraction A) et 4'-((2-butyl-1H-imidazo(4,5b) pyridin-3-yl) méthyl 1,1'-biphényl) 2-carboxylate de méthyle (fraction B)**

On opère comme au stade B de l'exemple 1 à partir de 876 mg du produit obtenu au stade A de l'exemple 23 en utilisant 250 mg d'hydrure de sodium à 50 % dans l'huile et 1,53 g de 4'-bromo-(1,1'-biphényl) 2-carboxylate de méthyle. Après chromatographie sur silice (éluant : chlorure de méthylène-méthanol (9-1)), on obtient 1,2 g du composé fraction A, F = 135°C et 450 mg du composé fraction B.

On recristallise 100 mg du composé A dans l'éther éthylique, on recueille 80 mg, F = 140°C.

Spectre de RMN : ($CDCl_3$, 250 MHz)

| | |
|---|---|
| $CH_2-CH_2-CH_2-\underline{CH_3}$ | 0,94 (t) |
| $CH_2-\underline{CH_2}-\underline{CH_2}-CH_3$ | 1,46 - 1,89 (m) |
| $\underline{CH_2}-CH_2-CH_2-CH_3$ | 2,92 (m) |

| | | |
|---|---|---|
| $N-\underline{CH_2}-C_6H_4$ | | 5,39 (s) |
| 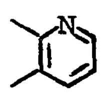 | 2H | 8,51 (dd,J = 5 et 1) |
| | | 7,11 (dd,J = 8 et 5) |
| **Fraction B :** | | |
| $CH_2-CH_2-CH_2-\underline{CH_3}$ | | 0,93 (t) |
| $CH_2-\underline{CH_2}-\underline{CH_2}-CH_3$ | | 1,42 - 1,82 (m) |
| $\underline{CH_2}-CH_2-CH_2-CH_3$ | | 2,84 (m) |
| $N-\underline{CH_2}-C_6H_4$ | | 5,55 (s) |
| | | 8,02 (dd,J = 8 et 1,5) |
| 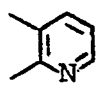 | 3H | 7,11 (dd,J = 8 et 5) |
| | | 8,36 (dd,J = 5 et 1,5) |
| Les aromatiques | | 7,18 (dl) à 7,24 (d,l) 4H |
| | | 7,30 (d,l) 1H |
| | | 7,40 (d,t) 1H |
| | | 7,51 (d,t) 1H |
| | | 7,81 (d,t) 1H |

**EXEMPLE 27** : **4'-((2-butyl-1H-imidazo(4,5b) pyridin-1-yl) méthyl) (1,1'-biphényl) 2-carboxylique**

On opère comme à l'exemple 12 à partir de 680 mg du produit de fraction A, obtenu à l'exemple 26 en utilisant 0,7 cm³ d'hydroxyde de sodium concentré, on obtient 650 mg du produit recherché F = 170°C. On dissout 900 mg de produit obtenu comme ci-dessus dans 200 cm³ d'isopropanol au reflux, concentre à 20 cm³, ajoute 30 cm³ d'eau. Après essorage, on obtient 810 mg de produit (F = 205°C) que l'on recristallise à nouveau dans 5 cm³ d'éthanol. On obtient 640 mg du produit attendu F = 205°C.

Analyse pour $C_{24}H_{23}N_3O_2$ = 385,45

| | C | H | N |
|---|---|---|---|
| % calculés | 74,78 | 6,01 | 10,9 |
| % trouvés | 74,45 | 6,0 | 10,7 |

Spectre IR Nujol

Absorption complexe région NH/OH

| | |
|---|---|
| C=O | 1708 $cm^{-1}$ |
| | 1616 $cm^{-1}$ |
| aromatique | 1589 $cm^{-1}$ |
| hétéroatome | 1574 $cm^{-1}$ |
| | 1500 $cm^{-1}$ |

**EXEMPLE 28** : **Acide 4'-((2-butyl-3H-imidazo(4,5b) pyridin-3-yl) méthyl) (1,1'-biphényl) 2-carboxylique**

On opère comme à l'exemple 12 à partir de 410 mg du produit, fraction B, obtenu à l'exemple 26, en utilisant 0,4 cm³ de lessive de soude.

On obtient 320 mg de produit attendu (F = 185°C) que l'on recristallise dans 10 cm³ d'un mélange isopropanol-eau (1-1). On recueille 210 mg de produit F = 190°C. On traite le produit obtenu en solution dans l'acétate

d'éthyle avec du charbon actif et après filtration concentre à 3 $cm^3$, on obtient, après essorage : 140 mg du produit recherché. F = 190°C.

Analyse pour $C_{24}H_{23}N_3O_2$ = 385,45

| | C | H | N |
|---|---|---|---|
| % calculés | 74,78 | 6,01 | 10,9 |
| % trouvés | 74,6 | 5,9 | 10,7 |

Spectre IR (Nujol)

Absorption complexe OH/NH

| | |
|---|---|
| C=O | 1684 $cm^{-1}$ |
| aromatique hétéroatome | 1600 $cm^{-1}$<br>1517 $cm^{-1}$<br>1498 $cm^{-1}$ |

**Préparation des exemples 29 et 30**

STADE A : 2-nitro 3-thiophénamine

On dissout à la température ambiante 12,8 g de 2-nitro thiophène à 85 % (ALDRICH), 33,6 g de 4-amino-4H-1,2,4 triazole (1-1, 3-4) (FLUKA) dans 100 $cm^3$ de diméthylsulfoxyde anhydre.

On refroidit à 5°C et ajoute en 15 minutes environ la solution de 22,4 g de terbutylate de potassium dans 100 $cm^3$ de diméthylsulfoxyde anhydre.

La suspension obtenue est agitée encore environ 15 minutes à la température ambiante puis versée dans 0,6 litre de solution saturée en chlorure d'ammonium.

On extrait par 3 fois 500 $cm^3$ d'acétate d'éthyle, lave par 3 fois 400 $cm^3$ d'eau, sèche, filtre et évapore à sec.

Le produit obtenu est dissous dans 800 $cm^3$ de chlorure de méthylène. On filtre et évapore à sec.

Le produit est cristallisé dans 20 $cm^3$ d'éther isopropylique. On essore, lave par de l'éther isopropylique puis sèche à 80°C sous pression réduite.

On obtient 5,1 g de produit attendu F=159°C.

ANALYSE pour $C_4H_4N_2O_2S$ = 144,15

| | | | | |
|---|---|---|---|---|
| calculés : | C% 33,33 | H% 2,80 | N% 19,43 | S% 22,24 |
| trouvés : | 33,0 | 2,7 | 19,2 | 22,2 |

IR ($CHCl_3$)

| | |
|---|---|
| $-NH_2$ | 3510 $cm^{-1}$ + 3380 $cm^{-1}$ |
| | 1608 $cm^{-1}$ |
| $-NO_2$ | 1554 $cm^{-1}$ |
| | 1328 $cm^{-1}$ |

STADE B : (3-tertbutylcarbonate amino 2-nitro thiophène)

On dissout à température ambiante 1,3 g du produit obtenu au stade A dans 15 $cm^3$ de tétrahydrofuranne anhydre. On refroidit à +4°C et introduit en 10 minutes la solution de 2 g de diterbutyl dicarbonate (FLUKA) et 110 mg de diméthylaminopyridine dans 15 $cm^3$ de tétrahydrofuranne anhydre.

On agite encore 30 minutes puis évapore les solvants.

L'huile récupérée est purifiée par chromatographie sur silice (éluant : Acétate d'éthyle - Flugène : 2-8).

Le produit obtenu est cristallisé dans 3 $cm^3$ d'éther isopropylique.

On essore, lave à l'éther isopropylique et sèche sous pression réduite.

On obtient ainsi 2,13 g de produit attendu F=80°C.

<u>ANALYSE</u> pour $C_9H_{12}N_2O_4S$ = 244,27

| | | | | |
|---|---|---|---|---|
| calculés : | C% 44,25 | H% 4,95 | N% 11,47 | S% 13,13 |
| trouvés : | 44,4 | 4,9 | 11,5 | 13,2 |

<u>STADE C</u> : (3-tert-butylcarbonateamino 2-valérylamino thiophène)

On mélange à température ambiante 45 g de Nickel de Raney (PROLABO) préalablement lavé à l'eau, 50 cm³ d'anhydride valérique (MERCK) et 15 g du produit obtenu au stade B.

On hydrogène à la température ambiante pendant 6 heures, filtre, lave au chlorure de méthylène puis évapore à sec.

On ajoute 150 cm³ d'essence G, amorce la cristallisation, refroidit 1 heure à environ -10°C, essore et lave par l'essence G.

On sèche à environ 80°C sous pression réduite.

Poids : 12,5 g P.F = 122-124°C.

On obtient après cristallisation dans l'éther éthylique 30 mg de produit attendu à partir de 40 mg du produit ci-dessus.

F = 128°C

I.R.

| | |
|---|---|
| =C-NH | 3426 $cm^{-1}$ |
| >=O | 1698 $cm^{-1}$ |
| | 1676 $cm^{-1}$ |
| hétérocycle | 1592 $cm^{-1}$ |
| + | 1520 $cm^{-1}$ |
| amide II | 1500 $cm^{-1}$ |

<u>STADE D</u> : (trifluoroacétate de 2-tert-butylcarbonate amino 3-amino thiophène

On introduit dans 25 cm³ d'acide trifluoroacétique (FLUKA) en 10 minutes à environ +5°C, 8,5 g du produit obtenu au stade C puis on agite à la température ambiante pendant 50 minutes.

On évapore à sec.

On dissout l'huile obtenue, dans 500 cm³ d'éther éthylique à reflux, traite avec du charbon actif et filtre. On concentre à environ 30 cm³, ajoute 30 cm³ d'éther isopropylique, concentre à environ 40 cm³ cristallise, essore, lave par de l'éther isopropylique puis par du chlorure de méthylène.

On sèche sous pression réduite à environ 70°C.

Poids : 5,13 g P.F = 100°C.

Par recristallisation dans l'éther éthylique de 370 mg du produit ci-dessus, on obtient 240 mg de produit attendu.

F = 100°C

<u>ANALYSE</u> pour $C_{11}H_{15}F_3N_2O_3S$ = 312,313

| | | | | | |
|---|---|---|---|---|---|
| calculés : | C% 42,30 | H% 4,84 | F% 18,25 | N% 8,97 | S% 10,27 |
| trouvés : | 42,2 | 4,8 | 18,6 | 8,9 | 10,3 |

<u>IR</u> (Nujol)

- Absorption complexe région OH/NH

>=O 1654 $cm^{-1}$ complexe

| | |
|---|---|
| hétéroaromatique | 1602 $cm^{-1}$ |
| Amide II | 1557 $cm^{-1}$ |
| | 1508 $cm^{-1}$ |

$CF_3$ : présent

STADE E : 2-butyl thiéno (2,3) imidazole

On dissout à la température ambiante 940 mg du produit obtenu au stade D dans 20 $cm^3$ d'eau. On ajoute 40 $cm^3$ d'une solution saturée en carbonate de sodium. On extrait par 3 fois avec 60 $cm^3$ de chlorure de méthylène, sèche, filtre et évapore. On dissout l'huile obtenue, à la température ambiante, dans 3 $cm^3$ d'oxychlorure de phosphore. On agite 10 minutes à la température ambiante, puis chauffe 1 heure à reflux à une température extérieure d'environ 115°C.

On évapore l'excès de réactif, ajoute 30 $cm^3$ de solution saturée en bicarbonate de sodium, extrait par 3 fois avec 60 $cm^3$ de chlorure de méthylène sèche, traite avec du charbon actif et évapore à sec.

Le produit obtenu est dissous dans 300 $cm^3$ d'éther éthylique à reflux, filtre, concentre à 5 $cm^3$. Après cristallisation, on essore, lave par quelques gouttes d'éther éthylique et sèche sous pression réduite à environ 100°C.

On obtient 400 mg de produit attendu.
F = 160°C

ANALYSE pour $C_9H_{12}N_2S$ = 180,27

| | | | | |
|---|---|---|---|---|
| calculés : | C% 59,96 | H% 6,71 | N% 15,54 | S% 17,79 |
| trouvés : | 60,1 | 6,8 | 15,5 | 17,8 |

**EXEMPLE 29 : 4'-[(2-butyl-1H-thiéno (2,3-d) imidazol 1-yl) méthyl]-(1,1'-biphényl)-2-carboxylate de méthyle (isomère A)**

**EXEMPLE 30 : 4'-[(2-butyl-3H-thiéno (2,3-d) imidazol-3-yl) méthyl]-(1,1'-biphényl)-2-carboxylate de méthyle (isomère B)**

On dissout à la température ambiante 360 mg du produit obtenu au stade E, de la préparation des exemples 29 et 30, dans 7 $cm^3$ de tétrahydrofuranne anhydre.

A la température ambiante, on ajoute en deux fois 96 mg d'hydrure de sodium à 50 % dans l'huile.

Après dégagement d'hydrogène, on agite encore 10 minutes sous azote puis ajoute en 1 fois 610 mg de bromométhyl-(1,1'-biphényl) 2-carboxylate de méthyle (préparé selon EP 0253.310).

On agite à la température ambiante pendant environ 35 minutes puis évapore le solvant sous azote. On ajoute 20 $cm^3$ d'eau, extrait par 3 fois avec 60 $cm^3$ de chlorure de méthylène, lave par 2 fois avec 10 $cm^3$ d'eau, sèche la phase organique, filtre et évapore.

Par chromatographie sur silice (éluant : acétate d'éthyle 50/cyclohexane 50) on récupère les deux isomères attendus qui constituent l'exemple 29 ou isomère A et l'exemple 30 ou isomère B.

On récupère ainsi 600 mg d'isomère A (Rf=0,40) et 250 mg d'isomère B (Rf=0,28)

RMN isomère A $CDCl_3$ 250 MHz ppm
$CH_3$ 0,96 (t)
$CH_3$-<u>$CH_2$</u>-(<u>$CH_2$</u>)$_2$- 1,44 (m)
$CH_3CH_2$-<u>$CH_2$</u>-$CH_2$ 1,80 (m)
$CH_3$-$(CH_2)_2$-<u>$CH_2$</u> 2,82 (t)
$CO_2$<u>$CH_3$</u> 3,63 (s)
N-<u>$CH_2$</u>-$C_6H_4$ 5,29 (s)
S-<u>$CH_2$</u>-$CH_2$ 6,96 (d)
S-$CH_2$-<u>$CH_2$</u> 6,64 (d)

RMN (isomère B) $CDCl_3$ 250 MHz ppm
$CH_3$ 0,96 (t)
$CH_3$-<u>$CH_2$</u>-$(CH_2)_2$ 1,47 (m)
$CH_3$-$CH_2$-<u>$CH_2$</u>-$CH_2$ 1,84 (m)

$CH_3$-$(CH_2)_2$-$\underline{CH_2}$ 2,87 (t)
$CO_2\underline{CH_3}$ 3,63 (s)
N-$\underline{CH_2}$-$C_6H_4$ 5,24 (s)
S-$\underline{CH_2}$-$CH_2$- 7,11 (d)
S-$CH_2$-$\underline{CH_2}$- 6,87 (d)

**<u>EXEMPLE 31</u> : acide 4'-[(2-butyl-1H-thiéno (2,3-d) imidazol-1-yl) méthyl]-(1,1'-biphényl)-2-carboxylique**

On dissout 600 mg du produit de l'exemple 29 (isomère A) à la température ambiante dans 7 $cm^3$ de méthanol. On ajoute 0,5 $cm^3$ de lessive de soude (10 N). On chauffe 1 heure à reflux, évapore les solvants et dissout l'extrait sec dans 15 $cm^3$ d'eau.

On refroidit légèrement par un bain d'eau froide et ajoute de l'acide acétique glacial jusqu'à pH de 4-5.

On essore, lave à l'eau et sèche sous pression réduite à environ 100°C.

On obtient 400 mg du produit attendu.

F = 203°C

<u>ANALYSE</u> pour $C_{23}H_{22}N_2O_2S$ = 390,506

| | C% | H% | N% | S% |
|---|---|---|---|---|
| calculés : | 70,74 | 5,68 | 7,17 | 8,21 |
| trouvés : | 70,8 | 5,6 | 7,1 | 8,2 |

<u>IR</u> (Nujol)

| | |
|---|---|
| Acide avec >=O | 1704 $cm^{-1}$ |
| Aromatique | 1595 $cm^{-1}$ |
| Hétéroaromatique | 1504 $cm^{-1}$ |
| | 1516 $cm^{-1}$ |
| | 1482 $cm^{-1}$ |

**<u>EXEMPLE 32</u> : acide 4'-[(2-butyl-3H-thiéno (2,3-d) imidazol-3-yl) méthyl]-(1,1'-biphényl)-2-carboxylique**

On procède comme à l'exemple 31 à partir de 250 mg du produit de l'exemple 30 (isomère B) dans 4 $cm^3$ de méthanol puis addition de 0,3 $cm^3$ de soude (10 N).

On obtient 160 mg du produit attendu.

F = 203°C

<u>ANALYSE</u> pour $C_{23}H_{22}N_2O_2S$ = 390,506

| | C% | H% | N% | S% |
|---|---|---|---|---|
| calculés : | 70,74 | 5,68 | 7,17 | 8,21 |
| trouvés : | 70,6 | 5,7 | 7,0 | 8,4 |

<u>IR</u> (Nujol)

| | |
|---|---|
| >=O | 1710 $cm^{-1}$ |
| Aromatique | 1598 $cm^{-1}$ |
| Hétéroaromatiques | 1565 $cm^{-1}$ |
| | 1526 $cm^{-1}$ |
| | 1515 $cm^{-1}$ |

**Préparation de l'exemple 33**

<u>STADE A</u> : 3-amino 4-valérylamino thiophène

On mélange à la température ambiante 3,06 g de 3,4-diaminothiophène de JANSSEN dans 45 $cm^3$ de chlorure de méthylène et 9 $cm^3$ de triéthylamine.

On ajoute à la température de 20°C en une heure environ, la solution de 3,6 g de chlorure de valéroyle de

FLUKA dans 45 $cm^3$ de chlorure de méthylène.

On agite encore 30 minutes puis évapore à sec. Après séparation par chromatographie (éluant : méthanol/chlorure de méthylène : 5/95), on obtient 3,2 g du monoamide attendu que l'on cristallise dans 15 $cm^3$ d'un mélange 50/50 d'éther isopropylique/cyclohexane, essore, lave avec ce mélange et sèche sous pression réduite à environ 100°C.

On obtient 2,14 g du produit attendu.

F = 110°C

<u>ANALYSE</u> pour $C_9H_{14}N_2OS$ = 198,288

| | | | | |
|---|---|---|---|---|
| calculés : | C% 54,52 | H% 7,12 | N% 14,13 | S% 16,17 |
| trouvés : | 54,4 | 7,1 | 13,9 | 16,2 |

<u>IR</u> ($CHCl_3$)

NH (3411 $cm^{-1}$)
(3325 $cm^{-1}$)

>=O 1678 $cm^{-1}$

Système conjugué + Amide II + $NH_2$ déformation: 1615 $cm^{-1}$, 1563 $cm^{-1}$, 1524 $cm^{-1}$, 1502 $cm^{-1}$

<u>STADE B</u> : 2-butyl thiéno[3,4]imidazole

On dissout à la température ambiante sous agitation 1 g du produit obtenu au stade A, dans 3 $cm^3$ d'oxychlorure de phosphore puis on chauffe à reflux dans un bain à environ 120°C pendant environ 1 heure.

On évapore à sec, ajoute 100 $cm^3$ d'une solution aqueuse saturée en bicarbonate de sodium, agite 20 minutes à la température ambiante, extrait par deux fois avec 100 $cm^3$ de chlorure de méthylène, lave par 50 $cm^3$ de solution de bicarbonate de sodium, sèche la phase organique, filtre et évapore.

L'huile obtenue est dissoute dans 600 $cm^3$ d'éther éthylique à reflux. On filtre et évapore à sec. On obtient 570 mg de produit que l'on cristallise dans 2 $cm^3$ d'éther isopropylique. On obtient 500 mg du produit attendu.

F = 118°C

RMN $CDCl_3$ 60 MHz ppm

S-<u>$CH_2$</u> 6,71

-<u>$CH_2$</u> 2,68-2,80-2,91

les $CH_2$ centraux 1,1 à 2,13

$CH_3$ 0,83-0,93-1,03

**<u>EXEMPLE 33</u> : (4'-((2-butyl-1H-thièno (3,4-d)imidazol-1-yl) méthyl)-(1,1'-biphényl)-2-carboxylate de méthyle)**

On dissout à la température ambiante 500 mg du produit obtenu au stade B de la préparation de l'exemple 33, dans 10 $cm^3$ de tétrahydrofuranne anhydre.

On ajoute 120 mg d'hydrure de sodium à 50 %, agite jusqu'à fin de dégagement d'hydrogène puis sous azote pendant 10 minutes et ajoute 750 mg de bromo méthyl(1,1'biphényl) 2-carboxylate de méthyle (préparé selon EP 0253.310).

On agite à la température ambiante pendant environ 1 heure, évapore à sec, extrait par 3 fois avec 100 $cm^3$ de chlorure de méthylène, lave par deux fois avec 50 $cm^3$ d'eau. On sèche, filtre et évapore à sec. Après chromatographie sur 400 g de silice (éluant : Acétate d'éthyle 5 : cyclohexane 5). On récupère 1,14 g de produit attendu.

L'huile obtenue est cristallisée dans 2 $cm^3$ d'éther isopropylique. On essore, lave par quelques gouttes d'éther isopropylique et sèche sous pression réduite à 70°C.

Par deux recristallisation successives dans l'éther isopropylique, on obtient 70 mg du produit attendu.

F = 120°C

<u>ANALYSE</u> pour $C_9H_{12}N_2S$ = 180,27

| | C | H | N | S |
|---|---|---|---|---|
| % calculés : | 59,96 | 6,71 | 15,54 | 17,79 |
| % trouvés | 60,0 | 6,8 | 15,5 | 17,6 |

<u>EXEMPLE 34</u> : **Acide 4'-((2-butyl-1H-thiéno (3,4-d) imidazol-1-yl) méthyl)-(1,1'-biphényl)-2-carboxylique**

On dissout à la température ambiante sous agitation 1,14 g du produit obtenu à l'exemple 33, dans 25 $cm^3$ de méthanol. On ajoute 1 $cm^3$ de lessive de soude et chauffe à reflux pendant 1 heure.

On évapore le méthanol, dissout l'extrait sec obtenu, dans 75 $cm^3$ d'eau et ajoute de l'acide acétique glacial jusqu'à pH 6-5.

On agite encore 15 minutes, essore, lave à l'eau et sèche sous pression réduite à environ 80°C. On obtient ainsi 870 mg de produit attendu.

F = 168°C.

On dissout 1,02 g de produit obtenu comme ci-dessus, dans 50 $cm^3$ d'éthanol à reflux. On ajoute 1 goutte d'acide acétique puis 40 $cm^3$ d'eau. On cristallise, essore, lave par un mélange 50/50 eau/alcool et sèche sous pression réduite à environ 100°C. On obtient 900 mg du produit recherché.

F = 168°C

On dissout le produit obtenu ci-dessus dans 100 $cm^3$ d'acétate d'éthyle au reflux, filtre, concentre à environ 50 $cm^3$, cristallise, essore, lave à l'acétate d'éthyle et sèche sous pression réduite à environ 100°C. On obtient ainsi 700 mg de produit attendu.

F = 168°C

<u>ANALYSE</u> pour $C_{23}H_{22}N_2O_2S$ = 390,506

| | | | | |
|---|---|---|---|---|
| calculés : | C% 70,74 | H% 5,68 | N% 7,17 | S% 8,21 |
| trouvés : | 70,6 | 5,6 | 7,1 | 8,1 |

<u>IR</u> (Nujol)

| | |
|---|---|
| >=O | 1685 $cm^{-1}$ |
| Aromatique | 1597 $cm^{-1}$ |
| Hétéroatome | 1520 $cm^{-1}$ |
| | 1487 $cm^{-1}$ |

**Préparation de l'exemple 35**

<u>STADE A</u> : 5-bromo 2-butyl-1H-imidazole-4-méthanol

On introduit 10 g de 2-butyl-1H-imidazole-4-méthanol (obtenu selon EP 0.253.310) dans 150 $cm^3$ de dioxanne et 150 $cm^3$ de 2-méthoxyéthanol sous agitation. On ajoute 12,7 g de N-bromo succinimide et laisse sous agitation à environ 40°C pendant environ 2 heures.

On laisse refroidir le milieu réactionnel à la température ambiante pendant 1 heure 30. On évapore, reprend les cristaux dans 250 $cm^3$ d'acétate d'éthyle et 150 $cm^3$ d'eau. On extrait par deux fois avec 100 $cm^3$ d'acétate d'éthyle.

On lave par deux fois avec 100 $cm^3$ d'eau, séche, filtre, ajoute à la solution du charbon actif et agite pendant environ 10 minutes. On filtre et évapore. Les cristaux sont solubilisés dans 64 $cm^3$ d'acétate d'éthyle à chaud puis la solution est laissée 1 heure 30 à environ 0°C.

On essore, lave les cristaux par trois fois 6 $cm^3$ d'acétate d'éthyle puis par trois fois 6 $cm^3$ de chlorure de méthylène puis on essore et sèche. On obtient 4,39 g de produit attendu.

F = 160°C.

<u>RMN du proton (ppm, DMSO)</u>

0,88 (t) <u>$CH_3$</u>-$CH_2$-$CH_2$-$CH_2$-C=

1,29 (m) $CH_3$-$\underline{CH_2}$-$CH_2$-$CH_2$-C=
1,58 (m) $CH_3$-$CH_2$-$\underline{CH_2}$-$CH_2$-C=
2,55 (m) $CH_3$-$CH_2$-$CH_2$-$\underline{CH_2}$-C=
4,31 (sl) =C-$\underline{CH_2}$-OH
5,10 (tl) =C-$CH_2$-$\underline{OH}$
12,18 absorption large NH

STADE B : 5-bromo-2-butyl-1H-imidazole-4-carboxaldéhyde

On introduit 4,37 g du produit obtenu au stade A, dans 130 $cm^3$ de dioxanne puis ajoute sous agitation 16,32 g de dioxyde de manganèse.

Le mélange est chauffé à ≈ 100°C pendant environ 2 heures. On filtre, rince au dioxanne et évapore. On obtient 3,72 g du produit attendu.
F = 113°C

IR $CHCl_3$, $cm^{-1}$
=C-NH 3418 $cm^{-1}$
3222 $cm^{-1}$
>=O 1655 $cm^{-1}$
hétérocycle 1545 $cm^{-1}$
1504 $cm^{-1}$

STADE C : 4'-[[4-bromo-2-butyl-5-formyl-1H-imidazol-1-yl] méthyl]-(1,1'-biphényl)-2-carboxylate de méthyle

On introduit 3,7 g du produit obtenu au stade B dans 50 $cm^3$ de diméthylformamide. On ajoute sous agitation 2,465 g de bicarbonate de potassium puis laisse environ 5 minutes. On ajoute 5,86 g de bromométhyl (1-1'biphényl) 2-carboxylate de méthyle (préparé selon EP 0.253.310) dans 55 $cm^3$ de diméthylformamide.

Le mélange est laissé environ 3 jours à la température ambiante. On hydrolyse le milieu réactionnel par 100 $cm^3$ d'eau puis extrait par 3 fois avec 50 $cm^3$ d'acétate d'éthyle.

On lave par 100 $cm^3$ d'eau saturée en chlorure de sodium, sèche, filtre et évapore. Après chromatographie, (éluant acétate d'éthyle 5/chlorure de méthylène 95) on récupère 6,36 g de produit attendu.

IR $CHCl_3$, $cm^{-1}$
COOMe 1726 $cm^{-1}$
1434 $cm^{-1}$
C=O (aldéhyde conjugué) ≈ 1668 $cm^{-1}$
aromatique + hétérocycle 1600 $cm^{-1}$
1510 $cm^{-1}$
1483 $cm^{-1}$

**EXEMPLE 35 : 2-butyl-1-[(2'-(carboxy)]-(1,1'-biphényl)-4-yl) méthyl]-1H-thiéno (2,3-d) imidazole-5-carboxylate d'éthyle**

On introduit (149,5 mg X 2) d'éthylate de sodium dans 5 $cm^3$ d'éthanol et sous agitation ajoute (264 mg X 2) de mercapto acétate d'éthyle (préparé selon 0.153.229 brevet de TEUTSH ASZODI 85.211.781) dans 2 $cm^3$ d'éthanol.

On ajoute 500 mg du produit obtenu au stade C, dans 12 $cm^3$ d'éthanol. On chauffe à reflux à environ 78°C sous agitation pendant 3 heures. On élimine les solvants, ajoute 400 $cm^3$ d'eau et extrait par 5 fois 100 $cm^3$ de chlorure de méthylène. On sèche, filtre et évapore. Après chromatographie, (éluant acétate d'éthyle 5/chlorure de méthylène 95) on récupère 322,5 mg de produit attendu.

IR $CHCl_3$, $cm^{-1}$

| | | |
|---|---|---|
| >=O | 1716 $cm^{-1}$ | |
| | 1698 $cm^{-1}$ | |
| système conjugué | | 1600 $cm^{-1}$ |
| + | | 1529 $cm^{-1}$ |
| aromatique | | 1512 $cm^{-1}$ |

**EXEMPLE 36 : acide 2-butyl-1-((2'-carboxy-(1,1'-biphényl)-4-yl) méthyl)-1H-thiéno (2,3-d) imidazole-5-carboxylique**

On introduit 295,4 mg du produit de l'exemple 35 dans 10 $cm^3$ d'éthanol. On ajoute goutte à goutte 1,55 $cm^3$ de soude (2N) et laisse le mélange à température ambiante pendant environ 5 jours sous agitation. On évapore sous pression réduite et reprend dans 8 $cm^3$ d'eau.

On ajoute 1,55 $cm^3$ d'acide chlorhydrique (2N), filtre et séche sous pression réduite. Le produit est repris dans 9 $cm^3$ d'isopropanol à chaud. On ajoute 4 $cm^3$ d'eau, filtre et sèche. On obtient 140 mg de produit attendu. F = 264°C

IR nujol, $cm^{-1}$

absorption complexe région OH/NH

| | | | |
|---|---|---|---|
| >=O | 1708 $cm^{-1}$ | | |
| | 1650 $cm^{-1}$ | | |
| aromatique | | 1602 $cm^{-1}$ | |
| + | | 1519 $cm^{-1}$ | ep |
| hétéroaromatique | | 1504 $cm^{-1}$ | |

**Préparation de l'exemple 37**

STADE A : mercapto acétate de 1-1-diméthyléthyle

STADE ALPHA : thioacétate de 1,1-diméthyléthyle

On mélange 35,24 g de 0-éthyldithiocarbonate de potassium dans 150 $cm^3$ d'acétone à environ 0 à 2°C et ajoute 39,16 g de bromoacétate de 1-1 diméthyl éthyle en 10 minutes. Le mélange est agité à la température ambiante puis versé dans 800 $cm^3$ d'éther filtré et concentré.

Le résidu est repris dans 400 $cm^3$ d'éther, filtré et évaporé à nouveau pour donner 49,58 g de produit attendu.

STADE BETA : mercapto acétate de 1-1 diméthyléthyle

Le produit obtenu au stade alpha est refroidit à environ 0°C et on ajoute goutte à goutte 6,48 g de 1-2 diaminoéthane.

On agite environ 2 heures à la température ambiante et ajoute 200 $cm^3$ d'hexane, agite pendant environ 10 minutes et extrait le résidu à l'hexane. On lave la solution hexanique avec 200 $cm^3$ d'acide chlorhydrique (0,1N) puis avec 200 $cm^3$ de solution de bicarbonate de sodium, sèche et concentre.

Le résidu est distillé sous pression réduite et on obtient 21,71 g du produit attendu.

$$E_{b_{21\ mm/Hg}} = 72°C$$

STADE B : 3-amino 3[(2(1,1-diméthyléthoxy)-2-oxoéthyl) thio] 2-[(1-oxopentyl) amino] propénoate d'éthyle

On mélange 5 g de 2-[(1-oxo pentyl) amino] 2-cyano éthanoate d'éthyle, dans 45 $cm^3$ de chlorure de méthy-

lène puis ajoute 0,33 cm³ de triéthylamine et 2,15 g du produit obtenu au stade A. On agite à la température ambiante pendant environ 24 heures et obtient 8,49 g de produit attendu.

STADE C : 2-butyl-5-[(2(1,1-diméthyléthoxy)-2-oxoéthyl) thio]-1H-imidazole-4-carboxylate d'éthyle

On mélange 127 cm³ de chlorure de méthylène et 9,81 g de pentachlorure de phosphore. Le mélange est porté à environ -78°C et on ajoute goutte à goutte 6,33 g de diméthylaminopyridine solubilisé dans 59 cm³ de chlorure de méthylène. On agite pendant 5 minutes et ajoute 8,49 g du produit obtenu au stade B, puis agite de nouveau pendant environ 5 minutes à -78°C.

On laisse le milieu réactionnel revenir à la température ambiante et agite pendant environ 24 heures. Le milieu réactionnel est versé dans 400 cm³ de bicarbonate de soude et agite 20 minutes puis laisse décanter et extrait la phase aqueuse par 3 x 300 cm³ d'acétate d'éthyle.

Les phases organiques sont lavées par 500 cm³ d'eau, séchées, filtrées et évaporées sous pression réduite. On obtient 7,61 g d'huile que l'on purifie par chromatographie (éluant acétate d'éthyle 10/chlorure de méthylène 90). On obtient 3,49 g de produit attendu.
(Rf=0,26)

ANALYSE pour $C_{16}H_{26}O_4N_2S$ = 342,45

| | | | | |
|---|---|---|---|---|
| calculés : | C% 56,117 | H% 7,652 | N% 8,18 | S% 9,362 |
| trouvés : | 56,3 | 7,8 | 8 | 9,2 |

IR $CHCl_3$, $cm^{-1}$

-NH | 3438 $cm^{-1}$
| 3260 $cm^{-1}$

Système conjugée | 1544 $cm^{-1}$
| 1498 $cm^{-1}$

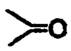 | 1726 $cm^{-1}$
| 1672 $cm^{-1}$ complexe

Me de tBu : 1369 $cm^{-1}$

STADE D : 2-butyl-1[(2'-(méthoxycarbonyl) (1,1'-biphényl)-4-yl) méthyl] 5-[(2(1,1-diméthyléthoxy) 2-oxoéthyl) thio]-1H-imidazole-5-carboxylate d'éthyle.

On mélange 1 g du produit obtenu au stade C, dans 6 cm³ de diméthylformamide et ajoute 450 mg de bicarbonate de potassium. On agite environ 5 minutes à la température ambiante puis introduit 1,07 g de bromométhyl-(1,1'-biphényl 2-carboxylate de méthyle (préparé selon EP 0.253.310) solubilisé dans 6 cm³ de diméthylformamide. Le mélange est laissé sous agitation à la température ambiante pendant environ trois jours.

Le milieu réactionnel est hydrolysé par 500 cm³ d'eau puis extrait par 3 fois 200 cm³ d'acétate d'éthyle. Les phases organiques sont lavées par 200 cm³ d'eau puis par 200 cm³ d'eau saturée en chlorure de sodium, séchées, filtrées et évaporées. On obtient 1,785 g d'huile que l'on purifie par chromatographie (éluant acétate 2/chlorure de méthylène 98). On obtient 1,381 g de produit attendu.
(Rf=0,26)

IR $CHCl_3$, $cm^{-1}$

- absence de NH
- C=O | 1726 $cm^{-1}$
  | 1692 $cm^{-1}$
- aromatique + 1600 $cm^{-1}$

hétérocycle 1577 $cm^{-1}$

1565 $cm^{-1}$

1512 $cm^{-1}$

1501 $cm^{-1}$

**EXEMPLE 37 : 2-butyl-6-hydroxy-1[(2'-(méthoxycarbonyl)-(1,1'-biphényl)-4-yl] méthyl] 1H-thiéno [4,5-b] imidazole-5-carboxylate de 1,1-diméthyléthyle**

On mélange 47 mg du produit obtenu au stade D, dans 1 cm³ de tétrahydrofuranne, porte la solution à environ -78°C et ajoute une solution lithium biotriméthyl silyle amide à 1 mole/l dans 0,42 cm³ de tétrahydrofuranne.

Le milieu réactionnel est laissé sous agitation à -78°C pendant environ 2 heures, puis on laisse revenir à la température ambiante pendant environ 2 heures. Le milieu réactionnel est refroidi à environ -78°C puis hydrolysé par une solution à 10 % d'acide acétique dans le tétrahydrofuranne.

On laisse revenir à la température ambiante évapore sous pression réduite et reprend dans 30 cm³ d'acétate d'éthyle. La phase organique est lavée par de l'eau puis de l'eau saturée en chlorure de sodium. La phase organique est séchée, filtrée et évaporée. On obtient 40 mg de produit que l'on purifie par chromatographie (éluant acétate d'éthyle 2/chlorure de méthylène 98) et obtient 16,5 mg de produit attendu.

RMN, 1H, ppm, $CDCl_3$, 250 MHz

0,92 (t) $CH_3$
1,40 (m) $CH_2$
1,76 (m) $CH_2$
2,78 (m) $CH_2-C=$
1,58 (s) $CO_2tBu$
3,62 (s) $CO_2CH_3$
5,40 (s) N $\underline{CH_2}$ $C_6H_4$

7,25 (m) 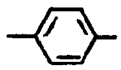
7,41 (dl) $H_3$
7,41 (dt) | $H_4$ $H_5$
7,53 (dt) |
7,84 (dd) $H_6$
10,55 (m) 1H mobile

**EXEMPLE 38 : 2-butyl-1-((2'-carboxy-(1,1'-biphényl)-4yl) méthyl)-6-hydroxy-1H-thiéno (2,3-d) imidazole-5-carboxylate de 1,1-diméthyléthyle**

On mélange 208,9 mg du produit de l'exemple 37 dans 12 cm³ d'éthanol et ajoute goutte à goutte 2 cm³ de soude 2N.

Le mélange est laissé à la température ambiante sous agitation pendant environ 3 jours. Le milieu réactionnel est évaporé puis repris par 10 cm³ d'eau à chaud.

On ajoute 2 $cm^3$ d'acide chlorhydrique 2N, filtre et sèche sous pression réduite pendant environ 24 heures. On obtient 185,6 mg de produit sous forme de poudre que l'on recristallise par solubilisation dans 14 $cm^3$ d'isopropanol à chaud, addition de 6 $cm^3$ d'eau et refroidit.

Les cristaux obtenus sont essorés, lavés à l'eau et séchés sous pression réduite à environ 40°C pendant environ 24 heures. On obtient 137,5 mg de produit attendu.
F=221°C - 222°C.

**EXEMPLE 39 : Acide 2-butyl-1-((2'-carboxy-(1,1'-biphényl)-4yl) méthyl)-6-hydroxy-1H-thiéno (2,3-d) imidazole-5-carboxylique**

Peut être préparé à partir de l'exemple 38 par saponification de la fonction ester $CO_2tBu$ pour donner COOH en position 5 du cycle thiéno.

**EXEMPLE 40 : 2-butyl-1-((2'-carboxy-(1,1'-biphényl)-4yl) méthyl)-1H-thiéno (2,3-d) imidazole-6-carboxylate de 1,1-diméthyléthyle**

Peut être préparé comme l'exemple 37.

**EXEMPLE 41 : 2-butyl-1-[(2'-(méthoxycarbonyl)-(1,1'-biphényl)-4yl] méthyl] 1H-thièno [4,5-b] imidazole-6-carboxylate de 1,1-diméthyléthyle**

Peut être préparé comme l'exemple 38.

**EXEMPLE 42 : Acide 2-butyl-1-((2'-carboxy-(1,1'-biphényl)-4yl) méthyl)-1H-thiéno (2,3-d) imidazole-6-carboxylique**

Peut être préparé comme l'exemple 39.

**EXEMPLE 43 de composition pharmaceutique**

On a préparé des comprimés répondant à la formule suivante :

Produit de l'exemple 10 .............................. 10 mg
Excipient pour un comprimé terminé à ................ 100 mg
(détail de l'excipient : lactose, talc, amidon, stéarate de magnésium)

**RESULTATS PHARMACOLOGIQUES**

1 - Test sur le récepteur de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g pendant 15 minutes avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, $MgCl_2$ 10 mM PMSF 0,3 mM, bacitracine 0,1 mM, BSA 0,2 %).

On répartit des fractions aliquotées de 2 $cm^3$ dans des tubes à hémolyse et ajoute de la $^{125}I$ angiotensine II (25 000 DPM/tube) et le produit à étudier. Le produit est d'abord testé à 3 x $10^{-5}$M en triple. Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à $10^{-5}$M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % ($CI_{50}$), c'est-à-dire en concentration de produit étudié, exprimée en nM, nécessaire pour déplacer 50 % de la radioactivité spécifique fixée sur le

récepteur étudié.

**Résultats :**

| Produit | $CI_{50}$ en nanomoles |
|---|---|
| 34 | 789 |
| 5 | 1440 |
| 10 | 2270 |
| 2 | 2350 |
| 20 | 2715 |
| 15 | 3610 |
| 6 | 3460 |
| 27 | 89 |

2 - Mise en évidence de l'activité antagoniste de l'angiotensine II sur la veine porte isolée

La veine porte est prélevée, sur des rats mâles Wistar (350 g environ) (IFFA Crédo France) après dislocation cervicale, et mise rapidement dans une solution physiologique (voir ci-dessous) à température ambiante. Un anneau de 1 mm environ est monté dans un bain à organe isolé contenant 20 $cm^3$ de la solution physiologique suivante (composition en mM : NaCl 118,3 - KCl 4,7 - $MgSO_4$ 1,2 - $KH_2PO_4$ 1,2 - $NaHCO_3$ 25 - glucose 11,1 - $CaCl_2$ 2,5) le milieu est maintenu à 37°C et oxygéné par un mélange $O_2$ (95 %), $CO_2$ (5 %). La tension initiale imposée est de 1 g, les anneaux sont laissés au repos pendant 60 à 90 minutes. Afin d'éviter les contractions spontanées, du vérapamil est ajouté au bain d'incubation ($1.10^{-6}$M).

A la fin de la période de repos l'angiotensine II (hypertensine Ciba) $3.10^{-8}$M est ajoutée dans le bain d'incubation et laissée au contact de la préparation pendant 1 minute. Cette opération est répétée chaque 30 minutes, le tissu étant lavé 3 ou 4 fois entre deux stimulations à l'angiotensine. Le composé à étudier est introduit dans le bain 15 minutes avant une nouvelle stimulation par l'angiotensine. Des concentrations croissantes de la molécule étant appliquées, une $CI_{50}$ (concentration qui produit une inhibition de 50 % de la réponse à l'angiotensine) peut être calculée, celle-ci est exprimée en nanomoles.

**Résultats :**

| Produit | $CI_{50}$ en nanomoles |
|---|---|
| 10 | 3280 |
| 2 | 3400 |
| 5 | 3600 |
| 25 | 830 |

**Revendications**

1) Produits de formule (I) :

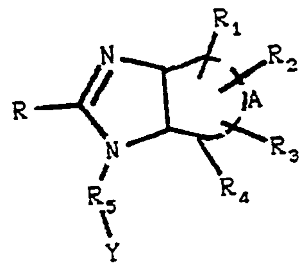

(I)

dans laquelle :

A représente le reste d'un radical monocyclique comprenant 3, 4 ou 5 chaînons ou d'un radical constitué de cycles condensés comprenant 6 à 12 chaînons, ces radicaux étant saturés ou insaturés et renfermant éventuellement un ou plusieurs hétéroatomes choisis parmi les atomes d'oxygène, et les atomes d'azote et de soufre éventuellement oxydés,

R représente un radical alkyle, alkényle ou alkynyle linéaire ou ramifié renfermant au plus 6 atomes de carbone et éventuellement substitués,

$R_1$, $R_2$, $R_3$ et $R_4$, identiques ou différents, représentent :

a) un atome d'hydrogène, un atome d'halogène, un radical hydroxyle, cyano, nitro, sulfo, formyle, benzoyle, acyle ou acyloxy ayant au plus 12 atomes de carbone, carboxy libre, salifié ou estérifié, mercapto,

b) un radical alkyle, alkényle, alkynyle, alkoxy ou alkylthio, ces radicaux étant linéaires ou ramifiés, renfermant au plus 6 atomes de carbone et étant éventuellement substitués,

c) un radical aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués,

d) un radical

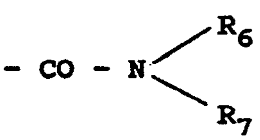 ou 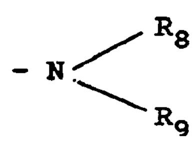

dans lesquels :

ou bien $R_6$ et $R_7$ ou $R_8$ et $R_9$, identiques ou différents, représentent :

– un atome d'hydrogène,

– un radical alkyle ou alkényle renfermant au plus 6 atomes de carbone et éventuellement substitué par un atome d'halogène, un radical hydroxyle ou un radical alkoxy renfermant au plus 6 atomes de carbone,

– un radical aryle ou arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié,

– un radical $-(CH_2)_m-SO_2-X-R_{14}$ dans lequel m représente un entier de 0 à 4 et

soit $X-R_{14}$ représente $NH_2$

soit X représente les radicaux -NH-, -NH-CO-HH- ou -NH-CO- ou une simple liaison

et $R_{14}$ représente un radical alkyle, alkényle et aryle, ces radicaux étant éventuellement substitués,

ou bien $R_6$ et $R_7$ ou $R_8$ et $R_9$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant

au plus 6 atomes de carbone, les radicaux carboxy libre, salifié ou estérifié,
ou bien $R_8$ et $R_9$, identiques ou différents, représentent un radical acyle dérivé d'acide carboxylique renfermant au plus 6 atomes de carbone, ou un radical alkyle ou arylsulfonyle dans lequelle radical alkyl renferme au plus 6 atomes de carbone et le radical aryle renferme au plus 8 atomes de carbone, ces radicaux étant éventuellement substitués par un ou plusieurs substituants choisis parmi les atomes d'halogène ou les radicaux alkyle renfermant au plus 6 atomes de carbone,

e) un radical $-(CH_2)_m-SO_2-X-R_{14}$ dans lequel m, X et $R_{14}$ ont la signification précédente,

$R_5$ représente un radical divalent alkylène, linéaire ou ramifié, renferment au plus 4 atomes de carbone,
Y représente le radical $-Y_1-B-Y_2$ dans lequel :
$Y_1$ représente un radical aryle monocyclique comprenant 5 ou 6 chaînons ou constitué de cycles condensés comprenant 8 à 10 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les radicaux que peuvent représenter $R_1$, $R_2$, $R_3$ et $R_4$,
B représente :
soit une simple liaison entre $Y_1$ et $Y_2$,
soit l'un des radicaux divalents suivants : -CO-, -NH-CO-, -CO-NH- ou $-O-(CH_2)_n-$ avec n représentant les valeurs 0 à 3,
$Y_2$ représente :
soit, quelle que soit la valeur de B autre qu'une simple liaison et $Y_2$ étant identique ou différent de $Y_1$, les valeurs définies pour $Y_1$,
soit, si B représente une simple liaison, un atome d'hydrogène, un radical cyano, carboxy libre, salifié ou estérifié, étant entendu que :
lorsque A représente un radical phényle éventuellement substitué par un ou deux radicaux choisis parmi les atomes d'halogène, les radicaux alkyle éventuellement substitué, alkoxy, acyle, carboxy libre ou phényle substitué par 5 atomes de fluor,
$R_5$ représente $-CH_2-$,
et Y représente le radical $-Y_1-B-Y_2$ dans lequel $Y_1$ représente un radical phényle non substitué et $Y_2$ représente un radical phényle substitué en ortho par un radical carboxy libre éventuellement salifié ou un radical tétrazolyle ou trifluorométhyl-sulfonamide et comportant éventuellement un second substituant choisi parmi les atomes d'halogène, les radicaux alkyle renfermant au plus 4 atomes de carbone, les radicaux nitro et méthoxy,
alors B représente -CO-NH- ou $-O-(CH_2)_n-$ avec n représentant les valeurs 2 ou 3,
lesdits produits de formule (I) étant sous toutes les formes isomères possibles racémiques, énantiomères et diastéréoisomères,
ainsi que les sels d'addition avec les acides minéraux et organiques ou avec les bases minérales et organiques desdits produits de formule (I).

**2)** Produits de formule (1) telle que définie à la revendication 1 caractérisés en ce que le ou les substituants, identiques ou différents que peuvent porter :

a) les radicaux alkyle, alkényle et alkynyle que peut représenter R,
b) les radicaux alkyle, alkényle, alkynyle, alkoxy et alkylthio que peuvent représenter $R_1$, $R_2$, $R_3$ et $R_4$,
c) les radicaux aryle, arylalkyle et arylalkényle que peuvent représenter $R_1$, $R_2$, $R_3$ et $R_4$
d) les radicaux alkyle, alkényle et aryle que peut représenter $R_{14}$,
sont choisis dans le groupe formé par :

– les atomes d'halogène, les radicaux hydroxyle, cyano, nitro, formyle, acyle ou acyloxy ayant au plus 6 atomes de carbone, benzoyle, carboxy libre, salifié ou estérifié par un radical alkyle renfermant au plus 6 atomes de carbone,
– les radicaux alkyle et alkényle renfermant au plus 6 atomes de carbone et éventuellement substitués par un ou plusieurs substituants choisis parmi les atomes d'halogène, le radical hydroxyle et les radicaux alkoxy renfermant au plus 6 atomes de carbone,
– les radicaux alkoxy linéaires et ramifiés renfermant au plus 6 atomes de carbone,
– les radicaux aryle et arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone,les radicaux carboxy libre, salifié ou estérifié,
– les radicaux

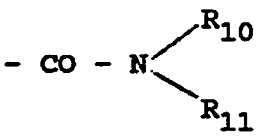 ou 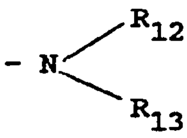

dans lesquels :

ou bien $R_{10}$ et $R_{11}$ ou $R_{12}$ et $R_{13}$, identiques ou différents, représentent :

– un atome d'hydrogène,

– un radical alkyle ou alkényle renfermant au plus 6 atomes de carbone et éventuellement substitué par un ou plusieurs substituants choisis parmi les atomes d'halogène, le radical hydroxyle et les radicaux alkoxy renfermant au plus 6 atomes de carbone,

– un radical aryle ou arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone,les radicaux carboxy libre, salifié ou estérifié, tétrazolyle, tétrazolylméthyle, tétrazolylcarbamoyle,

ou bien $R_{10}$ et $R_{11}$ ou $R_{12}$ et $R_{13}$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié, tétrazolyle, tétrazolylméthyle, tétrazolylcarbamoyle,

ou bien $R_{12}$ et $R_{13}$, identiques ou différents, représentent un radical acyle dérivé d'acide carboxylique renfermant au plus 6 atomes de carbone, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

**3)** Produits de formule (I) telle que définie aux revendications 1 et 2 et répondant à la formule (Ia) :

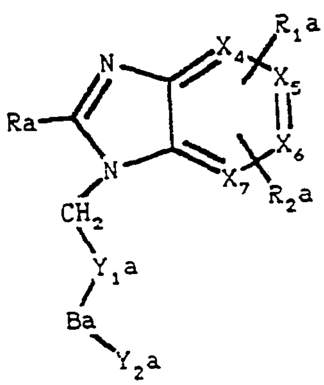

(Ia)

dans laquelle :

– $X_4$, $X_5$, $X_6$ et $X_7$ sont tels que :

soit ils représentent tous un radical méthine =CH-,

soit l'un ou deux quelconques d'entre eux représentent un atome d'azote et les autres représentent un radical méthine =CH-,

– $R_a$ représente un radical n-butyle ou butényle,

– $R_{1a}$ et $R_{2a}$, identiques ou différents, sont choisis dans le groupe formé par :

. l'atome d'hydrogène,

. les atomes d'halogène,

. le radical hydroxyle, le radical mercapto,

. les radicaux alkoxy linéaires ou ramifiés renfermant au plus 6 atomes de carbone,

. les radicaux alkyle, alkényle, alkynyle et alkylthio linéaires ou ramifiés renfermant au plus 6 atomes de carbone et les radicaux aryle, arylalkyle ou arylalkényle dans lesquels les radicaux alkyle et alkényle, linéaires ou ramifiés, renfermant au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkoxy, alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkyle, alkoxy ou alkylthio renfermant au plus 6 atomes de carbone, mercapto, acyl, acyloxy, tétrazolyle,

. le radical carboxy libre,

. les radicaux carboxy estérifié par un radical alkyle linéaire ou ramifié renfermant au plus 4 atomes de carbone,

– $Y_{1a}$ représente un radical phényle,

– $B_a$ représente une simple liaison ou un radical -CO-NH-,

– $Y_{2a}$ est tel que :

soit, si $B_a$ représente une simple liaison ou un radical -CO-NH-, $Y_{2a}$ représente un radical phényle éventuellement substitué par un radical $-(CH_2)_p-SO_2-X_a-R_{14a}$ dans lequel p représente les valeurs 0 et 1, $X_a$ représente les radicaux -NH-, -NH-CO-NH-, -NH-CO- ou une simple liaison et $R_{14a}$ représente un radical méthyle, éthyle, vinyle, allyle, pyridylméthyle, pyridyléthyle, pyridyle, phényle ou benzyle, par un radical carboxy libre, salifié ou estérifié, un radical tétrazolyle, tétrazolylalkyle ou tétrazolylcarbamoyle, dans lesquels le radical tétrazolyle est éventuellement substitué par un radical alkyle, alkényle, arylalkyle ou alcoxyalkyle,

soit, si $B_a$ représente une simple liaison, $Y_{2a}$ représente un radical cyano, carboxy libre, salifié ou estérifié, ou tétrazolyle, étant entendu que si $B_a$ représente une simple liaison alors l'un au moins de $X_4$, $X_5$, $X_6$ et $X_7$ ne représente pas un radical méthine, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

**4)** Produits de formule (I) telle que définie aux revendications 1 et 2 dans laquelle :

A représente un radical phényle, naphtyle, pyridyle, pyrimidinyle ou thiényle,

R représente un radical n-butyle ou butèn-1-yle,

$R_1$, $R_2$, $R_3$ et $R_4$ sont tels que deux d'entre eux représentent un atome d'hydrogène et les deux autres, identiques ou différents, représentent un atome d'hydrogène, un radical hydroxyle, un radical alkyle renfermant au plus 4 atomes de carbone, un radical carboxy libre ou estérifié par un radical alkyle linéaire ou ramifié renfermant au plus 4 atomes de carbone,

$R_5$ représente un radical méthylène,

et Y représente le radical $-Y_1-B-Y_2$ dans lequel $Y_1$ représente un radical phényle, B représente une liaison simple carbone-carbone ou le radical -CO-NH- et $Y_2$ représente un radical cyano, carboxy libre ou estérifié par un radical alkyle linéaire ou ramifié renfermant au plus 4 atomes de carbone, un radical indolyle ou un radical phényle éventuellement substitué par un radical carboxy libre, salifié ou estérifié, un radical tétrazolyle, tétrazolylalkyle, tétrazolylcarbamoyle, dans lesquels le radical tétrazolyle est éventuellement substitué par un radical alkyle, alkényle ou alcoxyalkyle, ou par un radical $-(CH_2)_p-SO_2-X_a-R_{14a}$ dans lequel p représente les valeurs 0 et 1, $X_a$ représente les radicaux -NH-, NHCO-NH-, -NH-CO- ou une simple liaison et $R_{14a}$ représente un radical méthyle, éthyle, vinyle, allyle, pyridylméthyle, pyridyléthyle, pyridyle, phényle ou benzyle, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

**5)** Produits de formule (I) telle que définie aux revendications 1 et 2 et répondant à la formule ($I_b$) :

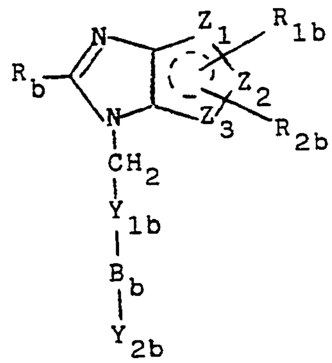

$(I_b)$

dans laquelle $R_b$ représente un radical n-butyle ou buténylе.

– $Z_1$, $Z_2$, $Z_3$ sont tels que :

l'un représente un atome de soufre

et les deux autres, identiques ou différents, représentent un radical méthine =CH-,

– $R_{1b}$ et $R_{2b}$, identiques ou différents, représentent :

. l'atome d'hydrogène,

. les atomes d'halogène,

. le radical hydroxyle, le radical mercapto,

. les radicaux alkoxy linéaires ou ramifiés renfermant au plus 6 atomes de carbone,

. les radicaux alkyle, alkényle, alkynyle et alkylthio, linéaires ou ramifiés renfermant au plus 6 atomes de carbone et les radicaux aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkoxy ou alkylthio renfermant au plus 4 atomes de carbone, mercapto, acyl, acyloxy,

. le radical carboxy libre,

. les radicaux carboxy estérifié par un radical alkyle linéaire ou ramifié renfermant au plus 4 atomes de carbone,

– $Y_{1b}$ représente un radical phényle,

– $B_b$ représente une simple liaison ou un radical -CO-NH-,

– $Y_{2b}$ est tel que :

soit, si $B_b$ représente une simple liaison ou un radical -CO-NH-, $Y_{2b}$ représente un radical phényle éventuellement substitué par un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, éthyle, n-propyle, vinyle, allyle, pyridylméthyle, pyridyléthyle, pyridyle, phényle, benzyle, pyrimidyle, tétrazolyle, thiazolyle, diazolyle, pipéridinyle ou tétrahydrofuranyle, ces radicaux étant éventuellement substitués par un radical méthyle, éthyle ou nitro,

soit, si $B_b$ représente une simple liaison, $Y_{2b}$ représente un radical cyano, formyle ou carboxy libre, salifié ou estérifié, lesdits produits de formule $(I_b)$ étant sous toutes les formes isomères possibles racémiques, énantiomères et diastéréoisomères, ainsi que les sels d'addition avec les acides minéraux et organiques ou avec les bases minérales et organiques desdits produits de formule $(I_b)$.

**6)** Produits de formule (I) telle que définie aux revendications 1 et 2, répondant à la formule $(I_d)$ :

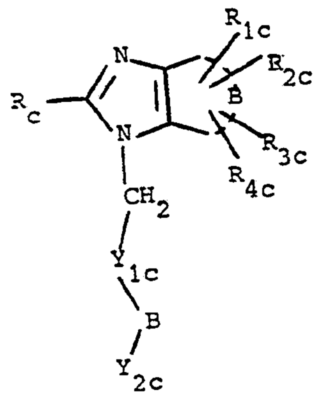

$(I_d)$

dans laquelle

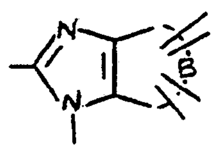

représente

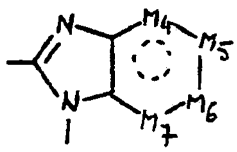 ou 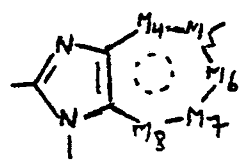

avec $M_4$, $M_5$, $M_6$, $M_7$ et $M_8$ identiques ou différents, représentent

- un radical méthine =CH- ou un atome d'azote éventuellement substitué par un radical choisi parmi les valeurs de $R_{1c}$, $R_{2c}$, $R_{3c}$ et $R_{4c}$,
- un radical méthylène $-CH_2-$ éventuellement substitué par un ou deux radicaux identiques ou différents choisis parmi les valeurs de $R_{1c}$, $R_{2c}$, $R_{3c}$ et $R_{4c}$,
- un radical -C=O,
- un atome de soufre tels que l'un au moins de $M_4$, $M_5$, $M_6$, $M_7$ et $M_8$ représente un atome d'azote ou de soufre,

$R_c$ représente un radical alkyle ou alkényle renfermant au plus 4 atomes de carbone,

$R_{1c}$, $R_{2c}$, $R_{3c}$, $R_{4c}$ identiques ou différents, sont choisis parmi :

- l'atome d'hydrogène,
- le radical carboxy libre, salifié ou estérifié par un radical alkyle renfermant au plus 4 atomes de carbone ou par un radical aryle, ces radicaux alkyle et aryle étant eux-mêmes éventuellement substitués,
- les radicaux alkyle, alkényle et alkoxy renfermant au plus 4 atomes de carbone éventuellement substitués,
- les radicaux aryle,
- les radicaux amino et carbamoyle éventuellement subtitués par un ou deux radicaux identiques ou différents choisis parmi les radicaux alkyle et aryle eux-mêmes éventuellement substitués,

tous les radicaux alkyle, alkényle et aryle ci-dessus étant éventuellement substitués par un ou plusieurs radicaux choisis parmi les atomes d'halogène, les radicaux hydroxyle, carboxy libre, salifié ou estérifié par un radical alkyle renfermant au plus 4 atomes de carbone, amino, alkylamino, phényle, alkyle, alkényle et alkoxy renfermant au plus 4 atomes de carbone,

- $Y_{1c}$ représente un radical phényle,

- $B_c$ représente une simple liaison ou un radical -CO-NH-,
- $Y_{2c}$ est tel que :

soit, si $B_c$ représente une simple liaison ou un radical -CO-NH-, $Y_{2c}$ représente un radical phényle éventuellemment substitué par un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, éthyle, n-propyle, vinyle, allyle, pyridylméthyle, pyridyléthyle, pyridyle, phényle, benzyle, pyrimidyle, tétrazolyle, thiazolyle, diazolyle, pipéridinyle ou tétrahydrofuranyle, ces radicaux étant éventuellement substitués par un radical méthyle, éthyle ou nitro, soit, si $B_c$ représente une simple liaison, $Y_{2c}$ représente un radical cyano, formyle ou carboxy libre, salifié ou estérifié, lesdits produits de formule ($I_c$) étant sous toutes les formes isomères possibles racémiques, énantiomères et diastéréoisomères, ainsi que les sels d'addition avec les acides minéraux et organiques ou avec les bases minérales et organiques desdits produits de formule ($I_c$).

**7)** Produits de formule ($I_b$) telle que définie à la revendication 5 répondant à la formule ($I_b'$) :

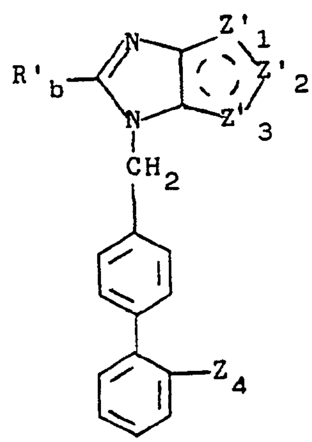

($I_b'$)

dans laquelle :

$R_b'$ représente un radical n-butyle,

$Z_{1'}$, $Z_2'$ et $Z_3'$ sont tels que :

l'un représente un atome de soufre,

et les deux autres, identiques ou différents, représentent un radical méthine =CH- éventuellement substitué par un radical hydroxyle ou un radical carboxy libre, salifié ou estérifié par un radical alkyle linéaire ou ramifié renfermant au plus 4 atomes de carbone,

et $Z_4$ représente un radical carboxy libre, salifié ou estérifié par un radical alkyle linéaire ou ramifié renfermant au plus 4 atomes de carbone ou un radical tétrazolyle,

lesdits produits de formule ($I_{b'}$) étant sous toutes les formes isomères possibles racémiques, énantiomères et diastéréoisomères, ainsi que les sels d'addition avec les acides minéraux et organiques ou avec les bases minérales et organiques desdits produits de formule ($I_b'$).

**8)** L'acide 2-butyl 1-[(4-carboxyphényl) méthyl] 1H-benzimidazole -6-carboxylique
l'acide 4-[(2-butyl-1H-benzimidazol-1-yl) méthyl] benzoïque
le 4-[(2-butyl-1H-benzimidazol-1-yl) méthyl] N-(1H-indol-4-yl) benzamide
l'acide 4-[(2-butyl-1H-naphth(2,3-d)imidazol-1-yl) méthyl] benzoïque
l'acide 4-[(2-butyl-5,6-diméthyl-1H-benzimidazol-1-yl) méthyl] benzoïque
l'acide 4-[(2-butyl-3H-imidazo(4,5-c)pyridin-3-yl) méthyl] benzoïque
l'acide 4'-((2-butyl-3H-imidazo(4,5b) pyridin-3-yl) méthyl) (1,1'-biphényl) 2-carboxylique
le 2-butyl-1-((2'-carboxy-(1,1'-biphényl)-4yl) méthyl)-6-hydroxy-1H-thiéno(2,3-d)imidazole-5-carboxylate de 1,1-diméthyléthyle et leurs sels.

**9)** Procédé de préparation de produits de formule (I) telle que définie à la revendication 1 caractérisé en ce que :

soit l'on fait réagir un composé de formule (II) :

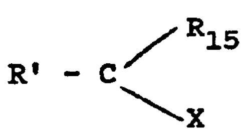

(II)

dans laquelle X représente un atome d'oxygène ou un radical =NH, $R_{15}$ représente un radical hydroxyle, alkoxy ou un atome d'halogène et $NH_2$, et R' a la signification indiquée à la revendication 1 pour R dans laquelle les éventuelles fonctions réactives sont éventuellement protégées par des groupements protecteurs, avec un composé de formule (III) :

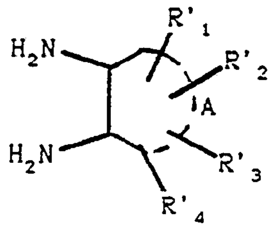

(III)

dans laquelle $R_1'$, $R_2'$, $R_3'$ et $R_4'$ ont les significations indiquées à la revendication 1 respectivement pour $R_1$, $R_2$, $R_3$ et $R_4$ dans lesquelles les éventuelles fonctions réactives sont éventuellement protégées par des groupements protecteurs, pour obtenir le composé de formule IV, après isolement éventuel d'un intermédiaire de formule (IV') :

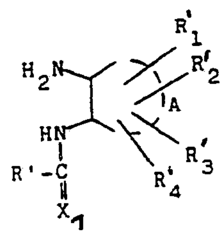

(IV')

dans laquelle A, $X_1$, R', $R'_1$, $R'_2$, $R'_3$, et $R'_4$ ont les significations indiquées ci-dessus, produit de formule (IV) :

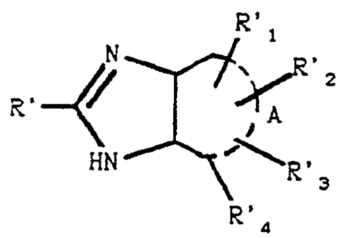

(IV)

dans laquelle R', $R_1'$, $R_2'$, $R_3'$ et $R_4'$ ont les significations indiquées ci-dessus, que l'on fait réagir avec un composé de formule (V) :

$$\text{Hal} - R_5 - Y' \qquad (V)$$

dans laquelle Hal représente un atome d'halogène, $R_5$ a la signification indiquée à la revendication 1 et Y' a la signification indiquée à la revendication 1 pour Y dans laquelle les éventuelles fonctions réactives sont éventuellement protégées par des groupements protecteurs, pour obtenir des produits de formule (IX) :

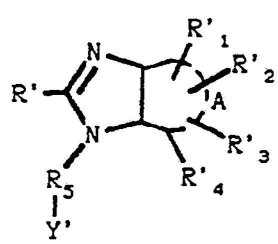

(IX)

dans laquelle R', $R_1'$, $R_2'$, $R_3'$, $R_4'$, $R_5$ et Y' ont les significations indiquées ci-dessus,
<u>soit</u> l'on fait réagir un composé de formule (VI) :

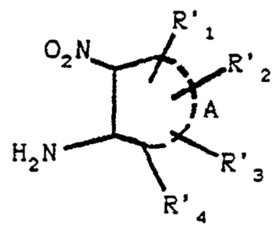

(VI)

dans laquelle $R_1'$, $R_2'$, $R_3'$ et $R_4'$ ont les significations indiquées à la revendication 1 respectivement pour $R_1$, $R_2$, $R_3$ et $R_4$ dans lesquelles les éventuelles fonctions réactives sont éventuellement protégées par des groupements protecteurs,
ou bien avec le composé de formule (II') :

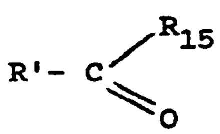

(II')

dans laquelle R' et $R_{15}$ ont les significations indiquées ci-dessus, pour obtenir le produit de formule (X) :

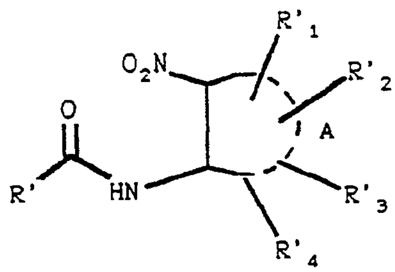

(X)

dans laquelle R', $R_1'$, $R_2'$, $R_3'$ et $R_4'$ ont les significations précédentes, produit de formule (X)
soit que l'on réduit en produit de formule (X') :

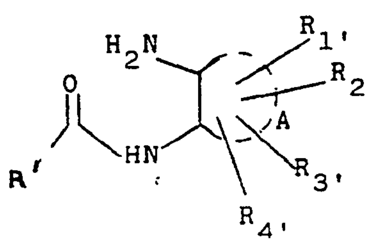

(X')

que l'on cyclise en produit de formule (IV) tel que défini ci-dessus que l'on traite ainsi qu'il est indiqué ci-dessus pour obtenir un produit de formule (IX) soit l'on fait réagir le produit de formule (X) avec le composé de formule (V) telle que définie ci-dessus, pour obtenir un produit de formule (XI) :

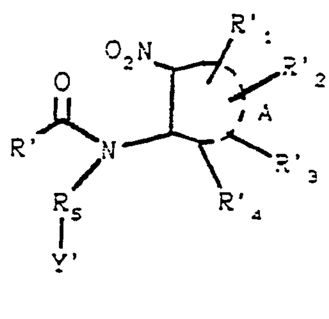

(XI)

dans laquelle R′, $R_1$′, $R_2$′, $R_3$′, $R_4$′, $R_5$ et Y′ ont les significations précédentes, que l'on soumet à une réaction de réduction sélective de la fonction nitro, pour obtenir le produit de formule (XII) :

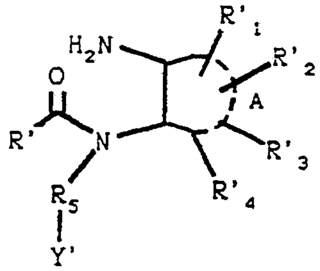

(XII)

dans laquelle R′, $R_1$′, $R_2$′, $R_3$′, $R_4$′, $R_5$ et Y′ ont les significations précédentes, que l'on soumet à une réaction de cyclisation pour obtenir des produits de formule (IX) telle que définie ci-dessus,
ou bien l'on fait réagir le composé de formule (VI) avec le composé de formule (V) telle que définie ci-dessus, pour obtenir des produits de formule (VII) :

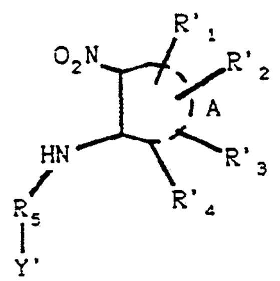

(VII)

dans laquelle $R_1$′, $R_2$′, $R_3$′, $R_4$′, $R_5$ et Y′ ont les significations indiquées ci-dessus, que :
ou bien l'on fait réagir avec le composé de formule (II′) tel que défini ci-dessus, pour obtenir un produit de formule (XI) tel que défini ci-dessus que l'on traite ensuite comme indiqué ci-dessus,
ou bien l'on soumet à une réaction de réduction du radical nitro en radical amino pour obtenir des produits de formule (VIII) :

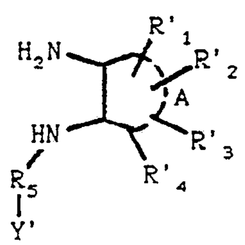

(VIII)

dans laquelle $R_1$′, $R_2$′, $R_3$′, $R_4$′, $R_5$ et Y′ ont les significations indiquées ci-dessus, que l'on fait réagir avec le produit de formule (II) telle que définie ci-dessus, pour obtenir un produit de formule (IX) telle que définie ci-dessus, produit de formule (IX) que l'on traite, si désiré et si nécessaire, à l'une ou plusieurs des réactions suivantes, dans un ordre quelconque :

- une réaction d'élimination des groupements protecteurs que peuvent porter les fonctions réactives protégées,
- une réaction de salification par un acide minéral ou organique ou par une base pour obtenir le sel correspondant,
- une réaction d'estérification ou salification de fonction acide,
- une réaction de saponification de fonction ester en fonction acide,
- une réaction de transformation de fonction alkoxy en fonction hydroxyle,
- une réaction de transformation de la fonction cyano en fonction acide,
- une réaction de réduction de la fonction carboxy en fonction alcool,
- une réaction de substitution de fonction hydroxyle ou mercapto par un atome d'halogène,

– une réaction de substitution sur un atome d'halogène,

– une réaction de dédoublement des formes racémiques en produits dédoublés,

lesdits produits de formule (I) ainsi obtenus étant sous toutes les formes isomères possibles racémiques, énantiomères et diastéréoisomères.

**10)** Procédé de préparation de produits de formule ($I_c$) :

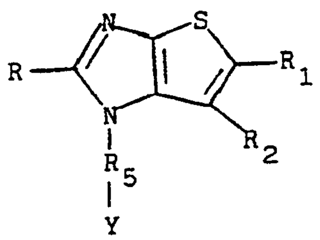

($I_C$)

dans laquelle R, $R_1$, $R_2$, $R_5$ et Y ont les significations indiquées à la revendication 1 caractérisé en ce que : soit, l'on soumet un composé de formule (XIII) :

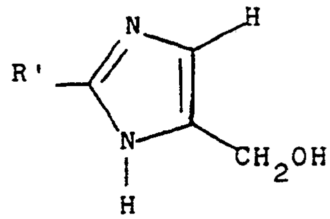

(XIII)

dans laquelle R' a la signification indiquée à la revendication 1 pour R dans laquelle les fonctions éventuellement réactions sont éventuellement protégées à une réaction d'halogénation pour obtenir un composé de formule (XIV) :

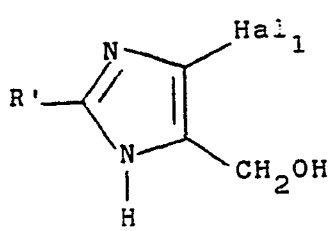

(XIV)

dans laquelle R' a la signification indiquée ci-dessus, et $Hal_1$ représente un atome d'halogène, de préférence le brome, que l'on soumet à une réaction d'oxydation pour obtenir le composé de formule (XV) :

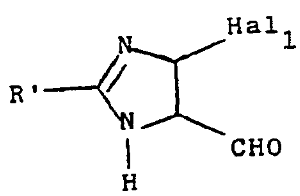

(XV)

dans laquelle R' et $Hal_1$ ont les significations indiquées ci-dessus, que l'on fait réagir avec un composé de formule (V) :

$$Hal\text{-}R_5\text{-}Y' \qquad (V)$$

dans laquelle Hal, $R_5$ et Y' ont les significations indiquées à la revendication 9 pour obtenir un composé de formule (XVI) :

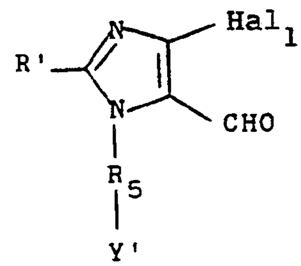

(XVI)

dans laquelle $Hal_1$, R', $R_5$ et Y' ont les significations indiquées ci-dessus que l'on fait réagir avec un composé de formule (XVII) :

$HS-CH_2-R_1'$ (XVII)

dans laquelle $R_1'$ a la signification indiquée à la revendication 1 pour $R_1$ dans laquelle les fonctions éventuellement réactives sont éventuellement protégées pour obtenir après cyclisation un composé de formule ($I_c'$) :

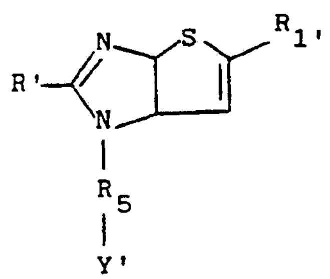

($I_c'$)

correspondant aux produits de formule ($I_c$) dans laquelle $R_2$ représente un atome d'hydrogène, soit, l'on fait réagir un composé de formule (XVIII) :

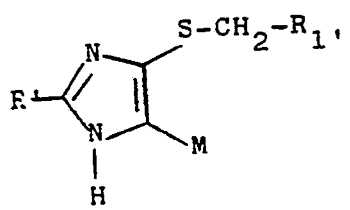

(XVIII)

dans laquelle R' et $R_1'$ ont les significations indiquées à la revendication 1 dans lesquelles les fonctions éventuellement réactives sont éventuellement protégées, et M représente le radical cyano ou le radical

$$\underset{Z}{-C-R_2'}$$

dans lequel $R_2'$ a la signification indiquée à la revendication 1 pour $R_2$ dans laquelle les éventuelles fonctions réactives sont éventuellement protégées et Z représente l'atome d'oxygène ou l'atome de soufre, avec le composé de formule (V) tel que défini ci-dessus pour obtenir le produit de formule (XIX) :

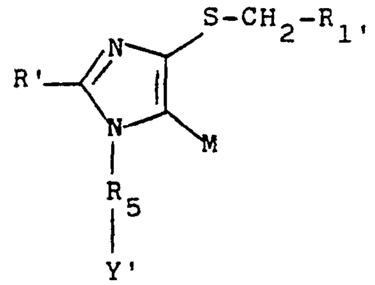

(XIX)

dans laquelle R′, $R_1$′, $R_5$′ et Y′ ont les significations indiquées ci-dessus, que l'on soumet à une réaction de cyclisation pour obtenir le composé de formule ($I_c$″) :

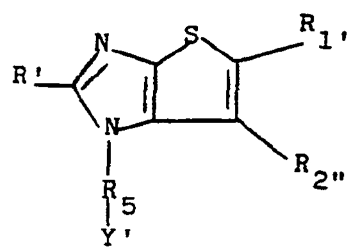

($I_c$")

dans laquelle R′, $R'_1$, $R'_2$, $R_5$ et Y′ ont les significations indiquées ci-dessus et $R_2$″ a la signification indiquée ci-dessus pour $R_2$′ produits de formule ($I_c$′) et ($I_c$″) que l'on soumet, si désiré et si nécessaire, à l'une ou plusieurs des réactions suivantes, dans un ordre quelconque :

– une réaction d'élimination des groupements protecteurs que peuvent porter les fonctions réactives protégées,
– une réaction de salification par un acide minéral ou organique ou par une base pour obtenir le sel correspondant,
– une réaction d'estérification ou salification de fonction acide,
– une réaction de saponification de fonction ester en fonction acide,
– une réaction de transformation de fonction alkoxy en fonction hydroxyle,
– une réaction de transformation de la fonction cyano en fonction acide,
– une réaction de réduction de la fonction carboxy en fonction alcool,
– une réaction de substitution de fonction hydroxyle ou mercapto par un atome d'halogène,
– une réaction de substitution sur un atome d'halogène,
– une réaction de dédoublement des formes racémiques en produits dédoublés,

lesdits produits de formule ($I_c$) ainsi obtenus étant sous toutes les formes isomères possibles racémiques, énantiomères et diastéréoisomères.

**11)** A titre de médicaments, les produits tels que définis par la formule (I) de la revendication 1, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

**12)** A titre de médicaments, les produits de formule (I) tels que définis aux revendications 2 à 7, ainsi que les sels d'addition avec les acides minéraux et organiques ou avec les bases minérales et organiques pharmaceutiquement acceptables desdits produits de formule (I).

**13)** Les compositions pharmaceutiques contenant à titre de principe actif, l'un au moins des médicaments tels que définis à l'une quelconque des revendications 11 et 12.

**14)** A titre de produits industriels nouveaux, les composés de formule (IV), (VII), (VIII), (XVI), (XVIII) et (XIX).

**Revendications pour l'Etat contractant suivant : ES**

**1.-** Procédé de préparation des produits de formule (I) :

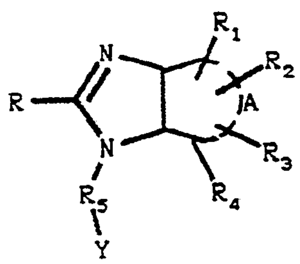

(I)

dans laquelle :

A représente le reste d'un radical monocyclique comprenant 3, 4 ou 5 chaînons ou d'un radical constitué de cycles condensés comprenant 6 à 12 chaînons, ces radicaux étant saturés ou insaturés et renfermant éventuellement un ou plusieurs hétéroatomes choisis parmi les atomes d'oxygène, et les atomes d'azote et de soufre éventuellement oxydés,

R représente un radical alkyle, alkényle ou alkynyle linéaire ou ramifié renfermant au plus 6 atomes de carbone et éventuellement substitués,

$R_1$, $R_2$, $R_3$ et $R_4$, identiques ou différents, représentent :

a) un atome d'hydrogène, un atome d'halogène, un radical hydroxyle, cyano, nitro, sulfo, formyle, benzoyle, acyle ou acyloxy ayant au plus 12 atomes de carbone, carboxy libre, salifié ou estérifié, mercapto,

b) un radical alkyle, alkényle, alkynyle, alkoxy ou alkylthio, ces radicaux étant linéaires ou ramifiés, renfermant au plus 6 atomes de carbone et étant éventuellement substitués,

c) un radical aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués,

d) un radical

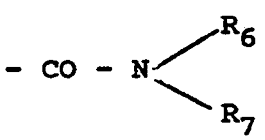 ou 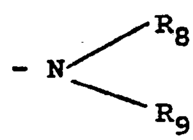

dans lesquels :

ou bien $R_6$ et $R_7$ ou $R_8$ et $R_9$, identiques ou différents, représentent :

– un atome d'hydrogène,

– un radical alkyle ou alkényle renfermant au plus 6 atomes de carbone et éventuellement substitué par un atome d'halogène, un radical hydroxyle ou un radical alkoxy renfermant au plus 6 atomes de carbone,

– un radical aryle ou arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié,

– un radical $-(CH_2)_m-SO_2-X-R_{14}$ dans lequel m représente un entier de 0 à 4 et

soit $X-R_{14}$ représente $NH_2$

soit X représente les radicaux -NH-, -NH-CO-HH- ou -NH-CO- ou une simple liaison

et $R_{14}$ représente un radical alkyle, alkényle et aryle, ces radicaux étant éventuellement substitués,

ou bien $R_6$ et $R_7$ ou $R_8$ et $R_9$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène les radicaux hydroxyle, nitro, les radicaux alkyle, alkényle, alkoxy et acyle, ces radicaux renfermant

au plus 6 atomes de carbone, les radicaux carboxy libre, salifié ou estérifié,
ou bien $R_8$ et $R_9$, identiques ou différents, représentent un radical acyle dérivé d'acide carboxylique renfermant au plus 6 atomes de carbone, ou un radical alkyle ou arylsulfonyle dans lequel le radical alkyl renferme au plus 6 atomes de carbone et le radical aryle renferme au plus 8 atomes de carbone, ces radicaux étant éventuellement substitués par un ou plusieurs substituants choisis parmi les atomes d'halogène ou les radicaux alkyle renfermant au plus 6 atomes de carbone,

e) un radical $-(CH_2)_m-SO_2-X-R_{14}$ dans lequel m, X et $R_{14}$ ont la signification précédente,

$R_5$ représente un radical divalent alkylène, linéaire ou ramifié, renfermant au plus 4 atomes de carbone,
Y représente le radical $-Y_1-B-Y_2$ dans lequel :
$Y_1$ représente un radical aryle monocyclique comprenant 5 ou 6 chaînons ou constitué de cycles condensés comprenant 8 à 10 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les radicaux que peuvent représenter $R_1$, $R_2$, $R_3$ et $R_4$,
B représente :
soit une simple liaison entre $Y_1$ et $Y_2$,
soit l'un des radicaux divalents suivants : -CO-, -NH-CO-, -CO-NH- ou $-O-(CH_2)_n-$ avec n représentant les valeurs 0 à 3,
$Y_2$ représente :
soit, quelle que soit la valeur de B autre qu'une simple liaison et $Y_2$ étant identique ou différent de $Y_1$, les valeurs définies pour $Y_1$,
soit, si B représente une simple liaison, un atome d'hydrogène, un radical cyano, carboxy libre, salifié ou estérifié, étant entendu que :
lorsque A représente un radical phényle éventuellement substitué par un ou deux radicaux choisis parmi les atomes d'halogène, les radicaux alkyle éventuellement substitué, alkoxy, acyle, carboxy libre ou phényle substitué par 5 atomes de fluor,
$R_5$ représente $-CH_2-$,
et Y représente le radical $-Y_1-B-Y_2$ dans lequel $Y_1$ représente un radical phényle non substitué et $Y_2$ représente un radical phényle substitué en ortho par un radical carboxy libre éventuellemment salifié ou un radical tétrazolyle ou trifluorométhyl-sulfonamide et comportant éventuellement un second substituant choisi parmi les atomes d'halogène, les radicaux alkyle renfermant au plus 4 atomes de carbone, les radicaux nitro et méthoxy, alors B représente -CO-NH- ou $-O-(CH_2)_n-$ avec n représentant les valeurs 2 ou 3,
lesdits produits de formule (I) étant sous toutes les formes isomères possibles racémiques, énantiomères et diastéréoisomères,
ainsi que les sels d'addition avec les acides minéraux et organiques ou avec les bases minérales et organiques desdits produits de formule (I), caractérisé en ce que :
soit l'on fait réagir un composé de formule (II) :

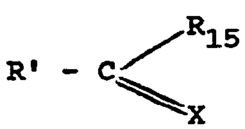

(II)

dans laquelle X représente un atome d'oxygène ou un radical =NH, $R_{15}$ représente un radical hydroxyle, alkoxy ou un atome d'halogène et $NH_2$, et R' a la signification indiquée ci-dessus pour R dans laquelle les éventuelles fonctions réactives sont éventuellement protégées par des groupements protecteurs, avec un composé de formule (III) :

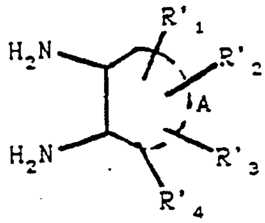

(III)

dans laquelle $R_1'$, $R_2'$, $R_3'$ et $R_4'$ ont les significations indiquées ci-dessus respectivement pour $R_1$, $R_2$, $R_3$ et

$R_4$ dans lesquelles les éventuelles fonctions réactives sont éventuellement protégées par des groupements protecteurs, pour obtenir le composé de formule IV, après isolement éventuel d'un intermédiaire de formule (IV') :

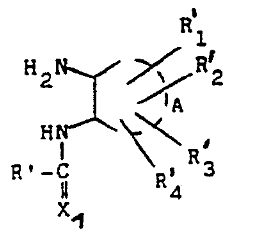

(IV')

dans laquelle A, $X_1$, R', $R'_1$, $R'_2$, $R'_3$, et $R'_4$ ont les significations indiquées ci-dessus, produit de formule (IV) :

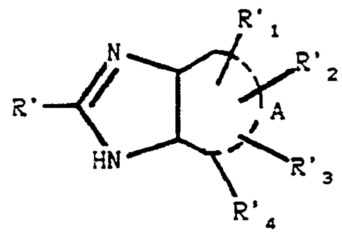

(IV)

dans laquelle R', $R_1'$, $R_2'$, $R_3'$ et $R_4'$ ont les significations indiquées ci-dessus, que l'on fait réagir avec un composé de formule (V) :

Hal - $R_5$ - Y' (V)

dans laquelle Hal représente un atome d'halogène, $R_5$ a la signification indiquée ci-dessus et Y' a la signification indiquée ci-dessus pour Y dans laquelle les les éventuelles fonctions réactives sont éventuellement protégées par des groupements protecteurs, pour obtenir des produits de formule (IX) :

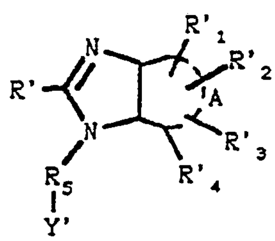

(IX)

dans laquelle R', $R_1'$, $R_2'$, $R_3'$, $R_4'$, $R_5$ et Y' ont les significations indiquées ci-dessus,
soit l'on fait réagir un composé de formule (VI) :

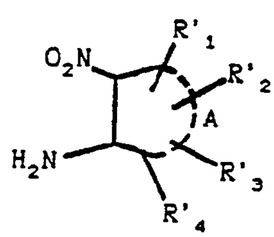

(VI)

dans laquelle $R_1'$, $R_2'$, $R_3'$ et $R_4'$ ont les significations indiquées ci-dessus respectivement pour $R_1$, $R_2$, $R_3$ et $R_4$ dans lesquelles les éventuelles fonctions réactives sont éventuellement protégées par des groupements protecteurs,
ou bien avec le composé de formule (II') :

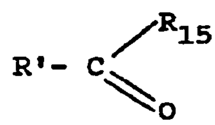

(II')

dans laquelle R′ et $R_{15}$ ont les significations indiquées ci-dessus, pour obtenir le produit de formule (X) :

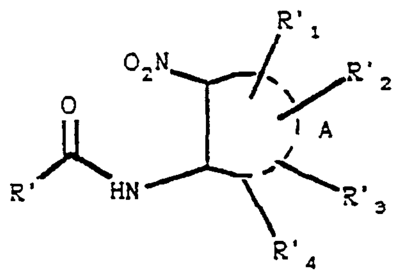

(X)

dans laquelle R′, $R_1'$, $R_2'$, $R_3'$ et $R_4'$ ont les significations précédentes, produit de formule (X) soit que l'on réduit en produit de formule (X′) :

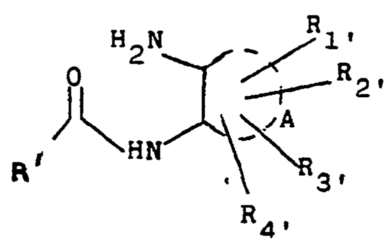

(X')

que l'on cyclise en produit de formule (IV) tel que défini ci-dessus que l'on traite ainsi qu'il est indiqué ci-dessus pour obtenir un produit de formule (IX) soit l'on fait réagir le produit de formule (X) avec le composé de formule (V) telle que définie ci-dessus, pour obtenir un produit de formule (XI) :

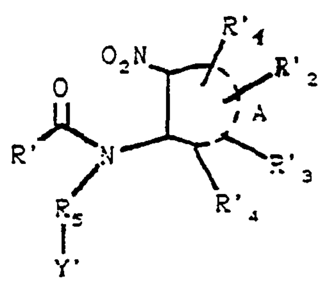

(XI)

dans laquelle R′, $R_1'$, $R_2'$, $R_3'$, $R_4'$, $R_5$ et Y′ ont les significations précédentes, que l'on soumet à une réaction de réduction sélective de la fonction nitro, pour obtenir le produit de formule (XII) :

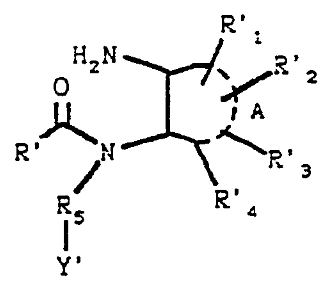

(XII)

dans laquelle R′, $R_1'$, $R_2'$, $R_3'$, $R_4'$, $R_5$ et Y′ ont les significations précédentes, que l'on soumet à une réaction

de cyclisation pour obtenir des produits de formule (IX) telle que définie ci-dessus,
ou bien l'on fait réagir le composé de formule (VI) avec le composé de formule (V) telle que définie ci-dessus, pour obtenir des produits de formule (VII) :

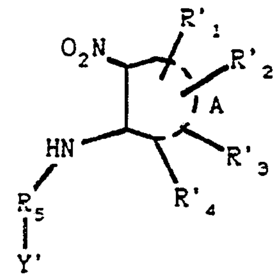

(VII)

dans laquelle $R_1'$, $R_2'$, $R_3'$, $R_4'$, $R_5$ et Y' ont les significations indiquées ci-dessus, que :
ou bien l'on fait réagir avec le composé de formule (II') tel que défini ci-dessus, pour obtenir un produit de formule (XI) tel que défini ci-dessus que l'on traite ensuite comme indiqué ci-dessus,
ou bien l'on soumet à une réaction de réduction du radical nitro en radical amino pour obtenir des produits de formule (VIII) :

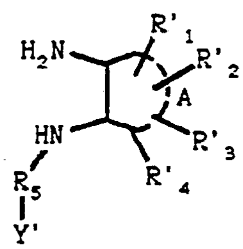

(VIII)

dans laquelle $R_1'$, $R_2'$, $R_3'$, $R_4'$, $R_5$ et Y' ont les significations indiquées ci-dessus, que l'on fait réagir avec le produit de formule (II) telle que définie ci-dessus, pour obtenir un produit de formule (IX) telle que définie ci-dessus, produit de formule (IX) que l'on traite, si désiré et si nécessaire, à l'une ou plusieurs des réactions suivantes, dans un ordre quelconque :

- une réaction d'élimination des groupements protecteurs que peuvent porter les fonctions réactives protégées,
- une réaction de salification par un acide minéral ou organique ou par une base pour obtenir le sel correspondant,
- une réaction d'estérification ou salification de fonction acide,
- une réaction de saponification de fonction ester en fonction acide,
- une réaction de transformation de fonction alkoxy en fonction hydroxyle,
- une réaction de transformation de la fonction cyano en fonction acide,
- une réaction de réduction de la fonction carboxy en fonction alcool,
- une réaction de substitution de fonction hydroxyle ou mercapto par un atome d'halogène,
- une réaction de substitution sur un atome d'halogène,
- une réaction de dédoublement des formes racémiques en produits dédoublés,

lesdits produits de formule (I) ainsi obtenus étant sous toutes les formes isomères possibles racémiques, énantiomères et diastéréoisomères.

**2.**- Procédé selon la revendication 1, pour la préparation des produits de formule (I') correspondant aux produits de formule (I) telle que définie à la revendication 1 dans laquelle $R_1$, $R_2$, $R_3$ et $R_4$, identiques ou différents, ont la signification indiquée à la revendication 1 à l'exclusion de la valeur radical $-(CH_2)_m-SO_2-X-R_{14}$ tel que défini à la revendication 1 ou radical

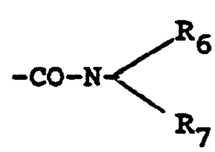

ou

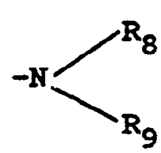

dans lesquels $R_8$ et $R_7$ ou $R_8$ et $R_9$ identiques ou différents représentent un radical aryle ou arylalkyle substitué par un ou plusieurs radicaux $-(CH_2)_m-SO_2-X-R_{14}$ tel que défini à la revendication 1, caractérisé en ce que l'on utilise au départ un composé de formule (III') :

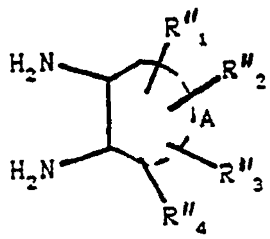

(III')

dans laquelle $R''_1$, $R''_2$, $R''_3$ et $R''_4$ ont les significations indiquées ci-dessus respectivement pour $R_1$, $R_2$, $R_3$ et $R_4$ dans lesquelles les fonctions réactives sont éventuellement protégées par des groupements protecteurs, ou un composé de formule (VI') :

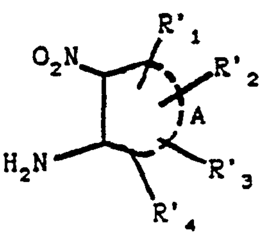

(VI')

dans laquelle $R''_1$, $R''_2$, $R''_3$ et $R''_4$ ont les significations indiquées ci-dessus.

3.- Procédé selon la revendication 1, caractérisé en ce que le ou les substituants, identiques ou différents que peuvent porter :

a) les radicaux alkyle, alkényle et alkynyle que peut représenter R,

b) les radicaux alkyle, alkényle, alkynyle, alkoxy et alkylthio que peuvent représenter $R_1$, $R_2$, $R_3$ et $R_4$,

c) les radicaux aryle, arylalkyle et arylalkényle que peuvent représenter $R_1$, $R_2$, $R_3$ et $R_4$

d) les radicaux alkyle, alkényle et aryle que peut représenter $R_{14}$,

sont choisis dans le groupe formé par :

– les atomes d'halogène, les radicaux hydroxyle, cyano, nitro, formyle, acyle ou acyloxy ayant au plus 6 atomes de carbone, benzoyle, carboxy libre, salifié ou estérifié par un radical alkyle renfermant au plus 6 atomes de carbone,

– les radicaux alkyle et alkényle renfermant au plus 6 atomes de carbone et éventuellement substitués par un ou plusieurs substituants choisis parmi les atomes d'halogène, le radical hydroxyle et les radicaux alkoxy renfermant au plus 6 atomes de carbone,

– les radicaux alkoxy linéaires et ramifiés renfermant au plus 6 atomes de carbone,

– les radicaux aryle et arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone,les radicaux carboxy libre, salifié ou estérifié,

– les radicaux

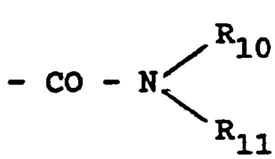

ou

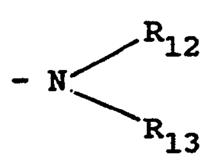

dans lesquels :

ou bien $R_{10}$ et $R_{11}$ ou $R_{12}$ et $R_{13}$, identiques ou différents, représentent :
– un atome d'hydrogène,
– un radical alkyle ou alkényle renfermant au plus 6 atomes de carbone et éventuellement substitué par un ou plusieurs substituants choisis parmi les atomes d'halogène, le radical hydroxyle et les radicaux alkoxy renfermant au plus 6 atomes de carbone,
– un radical aryle ou arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone,les radicaux carboxy libre, salifié ou estérifié, tétrazolyle, tétrazolylméthyle, tétrazolylcarbamoyle,

ou bien $R_{10}$ et $R_{11}$ ou $R_{12}$ et $R_{13}$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié, tétrazolyle, tétrazolylméthyle, tétrazolylcarbamoyle,

ou bien $R_{12}$ et $R_{13}$, identiques ou différents, représentent un radical acyle dérivé d'acide carboxylique renfermant au plus 6 atomes de carbone.

**4.-** Procédé selon la revendication 1, pour la préparation des produits de formule (I) telle que définie à la revendication 1 répondant à la formule (Ia) :

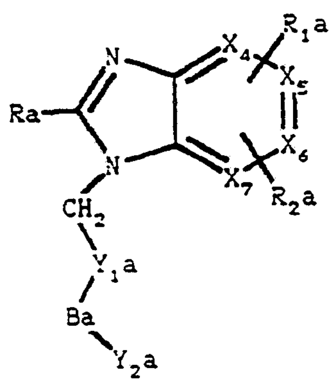

(Ia)

dans laquelle :

– $X_4$, $X_5$, $X_6$ et $X_7$ sont tels que :
soit ils représentent tous un radical méthine =CH-,
soit l'un ou deux quelconques d'entre eux représentent un atome d'azote et les autres représentent un radical méthine =CH-,

– $R_a$ représente un radical n-butyle ou buténysle,

– $R_{1a}$ et $R_{2a}$, identiques ou différents, sont choisis dans le groupe formé par :
. l'atome d'hydrogène,
. les atomes d'halogène,
. le radical hydroxyle, le radical mercapto,
. les radicaux alkoxy linéaires ou ramifiés renfermant au plus 6 atomes de carbone,
. les radicaux alkyle, alkényle, alkynyle et alkylthio linéaires ou ramifiés renfermant au plus 6 atomes de carbone et les radicaux aryle, arylalkyle ou arylalkényle dans lesquels les radicaux alkyle et alkényle, linéaires ou ramifiés, renfermant au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkoxy, alkyle, alkényle, alkynyle, alkylthio, aryle, arylalkyle et arylalkényle étant éventuellement substitués par un ou plusieurs substituants identiques ou différents choisis parmi les

atomes d'halogène, le radical hydroxyle, les radicaux alkyle, alkoxy ou alkylthio renfermant au plus 6 atomes de carbone, mercapto, acyl, acyloxy, tétrazolyle,

. le radical carboxy libre,

. les radicaux carboxy estérifié par un radical alkyle linéaire ou ramifié renfermant au plus 4 atomes de carbone,

– $Y_{1a}$ représente un radical phényle,

– $B_a$ représente une simple liaison ou un radical -CO-NH-,

– $Y_{2a}$ est tel que :

soit, si $B_a$ représente une simple liaison ou un radical -CONH-, $Y_{2a}$ représente un radical phényle éventuellement substitué par un radical $-(CH_2)_p-SO_2-X_a-R_{14a}$ dans lequel p représente les valeurs 0 et 1, $X_a$ représente les radicaux -NH-, -NH-CO-NH-, -NH-CO- ou une simple liaison et $R_{14a}$ représente un radical méthyle, éthyle, vinyle, allyle, pyridylméthyle, pyridyléthyle, pyridyle, phényle ou benzyle, par un radical carboxy libre, salifié ou estérifié, un radical tétrazolyle, tétrazolylalkyle ou tétrazolylcarbamoyle, dans lesquels le radical tétrazolyle est éventuellement substitué par un radical alkyle, alkényle, arylalkyle ou alcoxyalkyle,

soit, si $B_a$ représente une simple liaison, $Y_{2a}$ représente un radical cyano, carboxy libre, salifié ou estérifié, ou tétrazolyle, étant entendu'que si $B_a$ représente une simple liaison alors l'un au moins de $X_4$, $X_5$, $X_6$ et $X_7$ ne représente pas un radical méthine, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I), caractérisé en ce que l'on utilise au départ un composé de formule (II) ou (II') dans laquelle R' a la signification indiquée ci-dessus pour $R_a$, un composé de formule (III) ou (VI) dans laquelle le cycle

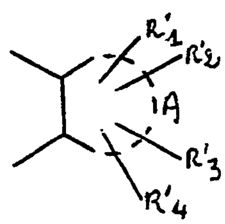

représente

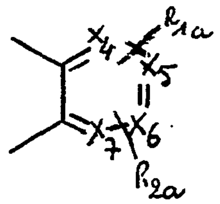

tel que

défini ci-dessus et un composé de formule (V) dans laquelle $R_5$ représente un radical $-CH_2-$ et Y' représente le groupe $Y_{1a}-B_a-Y_{2a}$ tel que défini ci dessus dans lequel les fonctions réactives sont éventuellement protégées.

**5.**- Procédé selon la revendication 2, pour la préparation des produits de formule (I') telle que définie à la revendication 2 répondant à la formule (I'a) :

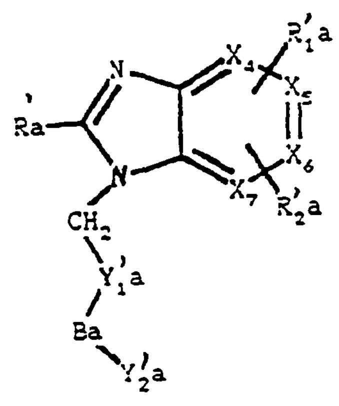

(I'a)

dans laquelle :

– $X_4$, $X_5$, $X_6$ et $X_7$ sont tels que :

soit ils représentent tous un radical méthine =CH-,

soit l'ün ou deux quelconques d'entre eux représentent un atome d'azote et les autres représentent un radical méthine =CH-,

– $R'_a$ représente un radical n-butyle ou butényle,

– $R'_{1a}$ et $R'_{2a}$, identiques ou différents, sont choisis dans le groupe formé par :

. l'atome d'hydrogène,

. le radical hydroxyle,

. les radicaux alkoxy linéaires ou ramifiés renfermant au plus 6 atomes de carbone,

. les radicaux alkyle linéaires ou ramifiés renfermant au plus 6 atomes de carbone et éventuellement substitués par un ou plusieurs substituants identiques ou différents choisis parmi les atomes d'halogène, le radical hydroxyle, les radicaux alkyle, alkoxy ou alkylthio renfermant au plus 4 atomes de carbone, mercapto,

. le radical carboxy libre,

. les radicaux carboxy estérifié par un radical alkyle linéaire ou ramifié renfermant au plus 4 atomes de carbone,

– $Y'_{1a}$ représente un radical phényle,

– $B_a$ représente une simple liaison ou un radical -CO-NH-,

– $Y'_{2a}$ est tel que :

soit, si $B_a$ représente une simple liaison ou un radical -CO-NH-, $Y'_{2a}$ représente un radical phényle substitué en ortho par un radical carboxy libre, salifié ou estérifié, ou un radical tétrazolyle,

soit, si $B_a$ représente une simple liaison, $Y'_{2a}$ représente un radical cyano, carboxy libre, salifié ou estérifié, étant entendu que si $B_a$ représente une simple liaison alors l'un au moins de $X_4$, $X_5$, $X_6$ et $X_7$ ne représente pas un radical méthine, lesdits produits de formule (I'a) étant sous toutes les formes isomères possibles racémiques, énantiomères et diastéréoisomères, ainsi que les sels d'addition avec les acides minéraux et organiques ou avec les bases minérales et organiques desdits produits de formule ($I'_a$), caractérisé en ce que l'on utilise au départ un composé de formule (III') ou (VI') telle que définie à la revendication 2 dans laquelle le cycle

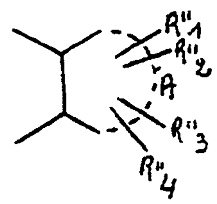

représente

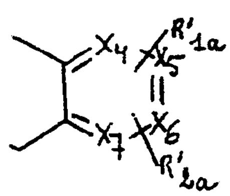

tel que défini ci-dessus dans lequel les fonctions réactives sont éventuellement protégées, un composé de formule (II) ou (II') dans laquelle R' a la signification indiquée ci-dessus $R'_a$ et un composé de formule (V) dans laquelle $R_5$ représente un radical $-CH_2-$ et Y' représente le groupe $Y'_{1a}$-$B_a$-$Y'_{2a}$ tel que défini ci-dessus dans lequel les fonctions réactives sont éventuellement protégées.

**6.**- Procédé selon la revendication 1 pour la préparation des produits de formule (I) telle que définie à la revendication 1, dans laquelle :

A représente un radical phényle, naphtyle, pyridyle, pyrimidinyle ou thiényle,

R représente un radical n-butyle ou butèn-1-yle,

$R_1$, $R_2$, $R_3$ et $R_4$ sont tels que deux d'entre eux représentent un atome d'hydrogène et les deux autres, identiques ou différents, représentent un atome d'hydrogène, un radical hydroxyle, un radical alkyle renfermant au plus 4 atomes de carbone, un radical carboxy libre ou estérifié par un radical alkyle linéaire ou ramifié renfermant au plus 4 atomes de carbone,

$R_5$ représente un radical méthylène,

et Y représente le radical $-Y_1$-B-$Y_2$ dans lequel $Y_1$ représente un radical phényle, B représente une liaison simple carbonecarbone ou le radical -CO-NH- et $Y_2$ représente un radical cyano, carboxy libre ou estérifié par un radical alkyle linéaire ou ramifié renfermant au plus 4 atomes de carbone, un radical indolyle ou un radical phényle éventuellement substitué par un radical carboxy libre, salifié ou estérifié, un radical tétrazolyle, tétrazolylalkyle, tétrazolylcarbamoyle, dans lesquels le radical tétrazolyle est éventuellement substitué par un radical alkyle, alkényle ou alcoxyalkyle, ou par un radical $-(CH_2)_p-SO_2-X_a-R_{14a}$ dans lequel p représente les valeurs 0 et 1, $X_a$ représente les radicaux -NH-, -NHCO-NH-, -NH-CO- ou une simple liaison et $R_{14a}$ représente un radical méthyle, éthyle, vinyle, allyle, pyridylméthyle, pyridyléthyle, pyridyle, phényle ou benzyle, lesdits produits de formule (I) étant sous toutes les formes isomères possibles racémiques, énantiomères et diastéréoisomères, ainsi que les sels d'addition avec les acides minéraux et organiques ou'avec les bases minérales et organiques desdits produits de formule (I), caractérisé en ce que l'on utilise au départ un composé de formule (II) ou (II') dans laquelle R' a la signification indiquée ci-dessus pour R, un composé de formule (III) ou (VI) dans laquelle le cycle comportant A et $R'_1$, $R'_2$, $R'_3$ et $R'_4$ ont la signification indiquée ci-dessus respectivement pour $R_1$, $R_2$, $R_3$ et $R_4$ dans lesquels les fonctions réactives sont éventuellement protégées et Y' représente le groupe $Y_1$-B-$Y_2$ tel que défini ci-dessus dans lequel les fonctions réactives sont éventuellement protégées.

**7.**- Procédé selon la revendication 2, pour la préparation des produits de formule (I') telle que définie à la revendication 2 dans laquelle le cycle comportant le groupe

A représente un radical phényle, naphtyle, pyridyle, pyrimidinyle,

R représente un radical n-butyle ou butèn-1-yle,

$R_1$, $R_2$, $R_3$ et $R_4$ sont tels que deux d'entre eux représentent un atome d'hydrogène et les deux autres, identiques ou différents, représentent un atome d'hydrogène, un radical alkyle libre ou estérifié par un radical alkyle linéaire ou ramifié renfermant au plus 4 atomes de carbone,

$R_5$ représente un radical méthylène,

et Y représente le radical $-Y_1$-B-$Y_2$ dans lequel $Y_1$ représente un radical phényle, B représente une liaison simple carbonecarbone ou le radical -CO-NH- et $Y_2$ représente un radical cyano, carboxy libre ou estérifié par un radical alkyle linéaire ou ramifié renfermant au plus 4 atomes de carbone, un radical indolyle, lesdits produits de formule (I') étant sous toutes les formes isomères possibles racémiques, énantiomères et diastéréoisomères, ainsi que les sels d'addition avec les acides minéraux et organiques ou avec les bases minérales et organiques desdits produits de formule (I'), caractérisé en ce que l'on utilise au départ un composé de formule (III') ou (VI') dans laquelle le cycle comportant le groupement A a la signification indiquée ci-dessus, et $R''_1$, $R''_2$, $R''_3$ et $R''_4$ ont la signification indiquée ci-dessus respectivement pour $R_1$, $R_2$, $R_3$ et $R_4$ dans lesquels les fonctions réactives sont éventuellement protégées et Y' représente le groupe $Y_1$-B-$Y_2$ tel que défini ci-dessus dans lequel les fonctions réactives sont éventuellement protégées.

**8.**- procédé selon la revendication 1 pour la préparation des produits de formule (I) telle que définie à la revendication 1 répondant à la formule ($I_b$) :

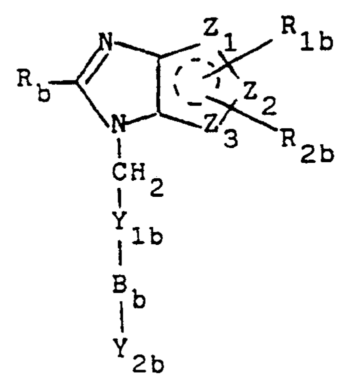

($I_b$)

dans laquelle $R_b$ représente un radical n-butyle ou butényle.

– $Z_1$, $Z_2$, $Z_3$ sont tels que :

l'un représente un atome de soufre

et les deux autres, identiques ou différents, représentent un radical méthine =CH-,

– $R_{1b}$ et $R_{2b}$, identiques ou différents, représentent :

. l'atome d'hydrogène,

. les atomes d'halogène,

. le radical hydroxyle, le radical mercapto,

. les radicaux alkoxy linéaires ou ramifiés renfermant au plus 6 atomes de carbone,

. les radicaux alkyle, alkényle, alkynyle et alkylthio, linéaires ou ramifiés renfermant au plus 6 atomes de carbone et les radicaux aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons

ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkoxy ou alkylthio renfermant au plus 4 atomes de carbone, mercapto, acyl, acyloxy,

. le radical carboxy libre,

. les radicaux carboxy estérifié par un radical alkyle linéaire ou ramifié renfermant au plus 4 atomes de carbone,

– $Y_{1b}$ représente un radical phényle,

– $B_b$ représente une simple liaison ou un radical -CO-NH-,

– $Y_{2b}$ est tel que :

soit, si $B_b$ représente une simple liaison ou un radical -CO-NH-, $Y_{2b}$ représente un radical phényle éventuellement substitué par un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, éthyle, n-propyle, vinyle, allyle, pyridylméthyle, pyridyléthyle, pyridyle, phényle, benzyle, pyrimidyle, tétrazolyle, thiazolyle, diazolyle, pipéridinyle ou tétrahydrofuranyle, ces radicaux étant éventuellement substitués par un radical méthyle, éthyle ou nitro,

soit, si $B_b$ représente une simple liaison, $Y_{2b}$ représente un radical cyano, formyle ou carboxy libre, salifié ou estérifié, lesdits produits de formule ($I_b$) étant sous toutes les formes isomères possibles racémiques, énantiomères et diastéréoisomères, ainsi que les sels d'addition avec les acides minéraux et organiques ou avec les bases minérales et organiques desdits produits de formule ($I_b$), caractérisé en ce que l'on utilise au départ un composé de formule (II) ou (II') dans laquelle R' a la signification indiquée ci-dessus pour $R_b$, un composé de formule (III) ou (VI) dans laquelle le cycle

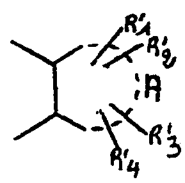

représente

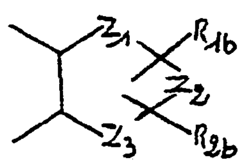

tel que défini ci-dessus dans lequel les fonctions réactives sont éventuellemerit protégées et un composé de formule (V) dans laquelle $R_5$ représente un radical $-CH_2-$ et Y' représente le groupe $Y_{1b}-B_b-Y_{2b}$ tel que défini ci-dessus dans lequel les éventuelles fonctions réactives sont éventuellement protégées.

**9.-** Procédé selon la revendication 1 pour la préparation des produits de formule (I) telle que définie à la revendication 1 répondant à la formule ($I_d$) :

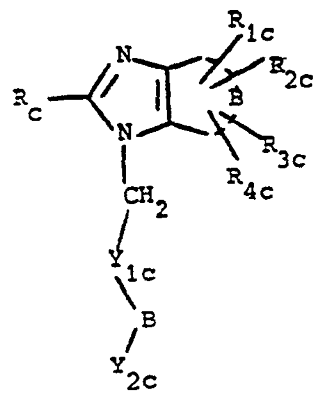

($I_d$)

dans laquelle

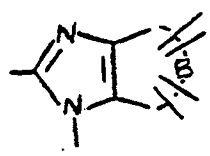

représente

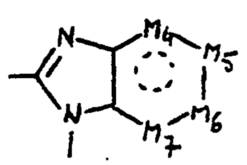 ou 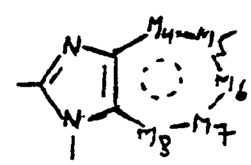

avec $M_4$, $M_5$, $M_6$, $M_7$ et $M_8$ identiques ou différents, représentent

- un radical méthine =CH- ou un atome d'azote éventuellement substitué par un radical choisi parmi les valeurs de $R_{1c}$, $R_{2c}$, $R_{3c}$, $R_{4c}$,
- un radical méthylène $-CH_2-$ éventuellement substitué par un ou deux radicaux identiques ou différents choisis parmi les valeurs de $R_{1c}$, $R_{2c}$, $R_{3c}$ et $R_{4c}$,
- un radical -C=O,
- un atome de soufre tels que l'un au moins de $M_4$, $M_5$, $M_6$, $M_7$ et $M_8$ représente un atome d'azote ou de soufre,

$R_c$ représente un radical alkyle ou alkényle renfermant au plus 4 atomes de carbone,

$R_{1c}$, $R_{2c}$, $R_{3c}$ et $R_{4c}$ identiques ou différents, sont choisis parmi :

- l'atome d'hydrogène,
- le radical carboxy libre, salifié ou estérifié par un radical alkyle renfermant'au plus 4 atomes de carbone ou par un radical aryle, ces radicaux alkyle et aryle étant eux-mêmes éventuellement substitués,
- les radicaux alkyle, alkényle et alkoxy renfermant au plus 4 atomes de carbone éventuellement substitués,
- les radicaux aryle,
- les radicaux amino et carbamoyle éventuellement subtitués par un ou deux radicaux identiques ou différents choisis parmi les radicaux alkyle et aryle eux-mêmes éventuellement substitués,

tous les radicaux alkyle, alkényle et aryle ci-dessus étant éventuellement substitués par un ou plusieurs radicaux choisis parmi les atomes d'halogène, les radicaux hydroxyle, carboxy libre, salifié ou estérifié par un radical alkyle renfermant au plus 4 atomes de carbone, amino, alkylamino, phényle, alkyle, alkényle et alkoxy renfermant au plus 4 atomes de carbone,

- $Y_{1c}$ représente un radical phényle,
- $B_c$ représente une simple liaison ou un radical -CO-NH-,
- $Y_{2c}$ est tel que :

soit, si $B_c$ représente une simple liaison ou un radical -CO-NH-, $Y_{2c}$ représente un radical phényle éventuellement substitué par un radical tétrazolyle, tétrazolylméthyl, tétrazolylcarbamoyle, le radical $-SO_2-X_b-$

$R_{14b}$ dans lequel $X_b$ représente une simple liaison, ou les radicaux -NH-, -CO- et -NH-CO- et $R_{14b}$ représente un radical méthyle, éthyle, n-propyle, vinyle, allyle, pyridylméthyle, pyridyléthyle, pyridyle, phényle, benzyle, pyrimidyle, tétrazolyle, thiazolyle, diazolyle, pipéridinyle ou tétrahydrofuranyle, ces radicaux étant éventuellement substitués par un radical méthyle, éthyle ou nitro,
soit, si $B_c$ représente une simple liaison, $Y_{2c}$ représente un radical cyano, formyle ou carboxy libre, salifié ou estérifié, lesdits produits de formule ($I_d$) étant sous toutes les formes isomères possibles racémiques, énantiomères et diastéréoisomères, ainsi que les sels d'addition avec les acides minéraux et organiques ou avec les bases minérales et organiques desdits produits de formule ($I_d$), caractérisé en ce que l'on utilise au départ un composé de formule (II) ou (II') dans laquelle R' a la signification indiquée ci-dessus pour $R_c$, un

composé de formule (III) ou (VI) dans laquelle 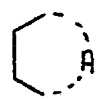
représente 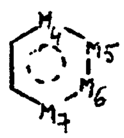 ou 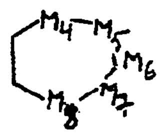 tel que défini ci-dessus

dans lequel les fonctions réactives sont éventuellement protégées et un composé de formule (V) dans laquelle $R_5$ représente un radical $-CH_2-$ et Y' représente le groupe $Y_{1c}$-$B_b$-$Y_{2c}$ ont les valeurs indiquées ci-dessus et dans lequel les éventuelles fonctions réactives sont éventuellement protégées.

**10.-** Procédé selon la revendication 8 pour la préparation des produits de formule ($I_b$) telle que définie à la revendication 8 répondant à la formule ($I_b'$) :

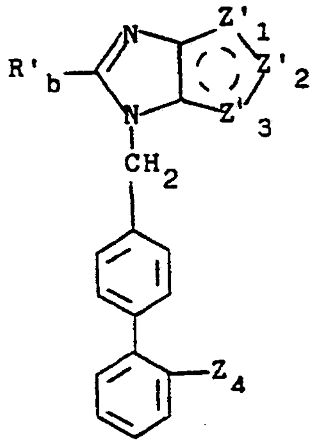

($I_b'$)

dans laquelle :
$R_b'$ représente un radical n-butyle,
$Z_1'$, $Z_2'$ et $Z_3'$ sont tels que :
l'un représente un atome de soufre,
et les deux autres, identiques ou différents, représentent un radical méthine =CH- éventuellement substitué par un radical hydroxyle ou un radical carboxy libre, salifié ou estérifié par un radical alkyle linéaire ou ramifié renfermant au plus 4 atomes de carbone,
et $Z_4$ représente un radical carboxy libre, salifié ou estérifié par un radical alkyle linéaire ou ramifié renfermant au plus 4 atomes de carbone ou un radical tétrazolyle, lesdits produits de formule ($I_b'$) étant sous toutes les formes isomères possibles racémiques, énantiomères et diastéréoisomères, ainsi que les sels d'addition avec les acides minéraux et organiques ou avec les bases minérales et organiques desdits produits de formule ($I_b'$), caractérisé en ce que l'on utilise au départ un composé de formule (III) ou (VI) dans

laquelle le cycle 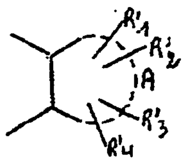 représente 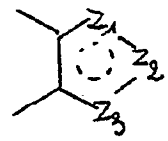 .

tel que défini ci-dessus et Y' représente le groupe 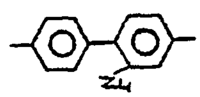

dans lequel $Z_4$ a la signification indiquée ci-dessus et dans lequel les éventuelles fonctions réactives sont éventuellement protégées.

**11.-** A titre de médicaments, les produits tels que définis par la formule (I) de la revendication 1, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

**Revendications pour l'Etat contractant suivant : GR**

**1.-** Procédé de préparation des produits de formule (I) :

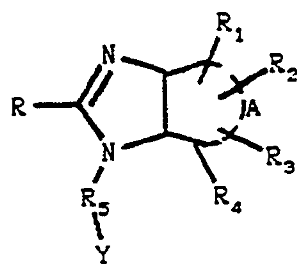

(I)

dans laquelle :

A représente le reste d'un radical monocyclique comprenant 3, 4 ou 5 chaînons ou d'un radical constitué de cycles condensés comprenant 6 à 12 chaînons, ces radicaux étant saturés ou insaturés et renfermant éventuellement un ou plusieurs hétéroatomes choisis parmi les atomes d'oxygène, et les atomes d'azote et de soufre éventuellement oxydés,

R représente un radical alkyle, alkényle ou alkynyle linéaire ou ramifié renfermant au plus 6 atomes de carbone et éventuellement substitués,

$R_1$, $R_2$, $R_3$ et $R_4$, identiques ou différents, représentent :

a) un atome d'hydrogène, un atome d'halogène, un radical hydroxyle, cyano, nitro, sulfo, formyle, benzoyle, acyle ou acyloxy ayant au plus 12 atomes de carbone, carboxy libre, salifié ou estérifié, mercapto,

b) un radical alkyle, alkényle, alkynyle, alkoxy ou alkylthio, ces radicaux étant linéaires ou ramifiés, renfermant au plus 6 atomes de carbone et étant éventuellement substitués,

c) un radical aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués,

d) un radical

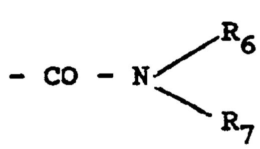 ou 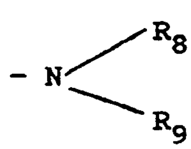

dans lesquels :

ou bien $R_6$ et $R_7$ ou $R_8$ et $R_9$, identiques ou différents, représentent :

– un atome d'hydrogène,

– un radical alkyle ou alkényle renfermant au plus 6 atomes de carbone et éventuellement substitué par un atome d'halogène, un radical hydroxyle ou un radical alkoxy renfermant au plus 6 atomes de carbone,

– un radical aryle ou arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié,

– un radical $-(CH_2)_m-SO_2-X-R_{14}$ dans lequel m représente un entier de 0 à 4 et

soit $X-R_{14}$ représente $NH_2$

soit X représente les radicaux -NH-, -NH-CO-HH- ou -NH-CO- ou une simple liaison

et $R_{14}$ représente un radical alkyle, alkényle et aryle, ces radicaux étant éventuellement substitués,

ou bien $R_6$ et $R_7$ ou $R_8$ et $R_9$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène; les radicaux hydroxyle, nitro, les radicaux alkyle, alkényle, alkoxy et acyle, ces radicaux renfermant au plus 6 atomes de carbone, les radicaux carboxy libre, salifié ou estérifié,

ou bien $R_8$ et $R_9$, identiques ou différents, représentent un radical acyle dérivé d'acide carboxylique renfermant au plus 6 atomes de carbone, ou un radical alkyle ou arylsulfonyle dans lequel le radical alkyl renferme au plus 6 atomes de carbone et le radical aryle renferme au plus 8 atomes de carbone, ces radicaux étant éventuellement substitués par un ou plusieurs substituants choisis parmi les atomes d'halogène ou les radicaux alkyle renfermant au plus 6 atomes de carbone,

e) un radical $-(CH_2)_m-SO_2-X-R_{14}$ dans lequel m, X et $R_{14}$ ont la signification précédente,

$R_5$ représente un radical divalent alkylène, linéaire ou ramifié, renfermant au plus 4 atomes de carbone,

Y représente le radical $-Y_1-B-Y_2$ dans lequel :

$Y_1$ représente un radical aryle monocyclique comprenant 5 ou 6 chaînons ou constitué de cycles condensés comprenant 8 à 10 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les radicaux que peuvent représenter $R_1$, $R_2$, $R_3$ et $R_4$,

B représente :

soit une simple liaison entre $Y_1$ et $Y_2$,

soit l'un des radicaux divalents suivants : -CO-, -NH-CO-, -CO-NH- ou $-O-(CH_2)_n-$ avec n représentant les valeurs 0 à 3,

$Y_2$ représente :

soit, quelle que soit la valeur de B autre qu'une simple liaison et $Y_2$ étant identique ou différent de $Y_1$, les valeurs définies pour $Y_1$,

soit, si B représente une simple liaison, un atome d'hydrogène, un radical cyano, carboxy libre, salifié ou estérifié, étant entendu que :

lorsque A représente un radical phényle éventuellement substitué par un ou deux radicaux choisis parmi les atomes d'halogène, les radicaux alkyle éventuellement substitué, alkoxy, acyle, carboxy libre ou phényle substitué par 5 atomes de fluor,

$R_5$ représente $-CH_2-$,

et Y représente le radical $-Y_1-B-Y_2$ dans lequel $Y_1$ représente un radical phényle non substitué et $Y_2$ représente un radical phényle substitué en ortho par un radical carboxy libre éventuellement salifié ou un radical tétrazolyle ou trifluorométhyl-sulfonamide et comportant éventuellement un second substituant choisi parmi les atomes d'halogène, les radicaux alkyle renfermant au plus 4 atomes de carbone, les radicaux nitro et méthoxy,

alors B représente -CO-NH- ou $-O-(CH_2)_n-$ avec n représentant les valeurs 2 ou 3,

lesdits produits de formule (I) étant sous toutes les formes isomères possibles racémiques, énantiomères et diastéréoisomères,

ainsi que les sels d'addition avec les acides minéraux et organiques ou avec les bases minérales et organiques desdits produits de formule (I), caractérisé en ce que :

soit l'on fait réagir un composé de formule (II) :

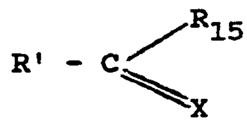

(II)

dans laquelle X représente un atome d'oxygène ou un radical =NH, $R_{15}$ représente un radical hydroxyle, alkoxy ou un atome d'halogène et $NH_2$, et R' a la signification indiquée ci-dessus pour R dans laquelle les éventuelles fonctions réactives sont éventuellement protégées par des groupements protecteurs, avec un composé de formule (III) :

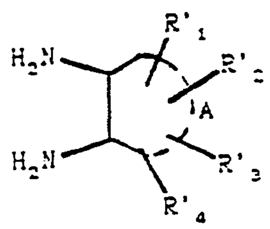

(III)

dans laquelle $R_1'$, $R_2'$, $R_3'$ et $R_4'$ ont les significations indiquées ci-dessus respectivement pour $R_1$, $R_2$, $R_3$ et $R_4$ dans lesquelles les éventuelles fonctions réactives sont éventuellement protégées par des groupements protecteurs, pour obtenir le composé de formule IV, après isolement éventuel d'un intermédiaire de formule (IV') :

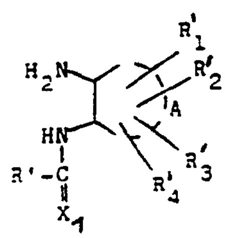

(IV')

dans laquelle A, $X_1$, R', $R'_1$, $R'_2$, $R'_3$, et $R'_4$ ont les significations indiquées ci-dessus, produit de formule (IV) :

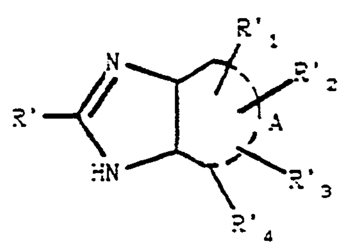

(IV)

dans laquelle R', $R_1'$, $R_2'$, $R_3'$ et $R_4'$ ont les significations indiquées ci-dessus, que l'on fait réagir avec un composé de formule (V) :

$$Hal - R_5 - Y' \qquad (V)$$

dans laquelle Hal représente un atome d'halogène, $R_5$ a la signification indiquée ci-dessus et Y' a la signification indiquée ci-dessus pour Y dans laquelle les les éventuelles fonctions réactives sont éventuellement protégées par des groupements protecteurs, pour obtenir des produits de formule (IX) :

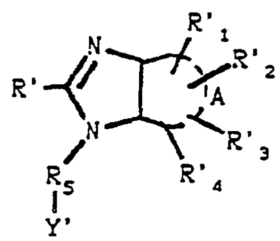

(IX)

dans laquelle R', $R_1'$, $R_2'$, $R_3'$, $R_4'$, $R_5'$ et Y' ont les significations indiquées ci-dessus, soit l'on fait réagir un composé de formule (VI) :

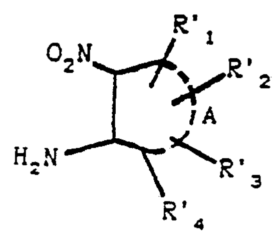

(VI)

dans laquelle $R_{1'}$, $R_2'$, $R_3'$ et $R_4'$ ont les significations indiquées ci-dessus respectivement pour $R_1$, $R_2$, $R_3$ et $R_4$ dans lesquelles les éventuelles fonctions réactives sont éventuellement protégées par des groupements protecteurs, ou bien avec le composé de formule (II') :

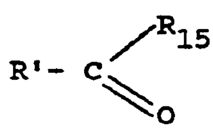

(II')

dans laquelle R' et $R_{15}$ ont les significations indiquées ci-dessus, pour obtenir le produit de formule (X) :

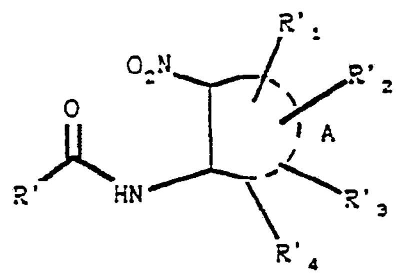

(X)

dans laquelle R', $R_1'$; $R_2'$, $R_3'$ et $R_4'$ ont les significations précédentes, produit de formule (X) soit que l'on réduit en produit de formule (X') :

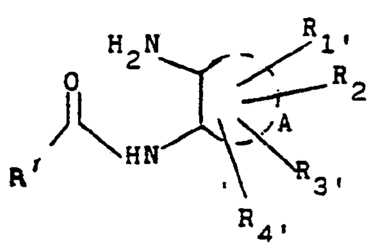

(X')

que l'on cyclise en produit de formule (IV) tel que défini ci-dessus que l'on traite ainsi qu'il est indiqué ci-dessus pour obtenir un produit de formule (IX) soit l'on fait réagir le produit de formule (X) avec le composé de formule

(V) telle que définie ci-dessus, pour obtenir un produit de formule (XI) :

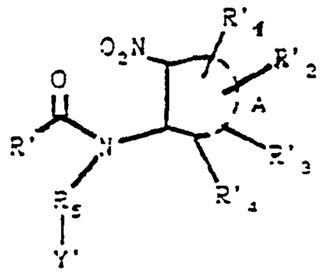

(XI)

dans laquelle R′, $R_1'$, $R_2'$, $R_3'$, $R_4'$, $R_5$ et Y′ ont les significations précédentes, que l'on soumet à une réaction de réduction sélective de la fonction nitro, pour obtenir le produit de formule (XII) :

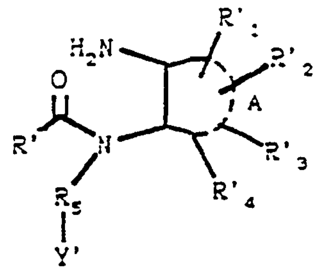

(XII)

dans laquelle R′, $R_1'$, $R_2'$, $R_3$, $R_4'$, $R_5$ et Y′ ont les significations précédentes, que l'on soumet à une réaction de cyclisation pour obtenir des produits de formule (IX) telle que définie ci-dessus;
ou bien l'on fait réagir le composé de formule (VI) avec le composé de formule (V) telle que définie ci-dessus, pour obtenir des produits de formule (VII) :

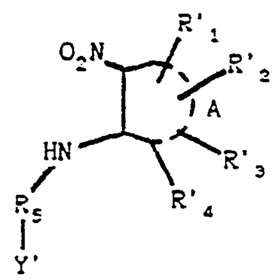

(VII)

dans laquelle $R_1'$, $R_2'$, $R_3'$, $R_4'$, $R_5$ et Y′ ont les significations indiquées ci-dessus, que :
ou bien l'on fait réagir avec le composé de formule (II′) tel que défini ci-dessus, pour obtenir un produit de formule (XI) tel que défini ci-dessus que l'on traite ensuite comme indiqué ci-dessus,
ou bien l'on soumet à une réaction de réduction du radical nitro en radical amino pour obtenir des produits de formule (VIII) :

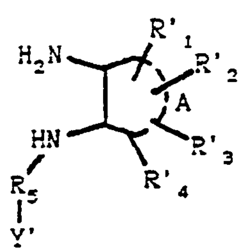

(VIII)

dans laquelle $R_1'$, $R_2'$, $R_3'$, $R_4'$, $R_5$ et Y′ ont les significations indiquées ci-dessus, que l'on fait réagir avec le produit de formule (II) telle que définie ci-dessus, pour obtenir un produit de formule (IX) telle que définie ci-dessus, produit de formule (IX) que l'on traite, si désiré et si nécessaire, à l'une ou plusieurs des réactions suivantes, dans un ordre quelconque :

– une réaction d'élimination des groupements protecteurs que peuvent porter les fonctions réactives protégées,
– une réaction de salification par un acide minéral ou organique ou par une base pour obtenir le sel correspondant,
– une réaction d'estérification ou salification de fonction acide,
– une réaction de saponification de fonction ester en fonction acide,
– une réaction de transformation de fonction alkoxy en fonction hydroxyle,
– une réaction de transformation de la fonction cyano en fonction acide,
– une réaction de réduction de la fonction carboxy en fonction alcool,
– une réaction de substitution de fonction hydroxyle ou mercapto par un atome d'halogène,
– une réaction de substitution sur un atome d'halogène,
– une réaction de dédoublement des formes racémiques en produits dédoublés,

lesdits produits de formule (I) ainsi obtenus étant sous toutes les formes isomères possibles racémiques, énantiomères et diastéréoisomères.

**2.**- Procédé selon la revendication 1, pour la préparation des produits de formule (I') correspondant aux produits de formule (I) telle que définie à la revendication 1 dans laquelle $R_1$, $R_2$, $R_3$ et $R_4$, identiques ou différents, ont la signification indiquée à la revendication 1 à l'exclusion de la valeur radical $-(CH_2)_m-SO_2-X-R_{14}$ tel que défini à la revendication 1 ou radical

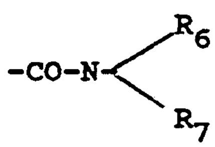

ou

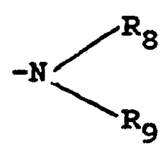

dans lesquels $R_6$ et $R_7$ ou $R_8$ et $R_9$ identiques ou différents représentent un radical aryle ou arylalkyle substitué par un ou plusieurs radicaux $-(CH_2)_m-SO_2-X-R_{14}$ tel que défini à la revendication 1, caractérisé en ce que l'on utilise au départ un composé de formule (III') :

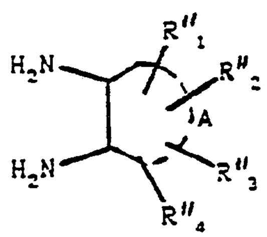

(III')

dans laquelle $R''_1$, $R''_2$, $R''_3$ et $R''_4$ ont les significations indiquées ci-dessus respectivement pour $R_1$, $R_2$, $R_3$ et $R_4$ dans lesquelles les fonctions réactives sont éventuellement protégées par des groupements protecteurs, ou un composé de formule (VI') :

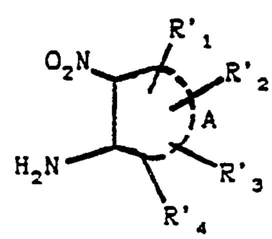

(VI')

dans laquelle $R''_1$, $R''_2$, $R''_3$ et $R''_4$ ont les significations indiquées ci-dessus.

**3.**- Procédé selon la revendication 1, caractérisé en ce que le ou les substituants, identiques ou différents que peuvent porter :

a) les radicaux alkyle, alkényle et alkynyle que peut représenter R,
b) les radicaux alkyle, alkényle, alkynyle, alkoxy et alkylthio que peuvent représenter $R_1$, $R_2$, $R_3$ et $R_4$,
c) les radicaux aryle, arylalkyle et arylalkényle que peuvent représenter $R_1$, $R_2$, $R_3$ et $R_4$

d) les radicaux alkyle, alkényle et aryle que peut représenter $R_{14}$,
sont choisis dans le groupe formé par :

– les atomes d'halogène, les radicaux hydroxyle, cyano, nitro, formyle, acyle ou acyloxy ayant au plus 6 atomes de carbone, benzoyle, carboxy libre, salifié ou estérifié par un radical alkyle renfermant au plus 6 atomes de carbone,

– les radicaux alkyle et alkényle renfermant au plus 6 atomes de carbone et éventuellement substitués par un ou plusieurs substituants choisis parmi les atomes d'halogène, le radical hydroxyle et les radicaux alkoxy renfermant au plus 6 atomes de carbone,

– les radicaux alkoxy linéaires et ramifiés renfermant au plus 6 atomes de carbone,

– les radicaux aryle et arylalkyle dans lequel le radical alkyle linéaire ou ramifié renferme au plus 6 atomes de carbone, ces radicaux agile et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone,les radicaux carboxy libre, salifié ou estérifié,

– les radicaux

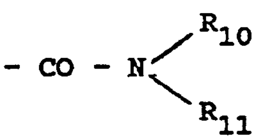

ou

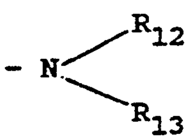

dans lesquels :

ou bien $R_{10}$ et $R_{11}$ ou $R_{12}$ et $R_{13}$, identiques ou différents, représentent :

– un atome d'hydrogène,

– un radical alkyle ou alkényle renfermant au plus 6 atomes de carbone et éventuellement substitué par un ou plusieurs substituants choisis parmi les atomes d'halogène, le radical hydroxyle et les radicaux alkoxy renfermant au plus 6 atomes de carbone,

– un radical aryle ou arylalkyle dans lequel le radical alkyle linéaire ou ramifié renferme au plus 6 atomes de carbone, ces radicaux aryle et arylalkyle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone,les radicaux carboxy libre, salifié ou estérifié, tétrazolyle, tétrazolylméthyle, tétrazolylcarbamoyle,

ou bien $R_{10}$ et $R_{11}$ ou $R_{12}$ et $R_{13}$ forment respectivement avec l'atome d'azote auquel ils sont liés un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote et de soufre, et étant éventuellement substitués par un ou plusieurs radicaux choisis parmi les atomes d'halogène, les radicaux hydroxyle, nitro, les radicaux alkyle, alkényle, alkoxy et acyle, ces radicaux renfermant au plus 6 atomes de carbone, les radicaux carboxy libre, salifié ou estérifié, tétrazolyle, tétrazolylméthyle, tétrazolylcarbamoyle,

ou bien $R_{12}$ et $R_{13}$, identiques ou différents, représentent un radical acyle dérivé d'acide carboxylique renfermant au plus 6 atomes de carbone.

**4.-** Procédé selon la revendication 1, pour la préparation des produits de formule (I) telle que définie à la revendication 1 répondant à la formule (Ia) :

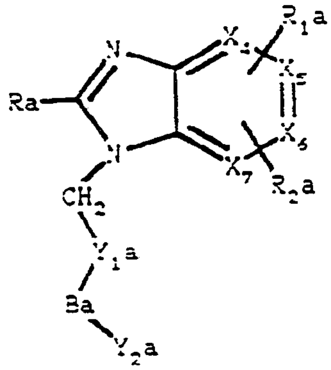

(Ia)

dans laquelle :

– $X_4$, $X_5$, $X_6$ et $X_7$ sont tels que :

soit ils représentent tous un radical méthine =CH-,

soit l'un ou deux quelconques d'entre eux représentent un atome d'azote et les autres représentent un radical méthine =CH-,

– $R_a$ représente un radical n-butyle ou butényle,

– $R_{1a}$ et $R_{2a}$, identiques ou différents, sont choisis dans le groupe formé par :

. l'atome d'hydrogène,

. les atomes d'halogène,

. le radical hydroxyle, le radical mercapto,

. les radicaux alkoxy linéaires ou ramifiés renfermant au plus 6 atomes de carbone,

. les radicaux alkyle, alkényle, alkynyle et alkylthio linéaires ou ramifiés renfermant au plus 6 atomes de carbone et les radicaux aryle, arylalkyle ou arylalkényle dans lesquels les radicaux alkyle et alkényle, linéaires ou ramifiés, renfermant au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkoxy, alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkyle, alkoxy ou alkylthio renfermant au plus 6 atomes de carbone, mercapto, acyl, acyloxy, tétrazolyle,

. le radical carboxy libre,

. les radicaux carboxy estérifié par un radical alkyle linéaire ou ramifié renfermant au plus 4 atomes de carbone,

– $Y_{1a}$ représente un radical phényle,

– $B_a$ représente une simple liaison ou un radical -CO-NH-,

– $Y_{2a}$ est tel que :

soit, si $B_a$ représente une simple liaison ou un radical -CO-NH-, $Y_{2a}$ représente un radical phényle éventuellement substitué par un radical $-(CH_2)_p-SO_2-X_a-R_{14a}$ dans lequel p représente les valeurs 0 et 1, $X_a$ représente les radicaux -NH-, -NH-CO-NH-, -NH-CO- ou une simple liaison et $R_{14a}$ représente un radical méthyle, éthyle, vinyle, allyle, pyridylméthyle, pyridyléthyle, pyridyle, phényle ou benzyle, par un radical carboxy libre, salifié ou estérifié, un radical tétrazolyle, tétrazolylalkyle ou tétrazolylcarbamoyle, dans lesquels le radical tétrazolyle est éventuellement substitué par un radical alkyle, alkényle, arylalkyle ou alcoxyalkyle,

soit, si $B_a$ représente une simple liaison, $Y_{2a}$ représente un radical cyano, carboxy libre, salifié ou estérifié, ou tétrazolyle, étant entendu-que si $B_a$ représente une simple liaison alors l'un au moins de $X_4$, $X_5$, $X_6$ et $X_7$ ne représente pas un radical méthine, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I), caractérisé en ce que l'on utilise au départ un composé de formule (II) ou (II') dans laquelle R' a la signification indiquée ci-dessus pour $R_a$, un composé de formule (III) ou (VI) dans laquelle le cycle

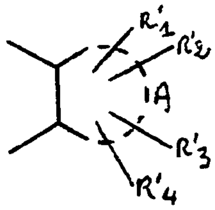

représente

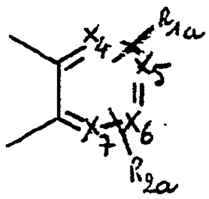

tel que défini ci-dessus et un composé de formule (V) dans laquelle $R_5$ représente un radical $-CH_2-$ et Y' représente le groupe $Y_{1a}$-$B_a$-$Y_{2a}$ tel que défrini ci-dessus dans lequel les fonctions réactives sont éventuellement protégées.

**5.-** Procédé selon la revendication 2, pour la préparation des produits de formule (I') telle que définie à la revendication 2 répondant à la formule ($I'_a$) :

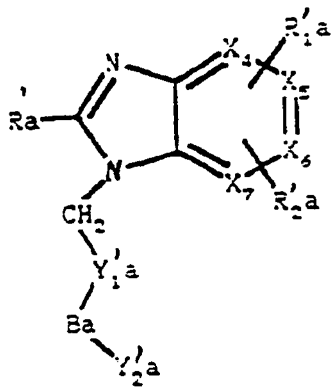

(I'a)

dans laquelle :

– $X_4$, $X_5$, $X_6$ et $X_7$ sont tels que :
soit ils représentent tous un radical méthine =CH-,
soit l'un ou deux quelconques d'entre eux représentent un atome d'azote et les autres représentent un radical méthine =CH-,

– $R'_a$ représente un radical n-butyle ou buténylе,
– $R'_{1a}$ et $R'_{2a}$, identiques ou différents, sont choisis dans le groupe formé par :
. l'atome d'hydrogène,
. le radical hydroxyle,
. les radicaux alkoxy linéaires ou ramifiés renfermant au plus 6 atomes de carbone,
. les radicaux alkyle linéaires ou ramifiés renfermant au plus 6 atomes de carbone et éventuellement substitués par un ou plusieurs substituants identiques ou différents choisis parmi les atomes d'halogène, le radical hydroxyle, les radicaux alkyle, alkoxy ou alkylthio renfermant au plus 4 atomes de carbone, mercapto,
. le radical carboxy libre,
. les radicaux carboxy estérifié par un radical alkyle linéaire ou ramifié renfermant au plus 4 atomes de carbone,
– $Y'_{1a}$ représente un radical phényle,
– $B_a$ représente une simple liaison ou un radical -CO-NH-,

– $Y'_{2a}$ est tel que :

soit, si $B_a$ représente une simple liaison ou un radical -CO-NH-, $Y'_{2a}$ représente un radical phényle substitué en ortho par un radical carboxy libre, salifié ou estérifié, ou un radical tétrazolyle,

soit, si $B_a$ représente une simple liaison, $Y'_{2a}$ représente un radical cyano, carboxy libre, salifié ou estérifié, étant entendu que si $B_a$ représente une simple liaison alors l'un au moins de $X_4$, $X_5$, $X_6$ et $X_7$ ne représente pas un radical méthine, lesdits produits de formule ($I'_a$) étant sous toutes les formes isomères possibles racémiques, énantiomères et diastéréoisomères, ainsi que les sels d'addition avec les acides minéraux et organiques ou avec les bases minérales et organiques desdits produits de formule ($I'_a$), caractirisé en ce que l'on utilise au départ un composé de formule (III') ou (VI') telle que définie à la revendication 2 dans laquelle le cycle

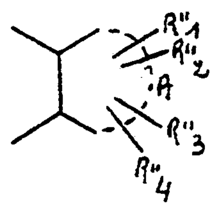

représente

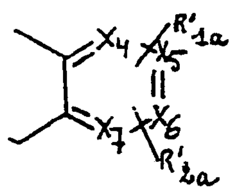

tel que défini ci-dessus dans lequel les fonctions réactives sont éventuellement protégées, un composé de formule (II) ou (II') dans laquelle R' a la signification indiquée ci-dessus $R'_a$ et un composé de formule (V) dans laquelle $R_5$ représente un radical $-CH_2-$ et Y' représente le groupe $Y'_{1a}$-$B_a$-$Y'_{2a}$ tel que défini ci-dessus dans lequel les fonctions réactives sont éventuellement protégées.

**6.**- Procédé selon la revendication 1 pour la préparation des produits de formule (I) telle que définie à la revendication 1, dans laquelle :

A représente un radical phényle, naphtyle, pyridyle, pyrimidinyle ou thiényle,

R représente un radical n-butyle ou butèn-1-yle,

$R_1$, $R_2$, $R_3$ et $R_4$ sont tels que deux d'entre eux représentent un atome d'hydrogène et les deux autres, identiques ou différents, représentent un atome d'hydrogène, un radical hydroxyle, un radical alkyle renfermant au plus 4 atomes de carbone, un radical carboxy libre ou estérifié par un radical alkyle linéaire ou ramifié renfermant au plus 4 atomes de carbone,

$R_5$ représente un radical méthylène,

et Y représente le radical $-Y_1$-B-$Y_2$ dans lequel $Y_1$ représente un radical phényle, B représente une liaison simple carbonecarbone ou le radical -CO-NH- et $Y_2$ représente un radical cyano, carboxy libre ou estérifié par un radical alkyle linéaire ou ramifié renfermant au plus 4 atomes de carbone, un radical indolyle ou un radical phényle éventuellement substitué par un radical carboxy libre, salifié ou estérifié, un radical tétrazolyle, tétrazolylalkyle, tétrazolylcarbamoyle, dans lesquels le radical tétrazolyle est éventuellement substitué par un radical alkyle, alkényle ou alcoxyalkyle, ou par un radical $-(CH_2)_p-SO_2-X_a-R_{14a}$ dans lequel p représente les valeurs 0 et 1, $X_a$ représente les radicaux -NH-, NHCO-NH-, -NH-CO- ou une simple liaison et $R_{14a}$ représente un radical méthyle, éthyle, vinyle, allyle, pyridylméthyle, pyridyléthyle, pyridyle, phényle ou benzyle, lesdits produits de formule (I) étant sous toutes les formes isomères possibles racémiques, énantiomères et diastéréoisomères, ainsi que les sels d'addition avec les acides minéraux et organiques ou-avec les bases minérales et organiques desdits produits de formule (I), caractérisé en ce que l'on utilise au départ un composé de formule (II) ou (II') dans laquelle R' a la signification indiquée ci-dessus pour R, un composé de formule (III) ou (VI) dans laquelle le cycle comportant A et $R'_1$, $R'_2$, $R'_3$ et $R'_4$ ont la signification indiquée ci-dessus respectivement pour $R_1$, $R_2$, $R_3$ et $R_4$ dans lesquels les fonctions réactives sont éventuellement protégées et Y' représente le groupe $Y_1$-B-$Y_2$ tel que défini ci-dessus dans lequel les fonctions réactives sont éventuellement protégées.

**7.**- Procédé selon la revendication 2, pour la préparation des produits de formule (I') telle que définie à la

revendication 2 dans laquelle le cycle comportant le groupe
A représente un radical phényle, naphtyle, pyridyle, pyrimidinyle,
R représente un radical n-butyle ou butèn-1-yle,
$R_1$, $R_2$, $R_3$ et $R_4$ sont tels que deux d'entre eux représentent un atome d'hydrogène et les deux autres, identiques ou différents, représentent un atome d'hydrogène, un radical alkyle libre ou estérifié par un radical alkyle linéaire ou ramifié renfermant au plus 4 atomes de carbone,
$R_5$ représente un radical méthylène,
et Y représente le radical $-Y_1$-B-$Y_2$ dans lequel $Y_1$ représente un radical phényle, B représente une liaison simple carbonecarbone ou le radical -CO-NH- et $Y_2$ représente un radical cyano, carboxy libre ou estérifié par un radical alkyle linéaire ou ramifié renfermant au plus 4 atomes de carbone, un radical indolyle, lesdits produits de formule (I') étant sous toutes les formes isomères possibles racémiques, énantiomères et diastéréoisomères, ainsi que les sels d'addition avec les acides minéraux et organiques ou avec les bases minérales et organiques desdits produits de formule (I'), caractérisé en ce que l'on utilise au départ un composé de formule (III') ou (VI') dans laquelle le cycle comportant le groupement A a la signification indiquée ci-dessus, et $R''_1$, $R''_2$, $R''_3$ et $R''_4$ ont la signification indiquée ci-dessus respectivement pour $R_1$, $R_2$, $R_3$ et $R_4$ dans lesquels les fonctions réactives sont éventuellement protégées et Y' représente le groupe $Y_1$-B-$Y_2$ tel que défini ci-dessus dans lequel les fonctions réactives sont éventuellement protégées.

**8.-** procédé selon la revendication 1 pour la préparation des produits de formule (I) telle que définie à la revendication 1 répondant à la formule ($I_b$) :

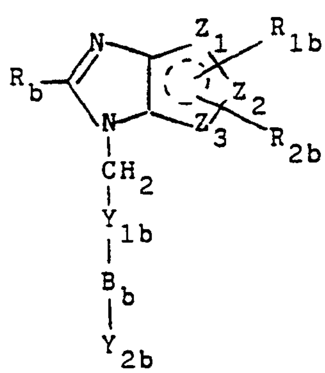

($I_b$)

dans laquelle $R_b$ représente un radical n-butyle ou butényle.

– $Z_1$, $Z_2$, $Z_3$ sont tels que :
l'un représente un atome de soufre
et les deux autres, identiques ou différents, représentent un radical méthine =CH-,

– $R_{1b}$ et $R_{2b}$, identiques ou différents, représentent :
. l'atome d'hydrogène,
. les atomes d'halogène,
. le radical hydroxyle, le radical mercapto,
. les radicaux alkoxy linéaires ou ramifiés renfermant au plus 6 atomes de carbone,
. les radicaux alkyle, alkényle, alkynyle et alkylthio, linéaires ou ramifiés renfermant au plus 6 atomes de carbone et les radicaux aryle, arylalkyle ou arylalkényle dans lesquels les radicaux alkyle et alkényle, linéaires ou ramifiés, renferment au plus 6 atomes de carbone, ces radicaux aryle, arylalkyle et arylalkényle étant tels que le radical aryle représente un radical monocyclique comprenant 5 ou 6 chaînons ou un radical constitué de cycles condensés comprenant 8 à 14 chaînons, ces radicaux renfermant éventuellement un ou plusieurs hétéroatomes choisis parmi les atomes d'oxygène, d'azote ou de soufre, tous ces radicaux alkyle, alkényle, alkynyle, alkylthio, aryle, arylalkyle et arylalkényle étant éventuellement substitués par un ou plusieurs substituants identiques ou différents choisis parmi les atomes d'halogène, le radical hydroxyle, les radicaux alkoxy ou alkylthio renfermant au plus 4 atomes de carbone, mercapto, acyl, acyloxy,
. le radical carboxy libre,
. les radicaux carboxy estérifié par un radical alkyle linéaire ou ramifié renfermant au plus 4 atomes de carbone,

– $Y_{1b}$ représente un radical phényle,
– $B_b$ représente une simple liaison ou un radical -CO-NH-,
– $Y_{2b}$ est tel que :

soit, si $B_b$ représente une simple liaison ou un radical -CO-NH-, $Y_{2b}$ représente un radical phényle éventuellement substitué par un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, éthyle, n-propyle, vinyle, allyle, pyridylméthyle, pyridyléthyle, pyridyle, phényle, benzyle, pyrimidyle, tétrazolyle, thiazolyle, diazolyle, pipéridinyle ou tétrahydrofuranyle, ces radicaux étant éventuellement substitués par un radical méthyle, éthyle ou nitro,
soit, si $B_b$ représente une simple liaison, $Y_{2b}$ représente un radical cyano, formyle ou carboxy libre, salifié ou estérifié, lesdits produits de formule $(I_b)$ étant sous toutes les formes isomères possibles racémiques, énantiomères et diastéréoisomères, ainsi que les sels d'addition avec les acides minéraux et organiques ou avec les bases minérales et organiques desdits produits de formule $(I_b)$, caractérisé en ce que l'on utilise au départ un composé de formule (II) ou (II') dans laquelle R' a la signification indiquée ci-dessus pour $R_b$, un composé de formule (III) ou (VI) dans laquelle le cycle

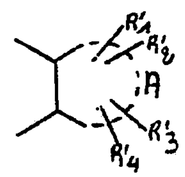

représente

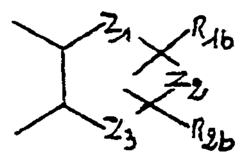

tel que défini ci-dessus dans lequel les fonctions réactives sont éventuellement protégées et un composé de formule (V) dans laquelle $R_5$ représente un radical $-CH_2-$ et Y' représente le groupe $Y_{1b}-B_b-Y_{2b}$ tel que défini ci-dessus dans lequel les éventuelles fonctions réactives sont éventuellement protégées.

**9.-** Procédé selon la revendication 1 pour la préparation des produits de formule (I) telle que définie à la revendication 1 répondant à la formule $(I_d)$ :

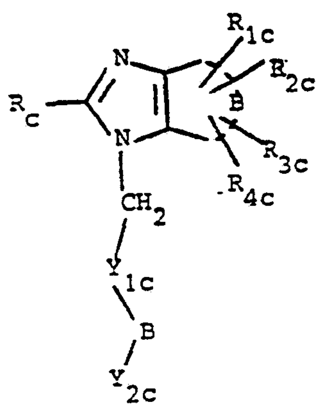

$(I_d)$

dans laquelle

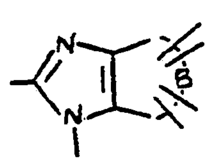

représente

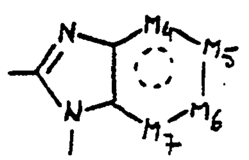 ou 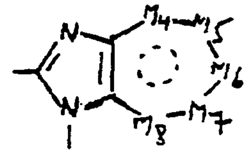

avec $M_4$, $M_5$, $M_6$, $M_7$ et $M_8$ identiques ou différents, représentent

– un radical méthine =CH- ou un atome d'azote éventuellement substitué par un radical choisi parmi les valeurs de $R_{1c}$, $R_{2c}$, $R_{3c}$et $R_{4c}$,

– un radical méthylène $-CH_2-$ éventuellement substitué par un ou deux radicaux identiques ou différents choisis parmi les valeurs de $R_{1c}$, $R_{2c}$, $R_{3c}$ et $R_{4c}$,

– un radical -C=O,

– un atome de soufre tels que l'un au moins de $M_4$, $M_5$, $M_6$, $M_7$ et $M_8$ représente un atome d'azote ou de soufre,

$R_c$ représente un radical alkyle ou alkényle renfermant au plus 4 atomes de carbone,

$R_{1c}$, $R_{2c}$, $R_{3c}$ et $R_{4c}$ identiques ou différents, sont choisis parmi :

– l'atome d'hydrogène,

– le radical carboxy libre, salifié ou estérifié par un radical alkyle renfermant au plus 4 atomes de carbone ou par un radical aryle, ces radicaux alkyle et aryle étant eux-mêmes éventuellement substitués,

– les radicaux alkyle, alkényle et alkoxy renfermant au plus 4 atomes de carbone éventuellement substitués,

– les radicaux aryle,

– les radicaux amino et carbamoyle éventuellement subtitués par un ou deux radicaux identiques ou différents choisis parmi les radicaux alkyle et aryle eux-mêmes éventuellement substitués,

tous les radicaux alkyle, alkényle et aryle ci-dessus étant éventuellement substitués par un ou plusieurs radicaux choisis parmi les atomes d'halogène, les radicaux hydroxyle, carboxy libre, salifié ou estérifié par un radical alkyle renfermant au plus 4 atomes de carbone, amino, alkylamino, phényle, alkyle, alkényle et alkoxy renfermant au plus 4 atomes de carbone,

– $Y_{1c}$ représente un radical phényle,

– $B_c$ représente une simple liaison ou un radical -CO-NH-,

– $Y_{2c}$ est tel que :

soit, si $B_c$ représente une simple liaison ou un radical -CO-NH-, $Y_{2c}$ représente un radical phényle éventuellement substitué par un radical tétrazolyle, tétrazolylméthyl, tétrazolylcarbamoyle, le radical $-SO_2-X_b-R_{14b}$ dans lequel $X_b$ représente une simple liaison, ou les radicaux -NH-, -CO- et -NH-CO- et $R_{14b}$ représente un radical méthyle, éthyle, n-propyle, vinyle, allyle, pyridylméthyle, pyridyléthyle, pyridyle, phényle, benzyle, pyrimidyle, tétrazolyle, thiazolyle, diazolyle, pipéridinyle ou tétrahydrofuranyle, ces radicaux étant éventuellement substitués par un radical méthyle, éthyle ou nitro,

soit, si $B_c$ représente une simple liaison, $Y_{2c}$ représente un radical cyano, formyle ou carboxy libre, salifié ou estérifié, lesdits produits de formule ($I_d$) étant sous toutes les formes isomères possibles racémiques, énantiomères et diastéréoisomères, ainsi que les sels d'addition avec les acides minéraux et organiques ou avec les bases minérales et organiques desdits produits de formule ($I_d$), caractérisé en ce que l'on utilise au départ un composé de formule (II) ou (II') dans laquelle R' a la signification indiquée ci-dessus pour $R_c$, un

composé de formule (III) ou (VI) dans laquelle 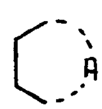

représente 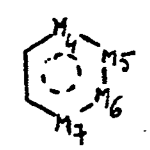 ou 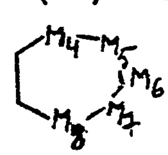 tel que défini ci-dessus

dans lequel les fonctions réactives sont éventuellement protégées et un composé de formule (V) dans laquëlle $R_5$ représente un radical $-CH_2-$ et Y' représente le groupe $Y_{1c}-B_b-Y_{2c}$ ont les valeurs indiquées ci-dessus et dans lequel les éventuelles fonctions réactives sont éventuellement protégées.

**10.-** Procédé selon la revendication 8 pour la préparation des produits de formule ($I_b$) telle que définie à la revendication 8 répondant à la formule ($I_b'$) :

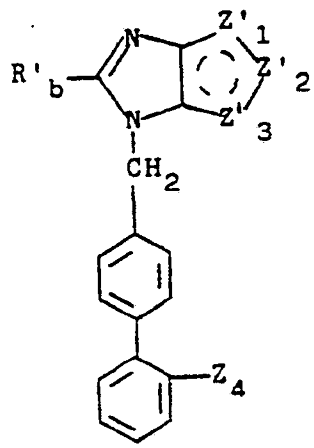

$(I_b')$

dans laquelle :
$R_b'$ représente un radical n-butyle,
$Z_1'$, $Z_2'$ et $Z_3'$ sont tels que :
l'un représente un atome de soufre,
et les deux autres, identiques ou différents, représentent un radical méthine =CH- éventuellement substitué par un radical hydroxyle ou un radical carboxy libre, salifié ou estérifié par un radical alkyle linéaire ou ramifié renfermant au plus 4 atomes de carbone,
et $Z_4$ représente un radical carboxy libre, salifié ou estérifié par un radical alkyle linéaire ou ramifié renfermant au plus 4 atomes de carbone ou un radical tétrazolyle, lesdits produits de formule $(I_b')$ étant sous toutes les formes isomères possibles racémiques, énantiomères et diastéréoisomères, ainsi que les sels d'addition avec les acides minéraux et organiques ou avec les bases minérales et organiques desdits produits de formule $(I_{b'})$, caractérisé en ce que l'on utilise au départ un composé de formule (III) ou (VI) dans laquelle le cycle

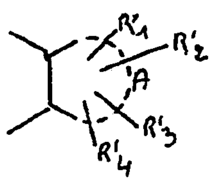

représente

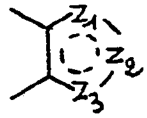

tel que défini ci-dessus et Y' représente le groupe

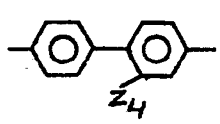

dans lequel $Z_4$ a la signification indiquée ci-dessus et dans lequel les éventuelles fonctions réactives sont éventuellement protégées.

**11.-** A titre de médicaments, les produits tels que définis par la formule (I) de la revendication 1, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

**12.**- Procédé selon l'une quelconque des revendications 1 à 11 caractérisé en ce que l'on utilise au départ des composés choisis de manière telle que l'on prépare l'un quelconque des produits dont les noms suivent :
l'acide 2-butyl 1-[(4-carboxyphényl) méthyl) 1H-benzimidazole -6-carboxylique
l'acide 4-[(2-butyl-1H-benzimidazol-1-yl) méthyl) benzoïque
le 4-[(2-butyl-1H-benzimidazol-1-yl) méthyl] N-(1H-indol-4-yl) benzamide
l'acide 4-[(2-butyl-1H-naphth(2,3-d)imidazol-1-yl) méthyl] benzoïque
l'acide 4-[(2-butyl-5,6-diméthyl-1H-benzimidazol-1-yl) méthyl) benzoïque
l'acide 4-[(2-butyl-3H-imidazo(4,5-c)pyridin-3-yl) méthyl] benzoïque
l'acide 4'-((2-butyl-3H-imidazo(4,5b) pyridin-3-yl) méthyl) (1,1'-biphényl) 2-carboxylique
le 2-butyl-1-((2'-carboxy-(1,1'-biphényl)-4yl) méthyl)-6-hydroxy-1H-thiéno(2,3-d)imidazole-5-carboxylate de 1,1-diméthyléthyle et leurs sels.

**13.**- Procédé de préparation de compositions pharmaceutiques caractérisé en ce que l'on met à titre de principe actif l'un au moins des produits de formule (I) telle que définie à la revendication 1 ou l'un au moins de leurs sels d'addition avec les acides et les bases pharmaceutiquement acceptables, sous une forme destinée à cet usage.

**14.**- Procédé de préparation de compositions pharmaceutiques caractérisé en ce que l'on met à titre de principe actif l'un au moins des produits de formule (I') telle que définie à la revendication 2 ou l'un au moins de leurs sels d'addition avec les acides et les bases pharmaceutiquement acceptables, sous une forme destinée à cet usage.

**15.**- procédé de préparation de compositions pharmaceutiques caractérisé en ce que l'on met à titre de principe actif l'un au moins des produits de formule (I') telle que définie à la revendication 12 ou l'un au moins de leurs sels d'addition avec les acides et les bases pharmaceutiquement acceptables, sous une forme destinée à cet usage.

**16.**- A titre de produits industriels nouveaux, les composés de formule (IV), (VII), (VIII), (XVI), (XVIII) et (XIX).

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande

EP 91 40 1481

## DOCUMENTS CONSIDERES COMME PERTINENTS

| | Catégorie | Citation du document avec indication, en cas de besoin, des parties pertinentes | Revendication concernée | CLASSEMENT DE LA DEMANDE (Int. CL5) |
|---|---|---|---|---|
| 1 | A | EP-A-0 186 190 (SUMITOMO CHEMICAL CO., LTD)<br>--- | | C 07 D 235/08<br>A 61 K 31/415<br>C 07 D 235/02<br>C 07 D 473/04<br>C 07 D 473/28<br>C 07 D 473/30<br>C 07 D 403/12<br>C 07 D 471/04<br>C 07 D 487/04<br>A 61 K 31/52<br>A 61 K 31/40 |
| 1 | A | US-A-4 880 804 (DAVID J. CARINI, A.O.[E.I.DU PONT NEMOURS AND CO.])<br>--- | | |
| 1 | A | EP-A-0 138 750 (CIBA-GEIGY AG)<br>--- | | |
| 1 | A | EP-A-0 291 969 (E.I.DU PONT DE NEMOURS AND CO.)<br>--- | | |
| 2 | P,A | EP-A-0 392 317 (DR. KARL THOMAE)<br>--- | | |
| 1 | P,A | EP-A-0 399 732 (IMPERIAL CHEMICAL IND.)<br>--- | | |
| 2 | P,A | EP-A-0 400 835 (MERCK & CO, INC.)<br>----- | | |

DOMAINES TECHNIQUES RECHERCHES (Int. CL5)

C 07 D 235/00
A 61 K 31/00
C 07 D 473/00
C 07 D 403/00
C 07 D 471/00
C 07 D 487/00
C 07 D 495/00
C 07 D 513/00

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches compl-tes:
Revendications ayant fait l'objet de recherches incompl-tes:
Revendications n'ayant pas fait l'objet de recherches:
Raison pour la limitation de la recherche:

La rédaction des revendications n'est pas claire ni concise (Art. 83-84 OEB), et représente une telle masse énorme de produits, qu'une recherche complète n'est pas possible pour des raisons d'économie (voir Directives relatives à l'examen pratique à l'OEB, Partie B, Chapitre III, 2.) De cette façon la recherche a été basés (règle 45) sur les composés, qui sont bien caractérisés par ses éléments physiques ou chimiques, c.a.d. les composés des examples.

| Lieu de la recherche | Date d'achèvement de la recherche | Examinateur |
|---|---|---|
| LA HAYE | 03-09-1991 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0401)